# EUROPEAN PATENT SPECIFICATION

(11) **EP 2 824 113 B1**
(45) Date of publication and mention of the grant of the patent: **03.05.2017**
(21) Application number: 14175482.0
(22) Date of filing: 02.07.2014
(51) Int. Cl.: C07K 16/28

(54) **BIOMARKER FOR SELECTING A SUBJECT FOR APPLICATION OF AN ANTI-C-MET ANTIBODY**
BIOMARKER ZUR AUSWAHL EINER PERSON ZUR ANWENDUNG EINES ANTI-C-MET-ANTIKÖRPERS
BIOMARQUEUR POUR SÉLECTIONNER UN SUJET POUR L'APPLICATION D'UN ANTICORPS DIRIGÉ CONTRE C-MET

(30) Priority: 09.07.2013 KR 20130080281; 30.10.2013 KR 20130130534
(43) Date of publication of application: 14.01.2015
(73) Proprietor: Samsung Electronics Co., Ltd, Gyeonggi-do 443-742 (KR)
(72) Inventor: Oh, Young Mi, Incheon (KR); Kim, Kyung Ah, Gyeonggi-do (KR)
(74) Representative: Grünecker Patent- und Rechtsanwälte PartG mbB

(56) References cited:
- EP-A1- 2 316 484
- WO-A2-2007/017758
- KR-A- 20130 037 189
- S. BYUN ET AL: "USP8 Is a Novel Target for Overcoming Gefitinib Resistance in Lung Cancer", CLINICAL CANCER RESEARCH, vol. 19, no. 14, 7 June 2013 (2013-06-07), pages 3894-3904, XP055152853, ISSN: 1078-0432, DOI: 10.1158/1078-0432.CCR-12-3696
- KARAMOUZIS M V ET AL: "Targeting MET as a strategy to overcome crosstalk-related resistance to EGFR inhibitors", LANCET ONCOLOGY, LANCET PUBLISHING GROUP, LONDON, GB, vol. 10, no. 7, 1 July 2009 (2009-07-01), pages 709-717, XP026238139, ISSN: 1470-2045, DOI: 10.1016/S1470-2045(09)70137-8 [retrieved on 2009-06-29]
- YOUNG MI OH ET AL: "USP8 modulates ubiquitination of LRIG1 for Met degradation", SCIENTIFIC REPORTS, vol. 4, 15 May 2014 (2014-05-15), XP055152854, DOI: 10.1038/srep04980

## Description

### BACKGROUND OF THE INVENTION

### 1. Field

A biomarker for selecting a subject for application of an anti-c-Met antibody, a method of selecting the subject for application of an anti-c-Met antibody including measuring a level of a ubiquitin peptidase and/or a ubiquitin peptidase coding gene, a method of preventing and/or treating a cancer including administering an anti-c-Met antibody to the selected subject, a pharmaceutical composition for preventing and/or treating a cancer including an anti-c-Met antibody and an inhibitor against a ubiquitin peptidase and/or a ubiquitin peptidase coding gene, and a method of preventing and/or treating a cancer including co-administering an anti-c-Met antibody and an inhibitor against a ubiquitin peptidase and/or a ubiquitin peptidase coding gene are provided.

### 2. Description of the Related Art

c-Met is a receptor for hepatocyte growth factor (HGF), a cytokine that binds the extracellular region of the c-Met receptor tyrosine kinase to induce cell division, movement, morphogenesis, and angiogenesis of various normal cells and tumor cells. c-Met is a representative receptor tyrosine kinase existing on the surface of cells, is itself a proto-oncogene, and is sometimes involved in various mechanisms related to cancer, such as cancer development, metastasis, migration, invasion, and angiogenesis, independent from the ligand, HGF.

In particular, c-Met is known to be involved in induction of resistance to commonly used anti-cancer drugs, and thus is regarded as important with respect to personalized treatments. Representative anti-cancer therapeutic drugs targeting epidermal growth factor receptor EGFR (ERBB1), i.e., Eribitux or Tarceva, work by blocking the signaling related to cancer development. In addition, Herceptin, which is well known as a breast cancer therapeutic drug, targets ERBB2 (HER2) and works by blocking the transduction of signals necessary for cell proliferation. Among patients resistant to the drugs described above, the signal transduction pathway that induces cell proliferation is not blocked due to the overexpression of c-Met. Thus, c-Met has emerged as a target of interest for many pharmaceutical companies, and antibodies against c-Met have been developed.

In order to increase the effect of therapies using the developed anti-c-Met antibodies, it is required to develop biomarkers for selecting a subject for application of the anti-c-Met antibody and/or for increasing the therapeutic effect of the anti-c-Met antibodies.

### BRIEF SUMMARY OF THE INVENTION

In one embodiment the invention pertains to an in vitro method of selecting a subject for administration of an anti-c-Met antibody, comprising: (i) determining the level of ubiquitin specific peptidase 8 (USP8), or the presence or absence of a mutation in an active site of USP8 in a biological sample from said subject; and (ii) selecting the subject for administration of the anti-c-Met antibody if the score measured by immunohistochemical staining reflecting the absence or low level of USP8 is -, 0, or 1; or the mutation is present in an active site affecting USP8 activity.

In another embodiment the invention pertains to a pharmaceutical composition for combination administration for use in a method for preventing or treating a cancer, comprising as active ingredients: i) an anti-c-Met antibody and ii) an inhibitor against USP8.

### BRIEF DESCRIPTION OF THE DRAWINGS

FIG. 1 is a photograph showing the results of immunoblotting (immunoprecipitation), which indicates the expression level of USP8 interacting with LRIG1 in LRIG1-overexpressed MKN45 cells after being treated with an anti-c-Met antibody.
FIG. 2 is a photograph showing the results of immunoblotting, which indicates the expression level of LRIG1 and USP8 in LRIG1- and USP8-overexpressed MKN45 cells after being treated with an anti-c-Met antibody.
FIG. 3 is a photograph showing the results of immunoblotting, which indicates the expression level of LRIG1, USP8, and ubiquitin in USP8-overexpressed EBC1 cells (left) and USP8 knock-down EBC1 cells (right) after being treated with an anti-c-Met antibody.
FIG. 4 is a graph showing cell viability (%) of USP8 knock-down EBC1 cells after being treated with an anti-c-Met antibody.
FIG. 5 is a photograph showing the results of immunoblotting, which indicates the expression level of LRIG1, USP8, and c-Met in wild type USP8-overexpressed EBC1 cells and mutated USP8-overexpressed EBC1 cells (right) after being treated with an anti-c-Met antibody.
FIG. 6 is a graph showing cell viability (%) of cells transfected with USP8-WT, USP8-CS or shUSP8 after being treated with an anti-c-Met antibody.
FIG. 7 is a graph showing the LRIG1 level in patient-derived lung tumor xenograft samples (# 1, 2, 3, 4, 5, & 6).
FIG. 8 is a photograph showing the results of western blotting, which indicates the USP8 level in patient-derived lung tumor xenograft samples # 1 and 2.
FIG. 9 is a graph showing the change of the c-Met level in patient-derived lung tumor xenograft samples # 1 and 2 by treating with an anti-c-Met antibody.
FIG. 10 is a graph showing the change of the tumor volume in patient-derived lung tumor xenograft samples # 1 and 2 by treating with an anti-c-Met antibody (Asterisks (*): P-values versus vehicle group according to repeated measures ANOVA (*P<0.05, **P<0.01, ***P<0.001, ****P<0.0001)).

### DETAILED DESCRIPTION OF THE INVENTION

Anti-c-Met antibodies can induce c-Met degradation in a LRIG1-dependent manner. When anti-c-Met antibodies induce c-Met degradation through LRIG1, c-Met activation is not required. Thus, re-use of c-Met can be prevented and the anticancer efficacy of the anti-c-Met antibody can be maximized.

Without wishing to be bound by any particular theory or mechanism of action, it is believed that anti-c-Met antibodies, particularly those described herein, can effectively lower the c-Met level in a subject in a LRIG1-dependent manner, wherein ubiquitination of LRIG1 is induced. It is further believed that anti-c-Met antibodies inhibit the interaction between LRIG1 and ubiquitin peptidase (e.g., ubiquitin specific peptidase (USP) family), whereby the ubiquitination of LRIG1 is induced.

Therefore, in anticancer therapy using an anti-c-Met antibody, specifically an anti-c-Met antibody acting in a LRIG1-dependent manner, when a patient group having high levels of LRIG1 and low levels of ubiquitin peptidase, relative to a reference sample as described below, is selected and treated with the specific antibody, more successful therapy by LRIG1-dependent c-Met targeting mechanism of the antibody can be achieved. In this case, the decrease of interaction between ubiquitin peptidase and LRIG1 leads to stimulation ubiquitination of LRIG1, thereby contributing to c-Met degradation by LRIG1.

Therefore, the presence/absence and/or level of ubiquitin peptidase can be utilized as a molecular marker (biomarker) in therapy using anti-c-Met antibodies.

One embodiment of the disclosure is a use of a ubiquitin peptidase and/or a gene for a ubiquitin peptidase as a biomarker for selecting (identifying) a subject for application of an anti-c-Met antibody.

In particular, a biomarker composition for selecting (identifying) a subject for application of an anti-c-Met antibody, including a ubiquitin peptidase and/or a gene for a ubiquitin peptidase is disclosed.

A kit is disclosed for selecting (identifying) a subject for application of an anti-c-Met antibody, including a means for detecting a ubiquitin peptidase and/or a gene for a ubiquitin peptidase and/or a means measuring the activity of a ubiquitin peptidase.

An embodiment of the invention provides a method of selecting (identifying) a subject for application of an anti-c-Met antibody including determining the presence and/or the level (or amount) of the ubiquitin peptidase USP8 and/or presence of absence of a mutation in the active site of USP8, in a biological sample.

In the selection of a subject for application of an anti-c-Met antibody, when a ubiquitin peptidase (e.g., USP8) and/or a gene for a ubiquitin peptidase is absent or present at a low level in the biological sample, the biological sample or the patient from which the biological sample is obtained (separated) may be considered (or selected as) a suitable subject for application of an anti-c-Met antibody. For example, the level of a ubiquitin peptidase (e.g., USP8) may be determined by immunohistochemical staining using a general antibody against the ubiquitin peptidase. The immunohistochemical staining is a method for identifying a material present in a cell or a tissue using antigen-antibody response, wherein a frozen or paraffin tissue section is generally used. A tissue section having a regular thickness is blocked for preventing non-specific binding of an antibody, and then, treated with a primary antibody. After a certain period, non-reacting primary antibody is removed, and the tissue section is treated with a secondary antibody. The secondary antibody attached tissue section can be detected using a streptavidin-attached material, such as streptavidin-HRP or streptavidin-alkaline phosphatase, which can bind to biotin attached to the secondary antibody. Most of the detecting responses are color reactions, which can be analyzed by a proper microscope. The staining may be scored on a scale ranging, e.g., from '-' or '0' to '+3,' wherein a score (stain intensity) of '-' or '0' represents no protein expression (no signal, negative), score of '+1' represents no or a slight protein expression (corresponding to a background signal), and scores of '+2' (strongly positive) to '+3' (very strongly positive) represent progressively increased levels of protein expression (the case showing the signal higher than '+3' is incorporated in the score of '+3') (the scores can be determined by a pathologist). Thus, when the score measured by immunohistochemical staining is "-", "0", or "+1", the level of the ubiquitin peptidase may be determined as "negative", and when the score measured by immunohistochemical staining is greater than "+1" (i.e., "+2", or "+3"), the level of the ubiquitin peptidase may be determined as "positive", where the "negative" may be understood as absence, or presence at a low level, of the ubiquitin peptidase (e.g., USP8) in the biological sample. Therefore, the above "absence, or presence at a low level, in the biological sample" may correspond to the case that the score measured by immunohistochemical staining using an antibody against the ubiquitin peptidase is "-", "0", or "+ 1 ".

Alternatively, the "low level of a ubiquitin peptidase (e.g., USP8) and/or a gene for a ubiquitin peptidase or an activity of a ubiquitin peptidase" may be determined by comparing the level of a ubiquitin peptidase (e.g., USP8) in a biological sample from a subject with that in a reference sample. The reference sample may be any one to which a c-Met inhibitor such as an anti-c-Met antibody has no effect or having a resistance to a c-Met inhibitor. For example, the reference sample may be at least one selected from the group consisting of cell lines H1373 (ATCC, CRL-5866), Caki-1 (ATCC, HTB-46), BT474 (ATCC, HTB-20), HT-29 (ATCC, HTB-38), SW620 (ATCC, CCL-227), Ls174T (ATCC, CL-188), and cells acquiring a resistance to a c-Met inhibitor by repeated and/or consistent administration of the c-Met inhibitor. Therefore, the method of selecting (identifying) a subject for application of an anti-c-Met antibody may further include a step of comparing the level of a ubiquitin peptidase and/or a ubiquitin peptidase coding gene in a biological sample from a patient with that of a reference sample as described above. In this case, the method may further include a step of measuring the level of a ubiquitin peptidase and/or a ubiquitin peptidase coding gene or an activity of a ubiquitin peptidase, in the reference sample. The method may further include a step of determining (considering or selecting) the biological sample or the patient from which the biological sample is obtained (separated) as a suitable subject for application of an anti-c-Met antibody, when a ubiquitin peptidase and/or a ubiquitin peptidase coding gene or an activity of a ubiquitin peptidase is absent in the biological sample, or present at a low level in the biological sample compared to that of the reference sample.

The enzymatic activity of the ubiquitin peptidase (e.g., USP8) relating to ubiquitination (binding of ubiquitin) of a target is important. Therefore, the subject, whose a ubiquitin peptidase has no enzymatic activity or low enzymatic activity due to a mutation in an active site of the enzyme, and the like-thereby leading to no effect of ubiquitination of a target or low level of the ubiquitination)-may be determined to be suitable for the application of the anti-c-Met antibody. Therefore, the step of measuring the activity of a ubiquitin peptidase may include a step of determining the presence or absence of a mutation in an active site of a ubiquitin peptidase (e.g., USP8), or measuring the degree of ubiquitination of a target (e.g., LRIG1). For example, the mutation in an active site of a ubiquitin peptidase (e.g., USP8) may be a substitution of the amino acid residue Cys at the 786^{th} position of NP_001122082 (human USP8; SEQ ID NO: 109) with Ser (C786S). When the mutation or a mutant having the mutation is found in the biological sample, or the ubiquitination of a target (e.g., LRIG1) is found at a low level or not at all in the biological sample, the biological sample or a patient from which the biological sample is obtained (separated) may be considered a suitable subject for the application of an anti-c-Met antibody.

The subject for the application of an anti-c-Met antibody may be any subject suitable for the application of a therapy using an anti-c-Met antibody, and for example, selected from the group consisting of any mammals including primates such as human, and monkey; and rodents such as mice and rats. In one specific embodiment, the subject may be a cancer patient. The biological sample may be the subject, or a cell, a tissue, or body fluid (e.g., blood, serum, saliva, urinary, etc.) derived (separated) from the subject. Specifically, biological sample may be a cancer cell or a cancer tissue.

The ubiquitin peptidase may be any enzyme capable of inducing the degradation of ubiquitin *in vivo* or *ex vivo*. It may be USP (Ubiquitin specific peptidase), and specifically, USP8 (Ubiquitin specific peptidase 8). USP8 is a typical deubiquitinating enzyme, and associated with a degradation mechanism of a target by deubiquitination of various cell membrane proteins.

A gene for a ubiquitin peptidase which encodes a ubiquitin peptidase, for example, USP (Ubiquitin specific peptidase), specifically USP8, may be at least one selected from the group consisting of full-length DNA, cDNA, and mRNA.

It is believed that inhibition of the USP8 gene (for example, by knock-down) may induce a decreased interaction between USP8 and LRIG1, thereby promoting the ubiquitination of LRIG1, when an anti-c-Met antibody is applied as part of treatment, leading to accelerating c-Met degradation, which contributes to the increased anticancer effect of the anti-c-Met antibody. Therefore, in one embodiment USP8 and/or a gene encoding USP8 may be used as a biomarker for selecting a subject for application of an anti-c-Met antibody.

USP8 may be derived from mammals such as primates, including humans and monkeys, and also rodents, including mice and rats. For example, USP8 may be a human USP8 comprising the amino acid sequence of NCBI Accession No. NP_001122082 (SEQ ID NO: 109) or the amino acid sequence encoded by the nucleotide sequence (mRNA) of NM_001128610 (SEQ ID NO: 110; coding domain: from 339^{th} to 3695^{th} positions), a mouse USP8 comprising the amino acid sequence of NP_001239509 or the amino acid sequence encoded by the nucleotide sequence (mRNA) of NM_001252580 or BC066126.

The measurement of the absence/presence and the level of a ubiquitin peptidase and/or a gene for a ubiquitin peptidase, and the activity of a ubiquitin peptidase may be performed by measuring using any ordinary means for a gene or protein quantitative assay or for measuring an enzymatic activity, and/or by evaluating the measured results. For example, the absence/presence and the level of a ubiquitin peptidase (e.g., USP8) may be measured via an ordinary enzyme reaction, fluorescence, luminescence, and/or radioactivity detection using at least one selected from the group consisting of ubiquitin peptidase specific antibodies, and aptamers. More particularly, it may be measured by a method selected from the group consisting of immunochromatography, immunohistochemistry, enzyme linked immunosorbent assay (ELISA), radioimmunoassay (RIA), enzyme immunoassay (EIA), fluorescence immunoassay (FIA), luminescence immunoassay (LIA), and western blotting, but is not limited thereto. In addition, the absence/presence and the level of a gene for a ubiquitin peptidase may be measured by using any ordinary gene quantification methods including, but not limited to, an ordinary polymerase chain reaction (PCR), FISH (fluorescent in situ hybridization) using a primer, probe or aptamer, which is hybridizable with the gene.

In a particular embodiment, the primer may be able to detect a gene fragment of 5 to 1000 bp, 10 to 500 bp, 20 to 200 bp, or 50 to 200 bp within the nucleotide sequence of a gene coding for a ubiquitin peptidase, for example USP8 gene (full-length DNA, cDNA, or mRNA), and may comprise or consist essentially of a nucleotide sequence hybridizable with (complementary to) a region of 5 to 100 bp, 5 to 50 bp, 5 to 30 bp, or 10 to 25 bp of the 3'-end and/or 5'-end of the gene fragment. The probe or aptamer capable of hybridizing with the gene may comprise or consist essentially of a nucleotide sequence with a size of from about 5 to about 100 bp, from about 5 to about 50 bp, from about 5 to about 30bp, or from about 5 to about 25 bp, which is capable of hybridizing with (or complementary to) a fragment (about 5 to about 100 bp, about 5 to about 50 bp, about 5 to about 30bp, or about 5 to about 25 bp) of the USP8 gene (full-length DNA, cDNA or mRNA). As used herein, the term "capable of hybridizing" may refer to complementarily binding to a specific region of the gene, with a sequence complementarity of 80% or higher, e.g., 90% or higher, 95% or higher, 98% or higher, 99% or higher, or 100% between the primer, probe or aptamer and the gene region.

The means for detecting a ubiquitin peptidase and/or a ubiquitin peptidase coding gene and/or a means measuring the activity of a ubiquitin peptidase may be any ordinary means used in the above described methods for measuring the absence/presence or the level of a ubiquitin peptidase and/or a gene for a ubiquitin peptidase or the activity of a ubiquitin peptidase.

In a particular embodiment, the activity of a ubiquitin peptidase may be measured by determining the presence or absence of a mutation of a ubiquitin peptidase (e.g., USP8) in an active site affecting its activity, or the level of ubiquitination of a target. The activity may be a mutation where amino acid residue Cys at 786^{th} position of NP_001122082 (human USP8; SEQ ID NO: 109) is substituted with Ser (C786S). For example, the enzymatic activity of a ubiquitin peptidase may be determined by measuring the ubiquitination of a target, i.e., the presence or the level of the target (e.g., LRIG1) to which ubiquitin binds. The measurement of the presence or the level of the target (e.g., LRIG1) to which ubiquitin binds may be performed via an ordinary enzyme reaction, fluorescence, luminescence, and/or radioactivity detection using at least one selected from the group consisting of LRIG1 specific antibodies, and aptamers. Certain specific methods are as described above. In addition, the enzymatic activity of a ubiquitin peptidase may be determined by an ordinary means capable of confirming the presence of the mutation described above and/or a mutant including the mutation. Accordingly, it is possible to select a patient group having a high level of LRIG1 and a low level of USP8, determined by measuring the amounts of LRIG1 and USP from a protein sample (about 5 µg) or RNA sample (about 1 µg) extracted from only a small amount of tissue or cells. This can increase the clinical efficiency of clinical c-Met targeting anticancer therapy. The method of selecting a subject for application of the anti-c-Met antibody may be extended to HGF/c-Met inhibitors other than the anti-c-Met antibody as well.

Another embodiment provides a method of c-Met inhibition or an anti-c-Met antibody for use in a method of preventing and/or treating a cancer comprising administering an anti-c-Met antibody to the selected subject.

The method of c-Met inhibition or anti-c-Met antibody for use in the method of preventing and/or treating a cancer may further comprise a step of identifying a subject for application of the anti-c-Met antibody prior to the step of administration. The step of identification may be performed by the method of selecting a subject for application of the anti-c-Met antibody as described above.

In an embodiment, the method of c-Met inhibition or the anti-c-Met antibody for use in method of preventing and/or treating a cancer may include:
identifying a subject for the application of an anti-c-Met antibody; and
administering an effective amount of an anti-c-Met antibody to the subject.

In another embodiment, the method of c-Met inhibition or the method of preventing and/or treating a cancer may include:
selecting a subject for the application of an anti-c-Met antibody by measuring the absence/presence and the level of a ubiquitin peptidase (e.g., USP8) and/or a gene for a ubiquitin peptidase, and the activity of a ubiquitin peptidase (e.g., USP8); and
administering an effective amount of an anti-c-Met antibody to the selected subject.

In a therapy using an anti-c-Met antibody, when ubiquitin peptidase (e.g., USP8) and/or a gene for a ubiquitin peptidase is absent or present at a low level, or the activity of a ubiquitin peptidase (e.g., USP8) is low or lost, more excellent therapeutic effect can be achieved. Therefore, the co-administration of an anti-c-Met antibody with an inhibitor against a ubiquitin peptidase (e.g., USP8) and/or a gene for a ubiquitin peptidase may lead to a synergistic effect.

Another embodiment provides a pharmaceutical composition for combination administration for preventing and/or treating a cancer comprising an anti-c-Met antibody or an antigen-binding fragment thereof and an inhibitor against a ubiquitin peptidase and/or a ubiquitin peptidase coding gene as active ingredients.

In one embodiment, the pharmaceutical composition for combination administration may be in a form for simultaneous administration of two drugs including a mixture of a pharmaceutically effective amount of an anti-c-Met antibody and a pharmaceutically effective amount of an inhibitor against a ubiquitin peptidase and/or a ubiquitin peptidase coding gene.

In another embodiment, the pharmaceutical composition for combination administration may be in a form of simultaneous or sequential administration of a pharmaceutically effective amount of an anti-c-Met antibody and a pharmaceutically effective amount of an inhibitor against a ubiquitin peptidase and/or a ubiquitin peptidase coding gene, each being individually formulated. In this case, the pharmaceutical composition for combination administration may be a pharmaceutical composition for combination administration for simultaneous or sequential administration including a first pharmaceutical composition containing a pharmaceutically effective amount of an anti-c-Met antibody and a second pharmaceutical composition containing a pharmaceutically effective amount of an inhibitor against a ubiquitin peptidase and/or a ubiquitin peptidase coding gene. In the case of sequential administration, it can be performed in any order.

Another embodiment provides a kit for preventing and/or treating cancer, including a first pharmaceutical composition containing a pharmaceutically effective amount of an anti-c-Met antibody, a second pharmaceutical composition containing a pharmaceutically effective amount of an inhibitor against a ubiquitin peptidase and/or a ubiquitin peptidase coding gene, and a package container.

Another embodiment provides an anti-c-Met antibody or an antigen-bonding fragment thereof and a pharmaceutically effective amount of an inhibitor against a ubiquitin peptidase and/or a ubiquitin peptidase coding gene for use in a method for preventing and/or treating cancer comprising co-administering a pharmaceutically effective amount of an anti-c-Met antibody or an antigen-bonding fragment thereof and a pharmaceutically effective amount of an inhibitor against a ubiquitin peptidase and/or a ubiquitin peptidase coding gene to a subject in need of prevention and/or treatment of cancer. The method may further include a step of identifying a subject who is in need of the prevention and/or treatment of cancer, prior to the administration step.

The combination administration step may be performed either by administering an anti-c-Met antibody and an inhibitor against a ubiquitin peptidase and/or a ubiquitin peptidase coding gene together (at the same time) or by administering them sequentially in any order. In one embodiment, the combination administration may be performed by administering a mixture of a pharmaceutically effective amount of an anti-c-Met antibody and a pharmaceutically effective amount of an inhibitor against a ubiquitin peptidase and/or a ubiquitin peptidase coding gene. In another embodiment, the combination administration may be done by performing a first step of administering a pharmaceutically effective amount of an anti-c-Met antibody and a second step of administering a pharmaceutically effective amount of an inhibitor against a ubiquitin peptidase and/or a ubiquitin peptidase coding gene simultaneously or sequentially. In the case of sequential administration, it can be performed in any order.

The subject may be mammals such as primates, including humans and monkeys, and rodents, including mice and rats, or cells or tissues isolated from the living body thereof.

Another embodiment provides an anti-c-Met antibody and an inhibitor against a ubiquitin peptidase and/or a ubiquitin peptidase coding gene for the use of combination administration of the anti-c-Met antibody and the inhibitor against a ubiquitin peptidase and/or a ubiquitin peptidase coding gene in preventing and/or treating cancer.

In accordance with the invention, by co-administering an anti-c-Met antibody and an inhibitor against a ubiquitin peptidase and/or a ubiquitin peptidase coding gene, excellent synergistic effects can be achieved in comparison with the use of the anti-c-Met antibody alone. Furthermore, even when administration concentrations are decreased and/or administration intervals are extended, at least equivalent effects can be obtained in comparison with the use of a single drug, and side effects against the anti-c-Met antibody can be minimized.

The inhibitors against a ubiquitin peptidase and/or a ubiquitin peptidase coding gene may be any compound(s) capable of inhibiting the expression and/or activity of the ubiquitin peptidase and/or the gene. For example, in the case of the gene, the inhibitors may be one or more selected from the group consisting of a chemical inhibitor (compound drug) against the gene, an siRNA against the gene, a microRNA against the gene, an shRNA against the gene, an aptamer against the gene. For example, they may be one or more selected from the group consisting of an siRNA, microRNA, shRNA, and aptamer, all of which are capable of hybridizing with adjacent 2 to 200 bp, particularly 10 to 100 bp or 20 to 50 bp regions within the nucleotide sequences of the above genes. The 'capable of hybridizing' or 'hybridizable' refers to when complementary binding is possible by having sequence homology of at least 80%, at least 90%, at least 95%, at least 98%, at least 99%, or 100% to the nucleotide sequences of the above gene regions. Also, in the case of the ubiquitin peptidase, the inhibitors may be one or more selected from the group consisting of a mutant of the ubiquitin peptidase having a mutation in an active site (for example, a mutant containing a substitution of at least one amino acid within the active site of the ubiquitin peptidase with different amino acid), a chemical inhibitor (e.g., synthetic compound drug) against the ubiquitin peptidase, an antibody against the ubiquitin peptidase, or an aptamer against the gene or the ubiquitin peptidase,.

For example, the inhibitor may be at least one selected from the group consisting of shRNAs specifically binding to a gene encoding a ubiquitin peptidase, particularly USP8, for example, shRNA including the nucleotide sequence of 5'-tatctcttccgattatcag-3' (SEQ ID NO: 112); shUSP8 mature antisense). In another example, the inhibitor may be at least one selected from the group consisting of USP8-specific inhibitors such as HBX 90,397 or HBX 90,659 (see WO2007017758), a General de-ubiquitinating enzyme (DUB) inhibitor such as PR-619, but not limited thereto.

HBX 90,397 has the following structure:

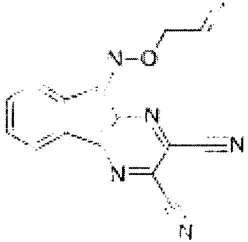

PR-619 has the following structure:

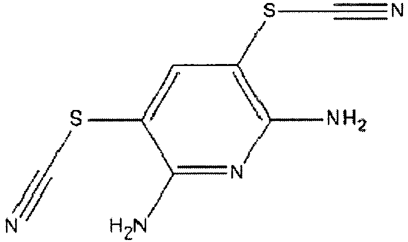

In another example, the inhibitor may be a mutant containing a mutation in an active site or a polynucleotide encoding the mutant. For example, the mutant may be a USP8 mutant (SEQ ID NO: 111) where amino acid residue Cys at 786^{th} position of USP8, for example NP_001122082 (human USP8; SEQ ID NO: 109) is substituted with Ser (C786S), or a polynucleotide encoding the mutant. The polynucleotide encoding the mutant may be inserted into a proper vector, and used for transfection (*in vivo* or *ex vivo*) by replacing a normal USP8 gene of a separated cell.

As described above, the selected subject for the application of an anti-c-Met antibody may be one having no or a low level of a ubiquitin peptidase and/or a gene for a ubiquitin peptidase or having no or a low level of ubiquitin peptidase activity a, wherein the no or low level of a ubiquitin peptidase and/or a gene for a ubiquitin peptidase or no or low enzymatic activity level may be inherent or achieved by artificial treatments for removing or lowering the ubiquitin peptidase and/or the gene or the enzymatic activity (e.g., mutation of USP8 as described above). Therefore, an artificial inhibition or mutation of a ubiquitin peptidase and/or a gene for a ubiquitin peptidase may make the subject suitable for the application of an anti-c-Met antibody or sensitive to the therapy using the anti-c-Met antibody.

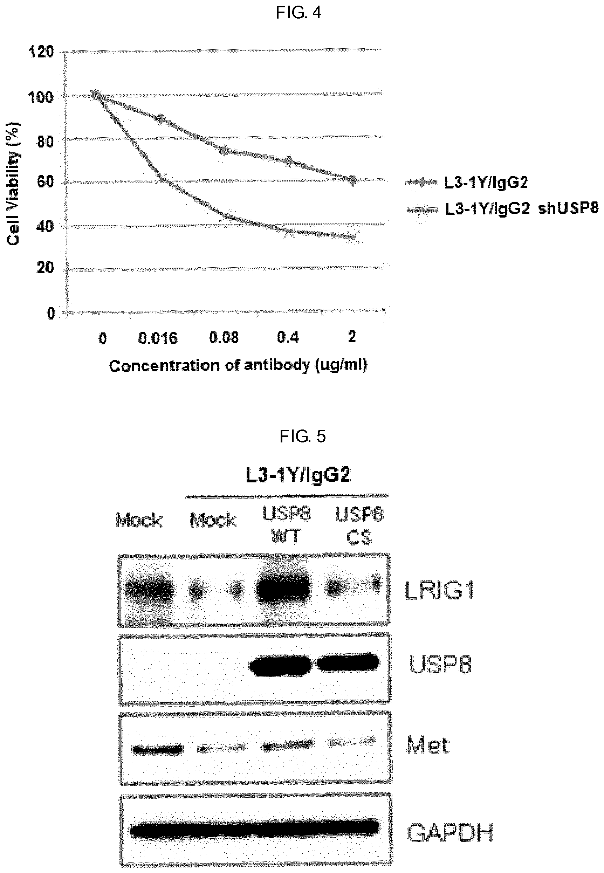

Therefore, another embodiment provides a composition for enhancing (increasing) an efficacy of an anti-c-Met antibody, including an inhibitor against a ubiquitin peptidase (e.g., USP8) and/or a gene for a ubiquitin peptidase as an active ingredient. Another embodiment provides a method of enhancing the efficacy of an anti-c-Met antibody including inhibiting a ubiquitin peptidase (e.g., USP8) and/or a gene for a ubiquitin peptidase. The step of inhibiting a ubiquitin peptidase and/or a gene for a ubiquitin peptidase may be performed by administering a pharmaceutical amount of an inhibitor against a ubiquitin peptidase (e.g., USP8) and/or a gene for a ubiquitin peptidase to a subject, or by mutating an active site of the ubiquitin peptidase or the gene for a ubiquitin peptidase. The subject may be one in need of treatment with an anti-c-Met antibody. Subjects may be mammals such as primates, including humans and monkeys, and rodents, including mice and rats, or cells or tissues isolated from the living body thereof. The subject may be a cancer patient, a cancer cell or a cancer tissue. Mutation in the active site of the ubiquitin peptidase or in the gene encoding the ubiquitin peptidase may be performed by substituting the amino acid residue Cys at 786^{th} position of USP8, for example human USP8 (NP_001122082; SEQ ID NO: 109) with Ser (C786S), or substituting the USP8 gene so as to encode the USP8 mutant (SEQ ID NO: 111). The mutation of the protein or the gene may be performed by any ordinary method in the relevant art. As shown in Example 5 and FIG. 5, an increase of USP8 leads to increased stability of LRIG1, whereas a mutation in an active site of USP8 decreases the stability of LRIG1, consequently increasing the activity of an anti-c-Met antibody.

The enhancement of the efficacy of an anti-c-Met antibody may include not only the increase of the anti-c-Met antibody's efficacy, i.e., the enhancement of the efficacy of cell internalization and/or degradation of c-Met, but also the decrease of side effects such as agonism of an anti-c-Met antibody.

The inhibitor against a ubiquitin peptidase and/or a gene for ubiquitin peptidase is as described above. In one embodiment, the inhibition of a ubiquitin peptidase and/or a gene for ubiquitin peptidase can be performed by knock-down of the gene for ubiquitin peptidase (e.g., USP8) using at least one selected from the group consisting of a chemical drug, siRNA, microRNA, shRNA, and aptamer, or by substitution or deletion of the gene. The substitution of the gene may refer to a substitution of at least one nucleotide of the cDNA or mRNA with different nucleotides, and the deletion of the gene may refer to the removal of the gene or a part thereof. Both the substitution and deletion of the gene may lead to inhibition of expression of a ubiquitin peptidase having its intact function. For example, the substitution of the gene may be performed so that the gene encodes the USP8 mutant as described above. For example, the substitution of the gene may be performed so that the codon which originally encodes the amino acid residue Cys at 786^{th} position of human USP8 (NP_001122082; SEQ ID NO: 109), encodes Ser instead.

Another embodiment provides a method for screening a (candidate) drug for preventing and/or treating a cancer using a ubiquitin peptidase and/or a ubiquitin peptidase coding gene.

The method for screening may include:
contacting (or treating) a candidate compound to a biological sample;
measuring a level of a ubiquitin peptidase and/or a ubiquitin peptidase coding gene in the biological sample; and
comparing the level of the ubiquitin peptidase and/or the ubiquitin peptidase coding gene in the biological sample contacted by (or treated with) the candidate compound to the level of the ubiquitin peptidase and/or the ubiquitin peptidase coding gene in a biological sample not contacted by (or treated) by the candidate compound.

The step of comparing may be performed by comparing the levels of the ubiquitin peptidase and/or the ubiquitin peptidase coding gene in the same biological sample measured before and after contact (or treatment) with the candidate compound, or by comparing the level of the ubiquitin peptidase and/or the ubiquitin peptidase coding gene in a part of the biological sample contacted by (or treated with) the candidate compound to that in other part of the biological sample not contacted by (or treated with) the candidate compound.

In cases where the level of the ubiquitin peptidase and/or the ubiquitin peptidase coding gene in the biological sample contacted by (or treated with) the candidate compound is decreased compared to that in the biological sample not contacted by (or treated with) the candidate compound, that is, in cases where it is confirmed that the candidate inhibits the ubiquitin peptidase and/or the ubiquitin peptidase coding gene, the candidate compound may be determined as a (candidate) drug for preventing and/or treating a cancer.

The biological sample may be a cell or tissue separated from a living body of a mammal including human, and for example, it may be a cancer cell or a cancer tissue.

The candidate compound may be at least one selected from the group consisting of various kinds of compounds, for example, proteins, polypeptides, oligopeptides, polynucleotides, oligonucleotides, and other various chemical materials.

The step of measuring the level of the ubiquitin peptidase and/or the ubiquitin peptidase coding gene in the biological sample may be performed by a measurement using an ordinary means for detection and/or quantification of a protein or a gene, and/or by analysis of the measurement results. Means for detection and/or quantification of a protein or a gene are as described above.

A drug for preventing and/or treating a cancer screened by the above method may exhibit an increased synergistic effect by being co-administered with an anti-c-Met antibody. In addition, due to the synergistic effect, it is possible to reduce the administration amount of the anti-c-Met antibody, thereby decreasing side effects thereof. Therefore, the drug for preventing and/or treating a cancer screened by the above method is a good partner drug for co-administration with an anti-c-Met antibody, that is, the drug is suitable for use in a combination therapy using an anti-c-Met antibody.

In an embodiment, the anti c-Met antibody may be any antibody and/or antigen-binding fragment thereof, which recognizes c-Met protein as an antigen. In particular, the anti c-Met antibody may recognize a specific region of c-Met, e.g., a specific region in the SEMA domain, as an epitope. It may be any antibody or antigen-binding fragment that acts on c-Met to induce c-Met intracellular internalization and degradation.

As used herein, unless otherwise stated, the term "anti-c-Met antibody" may be used to include not only an complete antibody but also an antigen-binding fragment thereof.

The term "c-Met" or "c-Met protein" refers to a receptor tyrosine kinase (RTK) which binds hepatocyte growth factor (HGF). c-Met may be a c-Met protein from any species, particularly a mammal, for instance, primates such as human c-Met (e.g., NP_000236 ) or monkey c-Met (e.g., Macaca mulatta, NP_001162100), or rodents such as mouse c-Met (e.g., NP_032617.2) or rat c-Met (e.g., NP_113705.1). The c-Met protein may include a polypeptide encoded by the nucleotide sequence identified as GenBank Accession Number NM_000245, a polypeptide having the amino acid sequence identified as GenBank Accession Number NP_000236 or extracellular domains thereof. The receptor tyrosine kinase c-Met participates in various mechanisms, such as cancer development, metastasis, migration of cancer cell, invasion of cancer cell, and angiogenesis.

c-Met, a receptor for hepatocyte growth factor (HGF), may be divided into three portions: extracellular, transmembrane, and intracellular. The extracellular portion is composed of an α (alpha)-subunit and a β (beta)-subunit which are linked to each other through a disulfide bond, and contains a SEMA domain responsible for binding HGF, a PSI domain (plexin-semaphorin-integrin homology domain) and an IPT domain (immunoglobulin-like fold shared by plexins and transcriptional factors domain). The SEMA domain of c-Met protein may have the amino acid sequence of SEQ ID NO: 79, and is an extracellular domain that functions to bind HGF. A specific region of the SEMA domain, that is, a region having the amino acid sequence of SEQ ID NO: 71, which corresponds to a range from amino acid residues 106 to 124 of the amino acid sequence of the SEMA domain (SEQ ID NO: 79) of c-Met protein, is a loop region between the second and the third propellers within the epitopes of the SEMA domain. The region acts as an epitope for the specific anti-c-Met antibody of the present disclosure.

The term "epitope" as used herein, refers to an antigenic determinant, a part of an antigen recognized by an antibody. In one embodiment, the epitope may be a region comprising 5 or more contiguous (consecutive or non-consecutive) amino acid residues within the SEMA domain (SEQ ID NO: 79) of c-Met protein, for instance, 5 to 19 contiguous amino acid residues within the amino acid sequence of SEQ ID NO: 71. For example, the epitope may be a polypeptide having 5 to 19 contiguous amino acids selected from among partial combinations of the amino acid sequence of SEQ ID NO: 71, wherein the polypeptide essentially includes the amino sequence of SEQ ID NO: 73 (EEPSQ) serving as an essential element for the epitope. For example, the epitope may be a polypeptide comprising, consisting essentially of, or consisting of the amino acid sequence of SEQ ID NO: 71, SEQ ID NO: 72, or SEQ ID NO: 73.

The epitope having the amino acid sequence of SEQ ID NO: 72 corresponds to the outermost part of the loop between the second and third propellers within the SEMA domain of a c-Met protein. The epitope having the amino acid sequence of SEQ ID NO: 73 is a site to which the antibody or antigen-binding fragment according to one embodiment most specifically binds.

Thus, the anti-c-Met antibody may specifically bind to an epitope which has 5 to 19 contiguous amino acids selected from the amino acid sequence of SEQ ID NO: 71, including SEQ ID NO: 73 (EEPSQ) as an essential element. For example, the anti-c-Met antibody may specifically bind to an epitope including the amino acid sequence of SEQ ID NO: 71, SEQ ID NO: 72, or SEQ ID NO: 73.

In one embodiment, the anti-c-Met antibody or an antigen-binding fragment thereof may comprise or consist essentially of:
at least one heavy chain complementarity determining region (CDR) selected from the group consisting of (a) a CDR-H1 comprising the amino acid sequence of SEQ ID NO: 4; (b) a CDR-H2 comprising the amino acid sequence of SEQ ID NO: 5, SEQ ID NO: 2, or an amino acid sequence comprising 8-19 consecutive amino acids within SEQ ID NO: 2 including amino acid residues from the 3^{rd} to 10^{th} positions of SEQ ID NO: 2; and (c) a CDR-H3 comprising the amino acid sequence of SEQ ID NO: 6, SEQ ID NO: 85, or an amino acid sequence comprising 6-13 consecutive amino acids within SEQ ID NO: 85 including amino acid residues from the 1^{st} to 6^{th} positions of SEQ ID NO: 85, or a heavy chain variable region comprising the at least one heavy chain complementarity determining region;
at least one light chain complementarity determining region (CDR) selected from the group consisting of (a) a CDR-L1 comprising the amino acid sequence of SEQ ID NO: 7, (b) a CDR-L2 comprising the amino acid sequence of SEQ ID NO: 8, and (c) a CDR-L3 comprising the amino acid sequence of SEQ ID NO: 9, SEQ ID NO: 86, or an amino acid sequence comprising 9-17 consecutive amino acids within SEQ ID NO: 89 including amino acid residues from the 1^{st} to 9^{th} positions of SEQ ID NO: 89, or a light chain variable region comprising the at least one light chain complementarity determining region;
a combination of the at least one heavy chain complementarity determining region and at least one light chain complementarity determining region; .or
a combination of the heavy chain variable region and the light chain variable region.

Herein, the amino acid sequences of SEQ ID NOS: 4 to 9 are respectively represented by following Formulas I to VI, below:

Formula I Xaa₁-Xaa₂-Tyr-Tyr-Met-Ser (SEQ ID NO: 4),

wherein Xaa₁ is absent or Pro or Ser, and Xaa₂ is Glu or Asp,

Formula II Arg-Asn-Xaa₃-Xaa₄-Asn-Gly-Xaa₅-Thr (SEQ ID NO: 5),

wherein Xaa₃ is Asn or Lys, Xaa₄ is Ala or Val, and Xaa₅ is Asn or Thr,

Formula III Asp-Asn-Trp-Leu-Xaa₆-Tyr (SEQ ID NO: 6),

wherein Xaa₆ is Ser or Thr,

Formula IV Lys-Ser-Ser-Xaa₇-Ser-Leu-Leu-Ala-Xaa₈-Gly-Asn-Xaa₉-Xaa₁₀-Asn-Tyr-Leu-Ala (SEQ ID NO: 7)

wherein Xaa₇ is His, Arg, Gln, or Lys, Xaa₈ is Ser or Trp, Xaa₉ is His or Gln, and Xaa₁₀ is Lys or Asn,

Formula V Trp-Xaa₁₁-Ser-Xaa₁₂-Arg-Val-Xaa₁₃ (SEQ ID NO: 8)

wherein Xaa₁₁ is Ala or Gly, Xaa₁₂ is Thr or Lys, and Xaa₁₃ is Ser or Pro, and

Formula VI Xaa₁₄-Gln-Ser-Tyr-Ser-Xaa₁₅-Pro-Xaa₁₆-Thr (SEQ ID NO: 9)

wherein Xaa₁₄ is Gly, Ala, or Gln, Xaa₁₅ is Arg, His, Ser, Ala, Gly, or Lys, and Xaa₁₆ is Leu, Tyr, Phe, or Met.

In one embodiment, the CDR-H1 may comprise or consist essentially of an amino acid sequence selected from the group consisting of SEQ ID NOS: 1, 22, 23, and 24. The CDR-H2 may comprise or consist essentially of an amino acid sequence selected from the group consisting of SEQ ID NOS: 2, 25, and 26. The CDR-H3 may comprise or consist essentially of an amino acid sequence selected from the group consisting of SEQ ID NOS: 3, 27, 28, and 85.

The CDR-L1 may comprise or consist essentially of an amino acid sequence selected from the group consisting of SEQ ID NOS: 10, 29, 30, 31, 32, 33, and 106. The CDR-L2 may comprise or consist essentially of an amino acid sequence selected from the group consisting of SEQ ID NOS: 11, 34, 35, and 36. The CDR-L3 may comprise or consist essentially of an amino acid sequence selected from the group consisting of SEQ ID NOS: 12, 13, 14, 15, 16, 37, 86, and 89.

In another embodiment, the antibody or antigen-binding fragment may include a heavy chain variable region comprising a polypeptide (CDR-H1) including an amino acid sequence selected from the group consisting of SEQ ID NOS: 1, 22, 23, and 24, a polypeptide (CDR-H2) including an amino acid sequence selected from the group consisting of SEQ ID NOS: 2, 25, and 26, and a polypeptide (CDR-H3) including an amino acid sequence selected from the group consisting of SEQ ID NOS: 3, 27, 28, and 85; and a light chain variable region comprising a polypeptide (CDR-L1) including an amino acid sequence selected from the group consisting of SEQ ID NOS: 10, 29, 30, 31, 32, 33 and 106, a polypeptide (CDR-L2) including an amino acid sequence selected from the group consisting of SEQ ID NOS: 11, 34, 35, and 36, and a polypeptide (CDR-L3) including an amino acid sequence selected from the group consisting of SEQ ID NOS 12, 13, 14, 15, 16, 37, 86, and 89.

In one embodiment of the anti-c-Met antibody or antigen-binding fragment, the variable region of the heavy chain includes the amino acid sequence of SEQ ID NO: 17, 74, 87, 90, 91, 92, 93, or 94 and the variable region of the light chain includes the amino acid sequence of SEQ ID NO: 18, 19, 20, 21, 75, 88, 95, 96, 97, 98, 99, or 107.

Animal-derived antibodies produced by immunizing non-immune animals with a desired antigen generally invoke immunogenicity when injected to humans for the purpose of medical treatment, and thus chimeric antibodies have been developed to inhibit such immunogenicity. Chimeric antibodies are prepared by replacing constant regions of animal-derived antibodies that cause an anti-isotype response with constant regions of human antibodies by genetic engineering. Chimeric antibodies are considerably improved in terms of anti-isotype response compared to animal-derived antibodies, but animal-derived amino acids still have variable regions, so that chimeric antibodies have side effects with respect to a potential anti-idiotype response. Humanized antibodies have been developed to reduce such side effects. Humanized antibodies are produced by grafting complementarity determining regions (CDR) which serve an important role in antigen binding in variable regions of chimeric antibodies into a human antibody framework.

In using CDR grafting to produce humanized antibodies, choosing which optimized human antibodies to use for accepting CDRs of animal-derived antibodies is critical. Antibody databases, analysis of a crystal structure, and technology for molecule modeling are used. However, even when the CDRs of animal-derived antibodies are grafted to the most optimized human antibody framework, amino acids positioned in a framework of the animal-derived CDRs affecting antigen binding are present. Therefore, in many cases, antigen binding affinity is not maintained, and thus application of additional antibody engineering technology for recovering the antigen binding affinity is necessary.

The anti c-Met antibodies may be, but are not limited to, animal antibodies (e.g., mouse-derived antibodies), chimeric antibodies (e.g., mouse-human chimeric antibodies), humanized antibodies, or human antibodies. The antibodies or antigen-binding fragments thereof may be isolated from a living body or non-naturally occurring. The antibodies or antigen-binding fragments thereof may be synthetic or recombinant.

An intact antibody includes two full-length light chains and two full-length heavy chains, in which each light chain is linked to a heavy chain by disulfide bonds. The antibody has a heavy chain constant region and a light chain constant region. The heavy chain constant region is of a gamma (γ), mu (µ), alpha (α), delta (δ), or epsilon (ε) type, which may be further categorized as gamma 1 (γ1), gamma 2(γ2), gamma 3(γ3), gamma 4(γ4), alpha 1(α1), or alpha 2(α2). The light chain constant region is of either a kappa (κ) or lambda (λ) type.

As used herein, the term "heavy chain" refers to full-length heavy chain, and fragments thereof, including a variable region V_{H} that includes amino acid sequences sufficient to provide specificity to antigens, and three constant regions, C_{H1}, C_{H2}, and C_{H3}, and a hinge. The term "light chain" refers to a full-length light chain and fragments thereof, including a variable region V_{L} that includes amino acid sequences sufficient to provide specificity to antigens, and a constant region C_{L}.

The term "complementarity determining region (CDR)" refers to an amino acid sequence found in a hyper variable region of a heavy chain or a light chain of immunoglobulin. The heavy and light chains may respectively include three CDRs (CDRH1, CDRH2, and CDRH3; and CDRL1, CDRL2, and CDRL3). The CDR may provide contact residues that play an important role in the binding of antibodies to antigens or epitopes. The terms "specifically binding" and "specifically recognized" are well known to one of ordinary skill in the art, and indicate that an antibody and an antigen specifically interact with each other to lead to an immunological activity.

The term "antigen-binding fragment" used herein refers to fragments of an intact immunoglobulin including portions of a polypeptide including antigen-binding regions having the ability to specifically bind to the antigen. In a particular embodiment, the antigen-binding fragment may be scFv, (scFv)₂, scFvFc, Fab, Fab', or F(ab')₂, but is not limited thereto.

Among the antigen-binding fragments, Fab that includes light chain and heavy chain variable regions, a light chain constant region, and a first heavy chain constant region C_{H1}, has one antigen-binding site.

The Fab' fragment is different from the Fab fragment, in that Fab' includes a hinge region with at least one cysteine residue at the C-terminal of C_{H1}.

The F(ab')₂ antibody is formed through disulfide bridging of the cysteine residues in the hinge region of the Fab' fragment.

Fv is the smallest antibody fragment with only a heavy chain variable region and a light chain variable region. Recombination techniques of generating the Fv fragment are widely known in the art.

Two-chain Fv includes a heavy chain variable region and a light chain region which are linked by a non-covalent bond. Single-chain Fv generally includes a heavy chain variable region and a light chain variable region which are linked by a covalent bond via a peptide linker or linked at the C-terminals to have a dimer structure like the two-chain Fv. The peptide linker may be the same as described above, including, but not limited to, those having an amino acid length of 1 to 100, 2 to 50, particularly 5 to 25, and any kinds of amino acids may be included without any restrictions.

The antigen-binding fragments may be obtained using protease (for example, the Fab fragment may be obtained by restricted cleavage of a whole antibody with papain, and the F(ab')₂ fragment may be obtained by cleavage with pepsin), or may be prepared by using a genetic recombination technique.

The term "hinge region," as used herein, refers to a region between CH1 and CH2 domains within the heavy chain of an antibody which functions to provide flexibility for the antigen-binding site.

When an animal antibody undergoes a chimerization process, the IgG1 hinge of animal origin is replaced with a human IgG1 hinge or IgG2 hinge while the disulfide bridges between two heavy chains are reduced from three to two in number. In addition, an animal-derived IgG1 hinge is shorter than a human IgG1 hinge. Accordingly, the rigidity of the hinge is changed. Thus, a modification of the hinge region may bring about an improvement in the antigen binding efficiency of the humanized antibody. The modification of the hinge region through amino acid deletion, addition, or substitution is well-known to those skilled in the art.

In one embodiment, the anti-c-Met antibody or an antigen-binding fragment thereof may be modified by any combination of deletion, insertion, addition, or substitution of at least one amino acid residue on the amino acid sequence of the hinge region so that it exhibit enhanced antigen-binding efficiency. For example, the antibody may include a hinge region including the amino acid sequence of SEQ ID NO: 100(U7-HC6), 101(U6-HC7), 102(U3-HC9), 103(U6-HC8), or 104(U8-HC5), or a hinge region including the amino acid sequence of SEQ ID NO: 105 (non-modified human hinge). In particular, the hinge region has the amino acid sequence of SEQ ID NO: 100 or 101.

In one embodiment, the anti-c-Met antibody may be a monoclonal antibody. The monoclonal antibody may be produced by the hybridoma cell line deposited with Accession No. KCLRF-BP-00220, which binds specifically to the extracellular region of c-Met protein (refer to Korean Patent Publication No. 2011-0047698). The anti-c-Met antibody may include all the antibodies defined in Korean Patent Publication No. 2011-0047698.

In the anti-c-Met antibody, the rest portion of the light chain and the heavy chain portion except the CDRs, the light chain variable region, and the heavy chain variable region as defined above, for example, the light chain constant region and the heavy chain constant region, may be those from any subtype of immunoglobulin (e.g., IgA, IgD, IgE, IgG (IgG1, IgG2, IgG3, IgG4), IgM, and the like).

By way of further example, the anti-c-Met antibody or the antibody fragment may include:
a heavy chain including the amino acid sequence selected from the group consisting of the amino acid sequence of SEQ ID NO: 62 (wherein the amino acid sequence from amino acid residues from the 1^{st} to 17^{th} positions is a signal peptide), or the amino acid sequence from the 18^{th} to 462^{nd} positions of SEQ ID NO: 62, the amino acid sequence of SEQ ID NO: 64 (wherein the amino acid sequence from the 1^{st} to 17^{th} positions is a signal peptide), the amino acid sequence from the 18^{th} to 461^{st} positions of SEQ ID NO: 64, the amino acid sequence of SEQ ID NO: 66 (wherein the amino acid sequence from the 1^{st} to 17^{th} positions is a signal peptide), and the amino acid sequence from the 18^{th} to 460^{th} positions of SEQ ID NO: 66; and
a light chain including the amino acid sequence selected from the group consisting of the amino acid sequence of SEQ ID NO: 68 (wherein the amino acid sequence from the 1^{st} to 20^{th} positions is a signal peptide), the amino acid sequence from the 21^{st} to 240^{th} positions of SEQ ID NO: 68, the amino acid sequence of SEQ ID NO: 70 (wherein the amino acid sequence from the 1^{st} to 20^{th} positions is a signal peptide), the amino acid sequence from the 21^{st} to 240^{th} positions of SEQ ID NO: 70, and the amino acid sequence of SEQ ID NO: 108.

For example, the anti-c-Met antibody may be selected from the group consisting of:
an antibody including a heavy chain including the amino acid sequence of SEQ ID NO: 62 or the amino acid sequence from the 18^{th} to 462^{nd} positions of SEQ ID NO: 62 and a light chain including the amino acid sequence of SEQ ID NO: 68 or the amino acid sequence from the 21^{st} to 240^{th} positions of SEQ ID NO: 68;
an antibody including a heavy chain including the amino acid sequence of SEQ ID NO: 64 or the amino acid sequence from the 18^{th} to 461^{st} positions of SEQ ID NO: 64 and a light chain including the amino acid sequence of SEQ ID NO: 68 or the amino acid sequence from the 21^{st} to 240^{th} positions of SEQ ID NO: 68;
an antibody including a heavy chain including the amino acid sequence of SEQ ID NO: 66 or the amino acid sequence from the 18^{th} to 460^{th} positions of SEQ ID NO: 66 and a light chain including the amino acid sequence of SEQ ID NO: 68 or the amino acid sequence from the 21^{st} to 240^{th} positions of SEQ ID NO: 68;
an antibody including a heavy chain including the amino acid sequence of SEQ ID NO: 62 or the amino acid sequence from the 18^{th} to 462^{nd} positions of SEQ ID NO: 62 and a light chain including the amino acid sequence of SEQ ID NO: 70 or the amino acid sequence from the 21^{st} to 240^{th} positions of SEQ ID NO: 70;
an antibody including a heavy chain including the amino acid sequence of SEQ ID NO: 64 or the amino acid sequence from the 18^{th} to 461^{st} positions of SEQ ID NO: 64 and a light chain including the amino acid sequence of SEQ ID NO: 70 or the amino acid sequence from the 21^{st} to 240^{th} positions of SEQ ID NO: 70;
an antibody including a heavy chain including the amino acid sequence of SEQ ID NO: 66 or the amino acid sequence from the 18^{th} to 460^{th} positions of SEQ ID NO: 66 and a light chain including the amino acid sequence of SEQ ID NO: 70 or the amino acid sequence from the 21^{st} to 240^{th} positions of SEQ ID NO: 70;
an antibody including a heavy chain including the amino acid sequence of SEQ ID NO: 62 or the amino acid sequence from the 18^{th} to 462^{nd} positions of SEQ ID NO: 62 and a light chain including the amino acid sequence of SEQ ID NO: 108;
an antibody including a heavy chain including the amino acid sequence of SEQ ID NO: 64 or the amino acid sequence from the 18^{th} to 461^{st} positions of SEQ ID NO: 64 and a light chain including the amino acid sequence of SEQ ID NO: 108; and
an antibody including a heavy chain including the amino acid sequence of SEQ ID NO: 66 or the amino acid sequence from the 18^{th} to 460^{th} positions of SEQ ID NO: 66 and a light chain including the amino acid sequence of SEQ ID NO: 108.

According to an embodiment, the anti-c-Met antibody may include a heavy chain including the amino acid sequence from the 18^{th} to 460^{th} positions of SEQ ID NO: 66 and a light chain including the sequence from the 21^{st} to 240^{th} positions of SEQ ID NO: 68, or a heavy chain including the amino acid sequence from the 18^{th} to 460^{th} positions of SEQ ID NO: 66 and a light chain including the sequence of SEQ ID NO: 108.

The polypeptide with the amino acid sequence of SEQ ID NO: 108 is a polypeptide obtained by replacing serine at position 32 (position 27e according to kabat numbering in the amino acid sequence from amino acid residues 21 to 240 of SEQ ID NO: 68; positioned within CDR-L1) with tryptophan. By such replacement, antibodies and antibody fragments including such sequences exhibits increased activities, such as c-Met biding affinity, c-Met degradation activity, and Akt phosphorylation inhibition.

In another embodiment, the anti-c-Met antibody may include a light chain complementarity determining region including the amino acid sequence of SEQ ID NO: 106, a light chain variable region including the amino acid sequence of SEQ ID NO: 107, or a light chain including the amino acid sequence of SEQ ID NO: 108.

The mixture wherein a pharmaceutically effective amount of an anti-c-Met antibody or an antigen-binding fragment thereof and a pharmaceutically effective amount of an inhibitor against a ubiquitin peptidase and/or a ubiquitin peptidase coding gene are mixed, the first pharmaceutical composition containing a pharmaceutically effective amount of an anti-c-Met antibody or an antigen-binding fragment thereof as an active ingredient and the second pharmaceutical composition containing a pharmaceutically effective amount an inhibitor against a ubiquitin peptidase and/or a ubiquitin peptidase coding gene as an active ingredient, may be provided (or administered) along with a pharmaceutically acceptable carrier, diluent, and/or excipient.

The pharmaceutically acceptable carrier to be included in the mixture or the pharmaceutical composition may be those commonly used for the formulation of antibodies, which may be one or more selected from the group consisting of lactose, dextrose, sucrose, sorbitol, mannitol, starch, gum acacia, calcium phosphate, alginates, gelatin, calcium silicate, micro-crystalline cellulose, polyvinylpyrrolidone, cellulose, water, syrup, methyl cellulose, methylhydroxy benzoate, propylhydroxy benzoate, talc, magnesium stearate, and mineral oil, but are not limited thereto. The pharmaceutical composition may further include one or more selected from the group consisting of a lubricant, a wetting agent, a sweetener, a flavor enhancer, an emulsifying agent, a suspension agent, and preservative.

The pharmaceutical composition, the mixture, or each active ingredient may be administered orally or parenterally. The parenteral administration may include intravenous injection, subcutaneous injection, muscular injection, intraperitoneal injection, endothelial administration, local administration, intranasal administration, intrapulmonary administration, and rectal administration. Since oral administration leads to digestion of proteins or peptides, an active ingredient in the compositions for oral administration must be coated or formulated to prevent digestion in the stomach. In addition, the compositions may be administered using an optional device that enables an active substance to be delivered to target cells.

The term "the pharmaceutically effective amount" as used in this specification refers to an amount at which each active ingredient can exert pharmaceutically significant effects.

For one-time administration, a pharmaceutically effective amount of an anti-c-Met antibody or an antigen-binding fragment thereof and a pharmaceutically effective amount of an inhibitor against the target substance may be prescribed in a variety way, depending on many factors including formulation methods, administration manners, ages of patients, body weight, gender, pathologic conditions, diets, administration time, administration interval, administration route, excretion speed, and reaction sensitivity. For example, the effective amount of the inhibitor against the target substance (e.g., a ubiquitin peptidase and/or its coding gene) may be, but not limited to, in ranges of 0.001 to 100 mg/kg, or 0.02 to 10 mg/kg for one-time administration and the effective amount of the anti-c-Met antibodies or antigen binding fragments thereof may be, but not limited to, in ranges of 0.001 to 100 mg/kg, or 0.02 to 10 mg/kg for their one-time administration.

The effective amount for one-time administration may be formulated into a single formulation in a unit dosage form or formulated in suitably divided dosage forms, or it may be manufactured to be contained in a multiple dosage container. For the kit, the effective amount of the inhibitor against the target substance and the effective amount of the anti-c-Met antibodies or antigen binding fragments thereof for one-time administration (single dose) may be contained in a package container as a base unit.

The administration interval between the administrations is defined as a period between the first administration and the following administration. The administration interval may be, but is not limited to, 24 hours to 30 days (e.g., 10 hours, 15 hours, 20 hours, 1 day, 2 days, 3 days, 4 days, 5 days, 6, days, 7 days, 10 days, 14 days, 21 days, or 28 days) and particularly 7 to 14 days or so. For the combined therapy, the first pharmaceutical composition containing a pharmaceutically effective amount of an anti-c-Met antibody or an antigen-binding fragment thereof as an active ingredient, and the second pharmaceutical composition containing a pharmaceutically effective amount of an inhibitor against a ubiquitin peptidase and/or a ubiquitin peptidase coding gene as an active ingredient may be co-administered in a given time interval (e.g., several minutes, several hours or several days, or several weeks) to be determined by considerations like the type of disease, and a patient's condition. For example, the first pharmaceutical composition and the second pharmaceutical composition may be simultaneously administered (administration interval within 1 minute) or sequentially administered (administration interval of 1 minute or over), and in case of sequential administration, the administration interval between the first pharmaceutical composition and the second pharmaceutical composition may be 1 to 60 minutes, particularly, 1 minute to 10 minutes, and they may be administered in any order.

The combined mixture or the pharmaceutical compositions may be a solution in oil or an aqueous medium, a suspension, a syrup, an emulsifying solution form, or they may be formulated into a form of an extract, elixirs, powders, granules, a tablet or a capsule, and they may further include a dispersing agent or a stabilizing agent in their formulation.

The pharmaceutical composition containing the anti-c-Met antibody or antigen binding fragments thereof may be formulated into an immunoliposome since it contains an antibody or an antigen binding fragment. A liposome containing an antibody may be prepared using any methods well known in the pertinent field. The immunoliposome may be a lipid composition including phosphatidylcholine, cholesterol, and polyethyleneglycol-derived phosphatidylethanolamine, and may be prepared by a reverse phase evaporation method. For example, Fab' fragments of an antibody may be conjugated to the liposome through a disulfide-exchange reaction. A chemical drug, such as doxorubicin, may further be included in the liposome.

The pharmaceutical compositions or the method may be used for the prevention and/or treatment of a cancer. The cancer may be associated with overexpression and/or (abnormal) activation of c-Met. The cancer may be a solid cancer or a blood cancer. For example, the cancer may be at least one selected from the group consisting of squamous cell carcinoma, small-cell lung cancer, non-small-cell lung cancer, adenocarcinoma of the lung, squamous cell carcinoma of the lung, peritoneal carcinoma, skin cancer, melanoma in the skin or eyeball, rectal cancer, cancer near the anus, esophagus cancer, small intestinal tumor, endocrine gland cancer, parathyroid cancer, adrenal cancer, soft-tissue sarcoma, urethral cancer, chronic or acute leukemia, lymphocytic lymphoma, hepatoma, gastrointestinal cancer, gastric cancer, pancreatic cancer, glioblastoma, cervical cancer, ovarian cancer, liver cancer, bladder cancer, hepatocellular adenoma, breast cancer, colon cancer, large intestine cancer, endometrial carcinoma or uterine carcinoma, salivary gland tumor, kidney cancer, prostate cancer, vulvar cancer, thyroid cancer, head and neck cancers, osteosarcoma, and brain cancer, but is not limited thereto.

The prevention and/or treatment effects of the cancers may include effects of not only suppressing the growth of the cancer cells but also suppressing progression of cancers due to migration, invasion, and metastasis thereof. Therefore, the cancers curable by the combined therapy of the disclosure include both primary cancers and metastatic cancers.

The aforementioned activities of a ubiquitin peptidase (e.g., USP8) and/or a gene encoding the same may be extended to HGF/c-Met inhibitors as well as anti-c-Met antibodies.

As described above, the finding of the use of a ubiquitin peptidase (e.g., USP8) and/or a gene encoding the same as a biomarker for HGF/c-Met inhibitor such as anti-c-Met antibodies can lead to the following effects: the efficacy of an anti-c-Met antibody can be improved by knock-down of USP8 in patients having high level of a ubiquitin peptidase (e.g., USP8) and/or a gene encoding the same, and agonism of anti-c-Met antibodies can be prevented by selecting patients having high level of LRIG1 and low level of USP8.

The present invention can be applied to diseases which are associated with c-Met/HGF signal transduction pathway and ubiquitination pathway, other than cancers.

### EXAMPLES

Hereafter, the present invention will be described in detail by examples.

The following examples are intended merely to illustrate the invention and are not construed to restrict the invention.

### Reference Example 1: Construction of Anti-c-Met Antibody

### 1.1. Production of "AbF46", a Mouse Antibody to c-Met

### 1.1.1. Immunization of mouse

To obtain immunized mice necessary for the development of a hybridoma cell line, each of five BALB/c mice (Japan SLC, Inc.), 4 to 6 weeks old, was intraperitoneally injected with a mixture of 100 µg of human c-Met/Fc fusion protein (R&D Systems) and one volume of complete Freund's adjuvant. Two weeks after the injection, a second intraperitoneal injection was conducted on the same mice with a mixture of 50 µg of human c-Met/Fc protein and one volume of incomplete Freund's adjuvant. One week after the second immunization, the immune response was finally boosted. Three days later, blood was taken from the tails of the mice and the sera were 1/1000 diluted in PBS and used to examine a titer of antibody to c-Met by ELISA. Mice found to have a sufficient antibody titer were selected for use in the cell fusion process.

### 1.1.2. Cell fusion and production of hybridoma

Three days before cell fusion, BALB/c mice (Japan SLC, Inc.) were immunized with an intraperitoneal injection of a mixture of 50 µg of human c-Met/Fc fusion protein and one volume of PBS. The immunized mice were anesthetized before excising the spleen from the left half of the body. The spleen was meshed to separate splenocytes which were then suspended in a culture medium (DMEM, GIBCO, Invitrogen). The cell suspension was centrifuged to recover the cell layer. The splenocytes thus obtained (1x10⁸ cells) were mixed with myeloma cells (Sp2/0) (1x10⁸ cells), followed by spinning to give a cell pellet. The cell pellet was slowly suspended, treated with 45% polyethylene glycol (PEG) (1 mL) in DMEM for 1 min at 37 °C, and supplemented with 1 mL of DMEM. To the cells was added 10 mL of DMEM over 10 min, after which incubation was conducted in a water bath at 37 °C for 5 min. Then the cell volume was adjusted to 50 mL before centrifugation. The cell pellet thus formed was resuspended at a density of 1~2×10⁵ cells/mL in a selection medium (HAT medium) and 0.1 mL of the cell suspension was allocated to each well of 96-well plates which were then incubated at 37 °C in a CO₂ incubator to establish a hybridoma cell population.

### 1.1.3. Selection of hybridoma cells producing monoclonal antibodies to c-Met protein

From the hybridoma cell population established in Reference Example 1.1.2, hybridoma cells which showed a specific response to c-Met protein were screened by ELISA using human c-Met/Fc fusion protein and human Fc protein as antigens.

Human c-Met/Fc fusion protein was seeded in an amount of 50 µL (2 µg/mL)/well to microtiter plates and allowed to adhere to the surface of each well. The antibody that remained unbound was removed by washing. For use in selecting the antibodies that do not bind c-Met but recognize Fc, human Fc protein was attached to the plate surface in the same manner.

The hybridoma cell culture obtained in Reference Example 1.1.2 was added in an amount of 50 µL to each well of the plates and incubated for 1 hour. The cells remaining unreacted were washed out with a sufficient amount of Tris-buffered saline and Tween 20 (TBST). Goat anti-mouse IgG-horseradish peroxidase (HRP) was added to the plates and incubated for 1 hour at room temperature. The plates were washed with a sufficient amount of TBST, followed by reacting the peroxidase with a substrate (OPD). Absorbance at 450 nm was measured on an ELISA reader.

Hybridoma cell lines which secrete antibodies that specifically and strongly bind to human c-Met but not human Fc were selected repeatedly. From the hybridoma cell lines obtained by repeated selection, a single clone producing a monoclonal antibody was finally separated by limiting dilution. The single clone of the hybridoma cell line producing the monoclonal antibody was deposited with the Korean Cell Line Research Foundation, an international depository authority located at Yungun-Dong, Jongno-Gu, Seoul, Korea, on Oct. 6, 2009, with Accession No. KCLRF-BP-00220 according to the Budapest Treaty (refer to Korean Patent Laid-Open Publication No. 2011-0047698).

### 1.1.4. Production and purification of monoclonal antibody

The hybridoma cell line obtained in Reference Example 1.1.3 was cultured in a serum-free medium, and the monoclonal antibody (AbF46) was produced and purified from the cell culture.

First, the hybridoma cells cultured in 50 mL of a medium (DMEM) supplemented with 10% (v/v) FBS were centrifuged and the cell pellet was washed twice or more with 20 mL of PBS to remove the FBS therefrom. Then, the cells were resuspended in 50 mL of DMEM and incubated for 3 days at 37 °C in a CO₂ incubator.

After the cells were removed by centrifugation, the supernatant was stored at 4 °C before use or immediately used for the separation and purification of the antibody. An AKTA system (GE Healthcare) equipped with an affinity column (Protein G agarose column; Pharmacia, USA) was used to purify the antibody from 50 to 300 mL of the supernatant, followed by concentration with a filter (Amicon). The antibody in PBS was stored before use in the following examples.

### 1.2. Construction of chAbF46, a chimeric antibody to c-Met

A mouse antibody is apt to elicit immunogenicity in humans. To solve this problem, chAbF46, a chimeric antibody, was constructed from the mouse antibody AbF46 produced in Experimental Example 1.1.4 by replacing the constant region, but not the variable region responsible for antibody specificity, with an amino sequence of the human IgG1 antibody.

In this regard, a gene was designed to include the nucleotide sequence of "EcoRI-signal sequence-VH-Nhel-CH-TGA-Xhol" (SEQ ID NO: 38) for a heavy chain and the nucleotide sequence of "EcoRI-signal sequence-VL-BsiWI-CL-TGA-Xhol" (SEQ ID NO: 39) for a light chain and synthesized. Then, a DNA fragment having the heavy chain nucleotide sequence (SEQ ID NO: 38) and a DNA fragment having the light chain nucleotide sequence (SEQ ID NO: 39) were digested with EcoRI (NEB, R0101S) and Xhol (NEB, R0146S) before cloning into a pOptiVEC^{™}-TOPO TA Cloning Kit enclosed in an OptiCHO^{™} Antibody Express Kit (Cat no. 12762-019, Invitrogen), and a pcDNA^{™}3.3-TOPO TA Cloning Kit (Cat no. 8300-01), respectively.

Each of the constructed vectors was amplified using Qiagen Maxiprep kit (Cat no. 12662), and a transient expression was performed using Freestyle^{™} MAX 293 Expression System (invitrogen). 293 F cells were used for the expression and cultured in FreeStyle™ 293 Expression Medium in a suspension culture manner. At one day before the transient expression, the cells were provided in the concentration of 5x10⁵ cells/ml, and after 24 hours, when the cell number reached 1x10⁶ cells/ml, the transient expression was performed. A transfection was performed by a liposomal reagent method using Freestyle^{™} MAX reagent (invitrogen), wherein in a 15 ml tube, the DNA was provided in the mixture ratio of 1:1 (heavy chain DNA : light chain DNA) and mixed with 2ml of OptiPro™ SFM (invitrogen) (tube A), and in another 15 ml tube, 100 µl (microliter) of Freestyle^{™} MAX reagent and 2 ml of OptiPro™ SFM were mixed (tube B), followed by mixing tube A and tube B and incubating for 15 minutes. The obtained mixture was slowly mixed with the cells provided one day before the transient expression. After completing the transfection, the cells were incubated in 130 rpm incubator for 5 days under the conditions of 37 °C, 80% humidity, and 8% CO₂.

Afterwards, the cells were incubated in DMEM supplemented with 10% (v/v) FBS for 5 hours at 37 °C under a 5% CO₂ condition and then in FBS-free DMEM for 48 hours at 37 °C under a 5% CO₂ condition.

After centrifugation, the supernatant was applied to AKTA prime (GE Healthcare) to purify the antibody. In this regard, 100 mL of the supernatant was loaded at a flow rate of 5 mL/min to AKTA Prime equipped with a Protein A column (GE healthcare, 17-0405-03), followed by elution with an IgG elution buffer (Thermo Scientific, 21004). The buffer was exchanged with PBS to purify a chimeric antibody AbF46 (hereinafter referred to as "chAbF46").

### 1.3. Construction of Humanized Antibody huAbF46 from Chimeric Antibody chAbF46

### 1.3.1. Heavy chain humanization

To design two domains H1-heavy and H3-heavy, human germline genes which share the highest identity/homology with the VH gene of the mouse antibody AbF46 purified in Reference Example 1.2 were analyzed. An Ig BLAST (www.ncbi.nlm.nih.gov/igblast/) result revealed that VH3-71 has an identity/ identity/homology of 83% at the amino acid level. CDR-H1, CDR-H2, and CDR-H3 of the mouse antibody AbF46 were defined according to Kabat numbering. A design was made to introduce the CDR of the mouse antibody AbF46 into the framework of VH3-71. Hereupon, back mutations to the amino acid sequence of the mouse AbF46 were conducted at positions 30 (S→T), 48 (V→L), 73 (D→N), and 78 (T→L). Then, H1 was further mutated at positions 83 (R→K) and 84 (A→T) to finally establish H1-heavy (SEQ ID NO: 40) and H3-heavy (SEQ ID NO: 41).

For use in designing H4-heavy, human antibody frameworks were analyzed by a BLAST search. The result revealed that the VH3 subtype, known to be most stable, is very similar in framework and sequence to the mouse antibody AbF46. CDR-H1, CDR-H2, and CDR-H3 of the mouse antibody AbF46 were defined according to Kabat numbering and introduced into the VH3 subtype to construct H4-heavy (SEQ ID NO: 42).

### 1.3.2. Light chain humanization

To design two domains H1-light (SEQ ID NO: 43) and H2-light (SEQ ID NO: 44), human germline genes which share the highest identity/homology with the VH gene of the mouse antibody AbF46 were analyzed. An Ig BLAST search result revealed that VK4-1 has a identity/homology of 75% at the amino acid level. CDR-L1, CDR-L2, and CDR-L3 of the mouse antibody AbF46 were defined according to Kabat numbering. A design was made to introduce the CDR of the mouse antibody AbF46 into the framework of VK4-1. Hereupon, back mutations to the amino acid sequence of the mouse AbF46 were conducted at positions 36 (Y→H), 46 (L→M), and 49 (Y→I). Only one back mutation was conducted at position 49 (Y→I) on H2-light.

To design H3-light (SEQ ID NO: 45), human germline genes which share the highest identity/homology with the VL gene of the mouse antibody AbF46 were analyzed by a search for BLAST. As a result, VK2-40 was selected. VL and VK2-40 of the mouse antibody AbF46 were found to have a identity/homology of 61% at an amino acid level. CDR-L1, CDR-L2, and CDR-L3 of the mouse antibody were defined according to Kabat numbering and introduced into the framework of VK4-1. Back mutations were conducted at positions 36 (Y→H), 46 (L→M), and 49 (Y→I) on H3-light.

For use in designing H4-light (SEQ ID NO: 46), human antibody frameworks were analyzed. A Blast search revealed that the Vk1 subtype, known to be the most stable, is very similar in framework and sequence to the mouse antibody AbF46. CDR-L1, CDR-L2, and CDR-L3 of the mouse antibody AbF46 were defined according to Kabat numbering and introduced into the Vk1 subtype. Hereupon, back mutations were conducted at positions 36 (Y→H), 46 (L→M), and 49 (Y→I) on H4-light.

Thereafter, DNA fragments having the heavy chain nucleotide sequences (H1-heavy: SEQ ID NO: 47, H3-heavy: SEQ ID NO: 48, H4-heavy: SEQ ID NO: 49) and DNA fragments having the light chain nucleotide sequences (H1-light: SEQ ID NO: 50, H2-light: SEQ ID NO: 51, H3-light: SEQ ID NO: 52, H4-light: SEQ ID NO: 53) were digested with EcoRI (NEB, R0101S) and Xhol (NEB, R0146S) before cloning into a pOptiVEC^{™}-TOPO TA Cloning Kit enclosed in an OptiCHO^{™} Antibody Express Kit (Cat no. 12762-019, Invitrogen) and a pcDNA^{™}3.3-TOPO TA Cloning Kit (Cat no. 8300-01), respectively, so as to construct recombinant vectors for expressing a humanized antibody.

Each of the constructed vectors was amplified using Qiagen Maxiprep kit (Cat no. 12662), and a transient expression was performed using Freestyle^{™} MAX 293 Expression System (invitrogen). 293 F cells were used for the expression and cultured in FreeStyle™ 293 Expression Medium in a suspension culture manner. At one day before the transient expression, the cells were provided in the concentration of 5x10⁵ cells/ml, and after 24 hours, when the cell number reached to 1x10⁶ cells/ml, the transient expression was performed. A transfection was performed by a liposomal reagent method using Freestyle^{™} MAX reagent (invitrogen), wherein in a 15 ml tube, the DNA was provided in the mixture ratio of 1:1 (heavy chain DNA : light chain DNA) and mixed with 2 ml of OptiPro™ SFM (invitrogen) (tube A), and in another 15 ml tube, 100 µl (microliter) of Freestyle^{™} MAX reagent and 2 ml of OptiPro™ SFM were mixed (tube B), followed by mixing tube A and tube B and incubating for 15 minutes. The obtained mixture was slowly mixed with the cells provided one day before the transient expression. After completing the transfection, the cells were incubated in 130 rpm incubator for 5 days under the conditions of 37 °C, 80% humidity, and 8% CO₂.

After centrifugation, the supernatant was applied to AKTA prime (GE Healthcare) to purify the antibody. In this regard, 100 mL of the supernatant was loaded at a flow rate of 5 mL/min to AKTA Prime equipped with a Protein A column (GE healthcare, 17-0405-03), followed by elution with an IgG elution buffer (Thermo Scientific, 21004). The buffer was exchanged with PBS to purify a humanized antibody AbF46 (hereinafter referred to as "huAbF46"). The humanized antibody huAbF46 used in the following examples comprised a combination of H4-heavy (SEQ ID NO: 42) and H4-light (SEQ ID NO: 46).

### 1.4. Construction of scFV Library of huAbF46 Antibody

For use in constructing an scFv of the huAbF46 antibody from the heavy and light chain variable regions of the huAbF46 antibody, a gene was designed to have the structure of "VH-linker-VL" for each of the heavy and the light chain variable region, with the linker having the amino acid sequence "GLGGLGGGGSGGGGSGGSSGVGS" (SEQ ID NO: 54). A polynucleotide sequence (SEQ ID NO: 55) encoding the designed scFv of huAbF46 was synthesized in Bioneer and an expression vector for the polynucleotide had the nucleotide sequence of SEQ ID NO: 56.

After expression, the product was found to exhibit specificity to c-Met.

### 1.5. Construction of Library Genes for Affinity Maturation

### 1.5.1. Selection of target CDRs and synthesis of primers

The affinity maturation of huAbF46 was achieved in the following steps. First, six complementary determining regions (CDRs) were defined according to Kabat numbering. The CDRs are given in Table 1, below.

**TABLE 1**

| CDR | Amino Acid Sequence |
|---|---|
| CDR-H1 | DYYMS (SEQ ID NO: 1) |
| CDR-H2 | FIRNKANGYTTEYSASVKG (SEQ ID NO: 2) |
| CDR-H3 | DNWFAY (SEQ ID NO: 3) |
| CDR-L1 | KSSQSLLASGNQNNYLA (SEQ ID NO: 10) |
| CDR-L2 | WASTRVS (SEQ ID NO: 11) |
| CDR-L3 | QQSYSAPLT (SEQ ID NO: 12) |

For use in the introduction of random sequences into the CDRs of the antibody, primers were designed as follows. Conventionally, N codons were utilized to introduce bases at the same ratio (25% A, 25% G, 25% C, 25% T) into desired sites of mutation. In this experiment, the introduction of random bases into the CDRs of huAbF46 was conducted in such a manner that, of the three nucleotides per codon in the wild-type polynucleotide encoding each CDR, the first and second nucleotides conserved over 85% of the entire sequence while the other three nucleotides were introduced at the same percentage (each 5%) and that the same possibility was imparted to the third nucleotide (33 % G, 33 % C, 33 % T).

### 1.5.2. Construction of a library of huAbF46 antibodies and affinity for c-Met

The construction of antibody gene libraries through the introduction of random sequences was carried out using the primers synthesized in the same manner as in Reference Example 1.5.1. Two PCR products were obtained using a polynucleotide covering the scFV of huAbF46 as a template, and were subjected to overlap extension PCR to give scFv library genes for huAbF46 antibodies in which only desired CDRs were mutated. Libraries targeting each of the six CDRs prepared from the scFV library genes were constructed.

The affinity for c-Met of each library was compared to that of the wildtype. Most libraries were lower in affinity for c-Met, compared to the wild-type. The affinity for c-Met was retained in some mutants.

### 1.6. Selection of Antibody with Improved Affinity from Libraries

After affinity maturation of the constructed libraries for c-Met, the nucleotide sequence of scFv from each clone was analyzed. The nucleotide sequences thus obtained are summarized in Table 2 and were converted into IgG forms. Four antibodies which were respectively produced from clones L3-1, L3-2, L3-3, and L3-5 were used in the subsequent experiments.

**TABLE 2**

| Clone | Library constructed | CDR Sequence |
|---|---|---|
| H11-4 | CDR-H1 | PEYYMS (SEQ ID NO: 22) |
| YC151 | CDR-H1 | PDYYMS (SEQ ID NO: 23) |
| YC193 | CDR-H1 | SDYYMS (SEQ ID NO: 24) |
| YC244 | CDR-H2 | RNNANGNT (SEQ ID NO: 25) |
| YC321 | CDR-H2 | RNKVNGYT (SEQ ID NO: 26) |
| YC354 | CDR-H3 | DNWLSY (SEQ ID NO: 27) |
| YC374 | CDR-H3 | DNWLTY (SEQ ID NO: 28) |
| L1-1 | CDR-L1 | KSSHSLLASGNQNNYLA (SEQ ID NO: 29) |
| L1-3 | CDR-L1 | KSSRSLLSSGNHKNYLA (SEQ ID NO: 30) |
| L1-4 | CDR-L1 | KSSKSLLASGNQNNYLA (SEQ ID NO: 31) |
| L1-12 | CDR-L1 | KSSRSLLASGNQNNYLA (SEQ ID NO: 32) |
| L1-22 | CDR-L1 | KSSHSLLASGNQNNYLA (SEQ ID NO: 33) |
| L2-9 | CDR-L2 | WASKRVS (SEQ ID NO: 34) |
| L2-12 | CDR-L2 | WGSTRVS (SEQ ID NO: 35) |
| L2-16 | CDR-L2 | WGSTRVP (SEQ ID NO: 36) |
| L3-1 | CDR-L3 | QQSYSRPYT (SEQ ID NO: 13) |
| L3-2 | CDR-L3 | GQSYSRPLT (SEQ ID NO: 14) |
| L3-3 | CDR-L3 | AQSYSHPFS (SEQ ID NO: 15) |
| L3-5 | CDR-L3 | QQSYSRPFT (SEQ ID NO: 16) |
| L3-32 | CDR-L3 | QQSYSKPFT (SEQ ID NO: 37) |

### 1.7. Conversion of Selected Antibodies into IgG

Respective polynucleotides encoding heavy chains of the four selected antibodies were designed to have the structure of "EcoRI-signal sequence-VH-Nhel-CH-Xhol" (SEQ ID NO: 38). The heavy chains of huAbF46 antibodies were used as they were because their amino acids were not changed during affinity maturation. In the case of the hinge region, however, the U6-HC7 hinge (SEQ ID NO: 57) was employed instead of the hinge of human IgG1. Genes were also designed to have the structure of "EcoRI-signal sequence-VL-BsiWI-CL-XhoI" for the light chain. Polypeptides encoding light chain variable regions of the four antibodies which were selected after the affinity maturation were synthesized in Bioneer. Then, a DNA fragment having the heavy chain nucleotide sequence (SEQ ID NO: 38) and DNA fragments having the light chain nucleotide sequences (DNA fragment comprising L3-1-derived CDR-L3: SEQ ID NO: 58, DNA fragment comprising L3-2-derived CDR-L3: SEQ ID NO: 59, DNA fragment comprising L3-3-derived CDR-L3: SEQ ID NO: 60, and DNA fragment comprising L3-5-derived CDR-L3: SEQ ID NO: 61) were digested with EcoRI (NEB, R01 01 S) and Xhol (NEB, R0146S) before cloning into a pOptiVEC^{™}-TOPO TA Cloning Kit enclosed in an OptiCHO^{™} Antibody Express Kit (Cat no. 12762-019, Invitrogen) and a pcDNA^{™}3.3-TOPO TA Cloning Kit (Cat no. 8300-01), respectively, so as to construct recombinant vectors for expressing affinity-matured antibodies.

Each of the constructed vectors was amplified using Qiagen Maxiprep kit (Cat no. 12662), and a transient expression was performed using Freestyle^{™} MAX 293 Expression System (invitrogen). 293 F cells were used for the expression and cultured in FreeStyle™ 293 Expression Medium in a suspension culture manner. At one day before the transient expression, the cells were provided in the concentration of 5x10⁵ cells/ml, and after 24 hours, when the cell number reached to 1x10⁶ cells/ml, the transient expression was performed. A transfection was performed by a liposomal reagent method using Freestyle^{™} MAX reagent (invitrogen), wherein in a 15 ml tube, the DNA was provided in the mixture ratio of 1:1 (heavy chain DNA : light chain DNA) and mixed with 2 ml of OptiPro™ SFM (invitrogen) (tube A), and in another 15 ml tube, 100 µl (microliter) of Freestyle^{™} MAX reagent and 2 ml of OptiPro™ SFM were mixed (tube B), followed by mixing tube A and tube B and incubating for 15 minutes. The obtained mixture was slowly mixed with the cells provided one day before the transient expression. After completing the transfection, the cells were incubated in 130 rpm incubator for 5 days under the conditions of 37 °C, 80% humidity, and 8% CO₂.

After centrifugation, the supernatant was applied to AKTA prime (GE Healthcare) to purify the antibody. In this regard, 100 mL of the supernatant was loaded at a flow rate of 5 mL/min to AKTA Prime equipped with a Protein A column (GE healthcare, 17-0405-03), followed by elution with an IgG elution buffer (Thermo Scientific, 21004). The buffer was exchanged with PBS to purify four affinity-matured antibodies (hereinafter referred to as "huAbF46-H4-A1 (L3-1 origin), huAbF46-H4-A2 (L3-2 origin), huAbF46-H4-A3 (L3-3 origin), and huAbF46-H4-A5 (L3-5 origin)," respectively).

### 1.8. Construction of Constant Region- and/or Hinge Region-Substituted huAbF46-H4-A1

Among the four antibodies selected in Reference Example 1.7, huAbF46-H4-A1 was found to be the highest in affinity for c-Met and the lowest in Akt phosphorylation and c-Met degradation degree. In the antibody, the hinge region, or the constant region and the hinge region, were substituted.

The antibody huAbF46-H4-A1 (U6-HC7) was composed of a heavy chain comprising the heavy chain variable region of huAbF46-H4-A1, U6-HC7 hinge, and the constant region of human IgG1 constant region, and a light chain comprising the light chain variable region of huAbF46-H4-A1 and human kappa constant region. The antibody huAbF46-H4-A1 (IgG2 hinge) was composed of a heavy chain comprising a heavy chain variable region, a human IgG2 hinge region, and a human IgG1 constant region, and a light chain comprising the light chain variable region of huAbF46-H4-A1 and a human kappa constant region. The antibody huAbF46-H4-A1 (IgG2 Fc) was composed of the heavy chain variable region of huAbF46-H4-A1, a human IgG2 hinge region, and a human IgG2 constant region, and a light chain comprising the light variable region of huAbF46-H4-A1 and a human kappa constant region. Hereupon, the histidine residue at position 36 on the human kappa constant region of the light chain was changed to tyrosine in all of the three antibodies to increase antibody production.

For use in constructing the three antibodies, a polynucleotide (SEQ ID NO: 63) encoding a polypeptide (SEQ ID NO: 62) composed of the heavy chain variable region of huAbF46-H4-A1, a U6-HC7 hinge region, and a human IgG1 constant region, a polynucleotide (SEQ ID NO: 65) encoding a polypeptide (SEQ ID NO: 64) composed of the heavy chain variable region of huAbF46-H4-A1, a human IgG2 hinge region, and a human IgG1 region, a polynucleotide (SEQ ID NO: 67) encoding a polypeptide (SEQ ID NO: 66) composed of the heavy chain variable region of huAbF46-H4-A1, a human IgG2 region, and a human IgG2 constant region, and a polynucleotide (SEQ ID NO: 69) encoding a polypeptide (SEQ ID NO: 68) composed of the light chain variable region of huAbF46-H4-A1, with a tyrosine residue instead of histidine at position 36, and a human kappa constant region were synthesized in Bioneer. Then, the DNA fragments having heavy chain nucleotide sequences were inserted into a pOptiVEC^{™}-TOPO TA Cloning Kit enclosed in an OptiCHO^{™} Antibody Express Kit (Cat no. 12762-019, Invitrogen) while DNA fragments having light chain nucleotide sequences were inserted into a pcDNA^{™}3.3-TOPO TA Cloning Kit (Cat no. 8300-01) so as to construct vectors for expressing the antibodies.

Each of the constructed vectors was amplified using Qiagen Maxiprep kit (Cat no. 12662), and a transient expression was performed using Freestyle^{™} MAX 293 Expression System (invitrogen). 293 F cells were used for the expression and cultured in FreeStyle™ 293 Expression Medium in a suspension culture manner. At one day before the transient expression, the cells were provided in the concentration of 5x10⁵ cells/ml, and after 24 hours, when the cell number reached to 1x10⁶ cells/ml, the transient expression was performed. A transfection was performed by a liposomal reagent method using Freestyle^{™} MAX reagent (invitrogen), wherein in a 15 ml tube, the DNA was provided in the mixture ratio of 1:1 (heavy chain DNA : light chain DNA) and mixed with 2 ml of OptiPro™ SFM (invitrogen) (tube A), and in another 15 ml tube, 100 ul (microliter) of Freestyle^{™} MAX reagent and 2 ml of OptiPro™ SFM were mixed (tube B), followed by mixing tube A and tube B and incubating for 15 minutes. The obtained mixture was slowly mixed with the cells provided one day before the transient expression. After completing the transfection, the cells were incubated in 130 rpm incubator for 5 days under the conditions of 37 °C, 80% humidity, and 8% CO₂.

After centrifugation, the supernatant was applied to AKTA prime (GE Healthcare) to purify the antibody. In this regard, 100 mL of the supernatant was loaded at a flow rate of 5 mL/min to AKTA Prime equipped with a Protein A column (GE healthcare, 17-0405-03), followed by elution with IgG elution buffer (Thermo Scientific, 21004). The buffer was exchanged with PBS to finally purify three antibodies (huAbF46-H4-A1 (U6-HC7), huAbF46-H4-A1 (IgG2 hinge), and huAbF46-H4-A1 (IgG2 Fc)). Among the three antibodies, huAbF46-H4-A1 (IgG2 Fc) was representatively selected for the following examples, and referred as anti-c-Met antibody L3-1Y/IgG2.

### Example 1: Decreased interaction between LRIG1 and USP8 by anti-c-Met antibody

LRIG1 (AAU44786) was overexpressed in MKN45 gastric cancer cells (JCRB, JCRB0254) using lipofectamin reagent (Invitrogen). Forty-eight hours after, the obtained LRIG1-overexpressed MKN45 gastric cancer cells were treated with 5ug/ml of anti-c-Met antibody L3-1Y/IgG2 for 1 hour, and the interaction between LRIG1 (AAU44786) and USP8 (NP_001122082: SEQ ID NO: 109) was measured. For this experiment, MKN45 gastric cancer cells were cultured in RPMI media containing 10%(v/v) FBS, and 10 ml of the cells (2x10⁵ cells/ml) were inoculated on 100 mm dish. To the 100 mm dish, the mixture of 10 µg of Flag-LRIG1 DNA and 40ul of lipofectamin reagent (Invitrogen) was added to perform the transfection of the cells, and 48 hours after, 5 µg/ml of anti-c-Met antibody L3-1 Y/IgG2 was treated.

An immunoprecipitation was conducted with the L3-1 Y/IgG2 antibody treated cells using anti-Flag antibody (Sigma). The cell culture (4 mL) was treated with 100 uM of concanamycin, and 4 hours after, the cells were collected. The collected cells were lysed with lysis buffer (Complete Lysis-M, Roche) and centrifuged at 13000 rpm for 15 minutes at 4 °C to obtain a protein solution. An immunoblotting was conducted for the obtained protein solution using anti-LRIG1 antibody (Abcam, Cambridge, UK) or anti-USP8 antibody (Cell Signaling, Danvers, MA, USA).

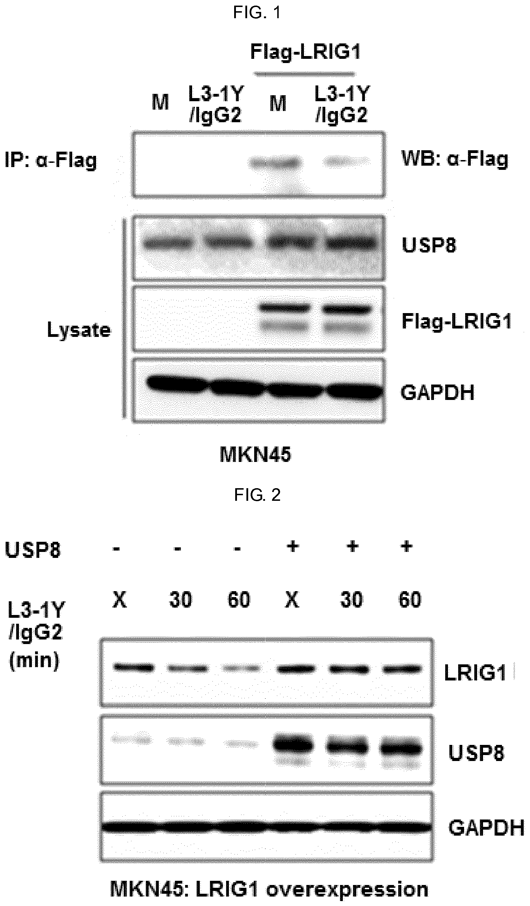

The obtained results are shown in FIG. 1. As shown in FIG. 1, the level of USP8 interacting with LRIG1 was decreased by treatment of anti-c-Met antibody L3-1Y/IgG2.

### Example 2: Inhibition of USP8 against degradation of LRIG1 by anti-c-Met antibody

Increased USP8 levels lead to increased stability of LRIG1. It was confirmed that when LRIG1-overexpressed MKN45 cells were treated with anti-c-Met antibody (5 µg/ml), the degradation of LRIG1 was stimulated, whereas in USP8-overexpressed MKN45 cells, LRIG1 was not degraded by anti-c-Met antibody.

In particular, LRIG1 (AAU44786) and USP8 (NP_001122082) were overexpressed in MKN45 gastric cancer cells (JCRB, JCRB0254) using lipofectamin reagent. Forty-eight hours after, the cells were treated with L3-1 Y/IgG2 (5 µg/ml), and cultured for the time indicated in FIG. 2 (30 minutes or 60 minutes). The MKN45 gastric cancer cells were cultured in 10% FBS RPMI media, and in 100 mm dish, the cells were transfected using the mixture of 5 µg of Flag-LRIG1 DNA, 5 µg or Flag-USP8 DNA and 40 µl lipofectamin reagent. Forty-eight hours after the transfection, the transfected cells were treated with 5 µg/ml of L3-1 Y/IgG2 antibody.

An immunoprecipitation was conducted for the L3-1 Y/IgG2 antibody treated cells using anti-Flag antibody (Sigma). The cells were treated with 100 µM concanamycin. 4 hours after, the cells were collected and lysed with lysis buffer. Then, the cell lysate was centrifuged at 13000 rpm for 15 minutes at 4 °C to obtain protein solution. For the protein solution, an immunoblotting was conducted using anti-LRIG1 antibody (Abcam, Cambridge, UK) or anti-USP8 antibody (Cell Signaling, Danvers, MA, USA).

The obtained results are shown in FIG. 2. As shown in FIG. 2, LRIG1 was degraded by the treatment of anti-c-Met antibody L3-1Y/IgG2, and the degree of LRIG1 degradation was decreased by the present of USP8.

### Example 3: Examination on relation between expression level of USP8 and ubiquitination of LRIG1 by anti-c-Met antibody

To confirm the deubiquitination of LRIG1 by USP8, it was tested whether the ubiquitination of LRIG1 by anti-c-Met antibody is decreased in USP8-overexpressed EBC1 cells.

In particular, Flag-USP8 was overexpressed in EBC1 cells (JCRB JCRB0820) using lipofectamin reagent. Forty-eight hours after, the cells were treated with 5ug/ml of anti-c-Met antibody L3-1Y/IgG2 for 1 hour. EBC1 cells (JCRB JCRB0820) were cultured in 10% FBS RPMI media, and in 100 mm dish, the cells were transfected using the mixture of 10 µg of Flag-USP8 DNA and 40 µl of lipofectamin reagent. Forty-eight hours after transfection, the transfected cells were treated with 5 µg/ml of the antibody.

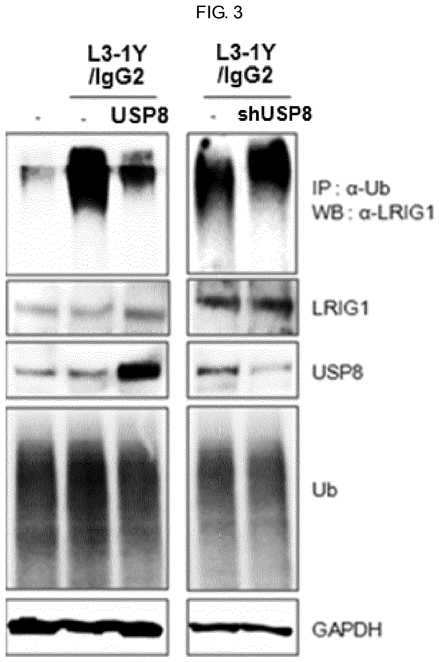

An immunoprecipitation was conducted for the L3-1 Y/IgG2 antibody treated cells using anti-Ubiquitin antibody (Santa Cruz). The cells were treated with 100 µM concanamycin. 4 hours after, the cells were collected and lysed with lysis buffer. Then, the cell lysate was centrifuged at 13000 rpm for 15 minutes at 4 °C to obtain protein solution. For the protein solution, an immunoprecipitation was conducted using anti-Ubiquitin antibody (Santa Cruz), and then, an immunoblotting was conducted using anti-LRIG1 antibody (Abcam, Cambridge, UK) or anti-Ubiquitin antibody. The obtained results are shown in FIG. 3 (left).

Meanwhile, to confirm that the ubiquitination of LRIG1 is stimulated by inhibition of USP8, it was examined whether the ubiquitination of LRIG1 is increased in USP8 knock-down EBC1 cells. In particular, EBC1 cells were transfected with control vector (Dharmacon) or shUSP8 (SEQ ID NO: 112; shUSP8 mature antisense: tatctcttccgattatcag) mixed with 10 µg of DNA and 40 µl of lipofectamin reagent. The transfected cells were treated with 5 µg/ml of L3-1Y/IgG2 and cultured at 37 °C for 1 hour. The obtained results are shown in FIG. 3 (right).

In FIG. 3, on the left are the results obtained by overexpression of wild type USP8, and on the right are the results obtained by knock-down of USP8. As shown in FIG. 3, the degree of ubiquitination of LRIG1 by the treatment of L3-1Y/IgG2 IgG2 is varied depending on the expression level of USP8, that is, whether or not the USP8 gene is knocked down or not.

### Example 4: Examination on anticancer efficacy of anti-c-Met antibody by knock-down of USP8

Seventy-two hours after knock down of USP8 in EBC1 cells, the level of cell proliferation was measured by CTG assay.

In particular, in 96 well plate, a reverse transfection of EBC1 cells (JCRB JCRB0820) using shUSP8(SEQ ID NO: 112) was performed to knock down the cells, referring to the method of Example 3. 24 hours after, the USP8 knock-down cells were treated with L3-1 Y/IgG2 in the amount of 0 µg/ml, 0.016 µg/ml, 0.08 µg/ml, 0.4 µg/ml, or 2 µg/ml for 72 hours. To the 96 well plate where the cells were cultured, Cell Titer Glo solution (Promega) was added in the amount of 100 µl per well, and 30 minutes after, luminescence signal was measured with Envision 2104 Multi-label Reader (Perkin Elmer, Foster City, CA, USA).

For comparison, the same experiment was performed using the control vector reverse-transfected cells.

The obtained cell viability (%) is shown in FIG. 4. As shown in FIG. 4, the anticancer efficacy of L3-1 Y/IgG2 IgG2 on the cells with USP8 knock down is considerably increased compared with that on the cells without USP8 knock down.

### Example 5: Examination of the change in c-Met degradation activity by USP8 mutation

In this example, USP8 wild type and USP8 C786S mutant were respectively overexpressed in EBC1 cells. It was confirmed that when the cells were treated with anti-c-Met antibody (5 µg/ml), degradation of LRIG1 is stimulated, where LRIG1 degradation by anti-c-Met antibody is induced in USP8 C786S mutant overexpressed cells, but not induced in USP8 wild type overexpressed cell. These results indicated that the increased level of USP8 leads to increase of LRIG1 stability (decrease of LRIG1 degradation), whereas the mutation in the active domain of USP8 leads to decrease of LRIG1 stability.

In particular, to examine whether or not USP8 affects the c-Met degradation activity of anti-c-Met antibody, USP8-WT(SEQ ID NO: 110) or inactive USP8-CS mutant(coding gene for SEQ ID NO: 111) were overexpressed in EBC1 cells (JCRB JCRB0820) using lipofectamin reagent. Forty-eight hours after, the cells were treated with L3-1Y/IgG2 (5 µg/ml) for 1 hour. The EBC1 cells (JCRB JCRB0820) were cultured in 10% FBS RPMI media, and in 100 mm dish, the cells were transfected by 10 µg of mixing Flag-LRIG1 DNA 10 µg and 40 µl lipofectamin reagent, and 48 hours after, treated with L3-1 Y/IgG2 (5 µg/ml).

The L3-1 Y/IgG2 antibody treated cells were collected, lysed with lysis buffer, and then centrifuged at 13000 rpm for 15 minutes at 4 °C to obtain protein solution. For the obtained protein solution, an immunoblotting was conducted using anti-c-Met antibody (Santa cruz, biotechnology Inc) or anti-LRIG1 antibody (Abcam, Cambridge, UK).

The obtained results are shown in FIG. 5. As shown in FIG. 5, when wild type USP8 is overexpressed, the c-Met degradation by L3-1 Y/IgG2 is inhibited, whereas when inactive USP8 mutant was overexpressed, , the c-Met degradation by L3-1Y/IgG2 is increased. The results indicate that the c-Met degradation activity of L3-1Y/IgG2 differs depending on the activation or inactivation of USP8.

### Example 6: Screening of anti-c-Met antibody sensitive cancer cells using USP8

To confirm the role of USP8 in tumor suppression, the following experiment was performed. Referring to the method of Example 5, EBC1 cells (JCRB JCRB0820) were transfected with USP8-WT (SEQ ID NO: 110), inactive USP8-CS mutant (coding gene for SEQ ID NO: 111), or shUSP8 (SEQ ID NO: 112) containing plasmid, to overexpress the genes.

Using Celltiter Glo (CTG) luminescent assay, the degree of cell growth of the EBC1 cells by treatment of anti-c-Met antibody (*in vitro* experimentation) was examined. The EBC1 cells were inoculated on 96-well plate (BD Biosciences, Palo Alto, CA, USA) in the density of 5x10³ cells per each well, and cultured in FBS 10% (v/v) RPMI 1640 medium (Gibco). 24 hours after, L3-1Y/IgG2 antibody was treated in the amount of 2 µg/ml which was diluted with 100 µL of 10% FBS (v/v) RPMI medium. 72 hours after, 100 µL of CTG solution (Promega) was added to each well, and the cells in the well were further cultured at 37 °C for 30 minutes. The luminescence signal from the cell culture was detected and recorded using Envision 2104 Multi-label Reader (Perkin Elmer). The cells transfected using control empty vector were used as a control (CTL).

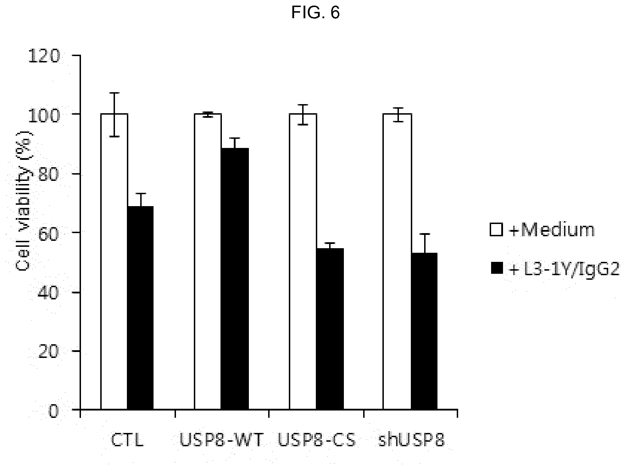

The obtained results are shown in FIG. 6. As shown in FIG. 6, the inhibition against the growth of EBC1 cells by L3-1Y/IgG2 is not achieved by the overexpression of wild-type USP8, but can be achieved by the overexpression of USP8-CS or shUSP8.

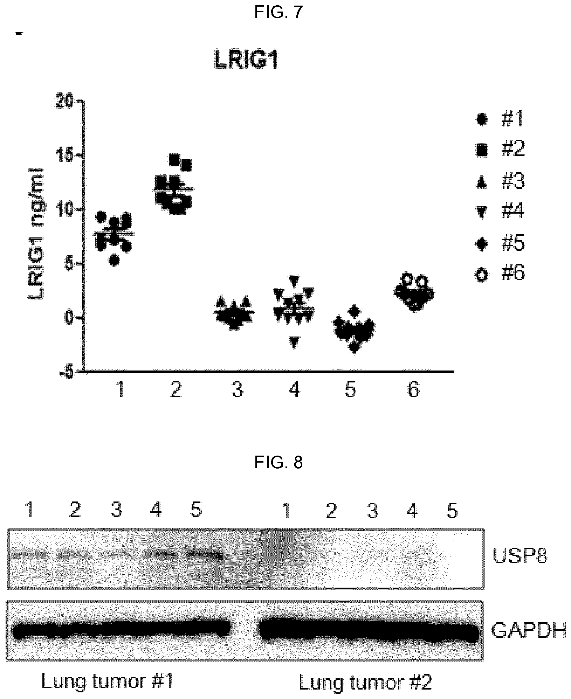

Based on the results, *in vivo* anti-tumor effect of L3-1Y/IgG2 antibody were determined using patient derived lung tumor xenograft samples (prepared by Oncotest, where cancer cells derived from lung cancer patients were grafted into male NRMI nu/nu mice). Based on the level of Met mRNA, 6 xenograft samples (#1, #2, #3, #4, #5, and #6) of lung tumor were selected, and the level of LRIG1 protein was measured using LRIG1 ELISA kit (MBS908302, MyBioSoure, CA, USA). The obtained results are shown in FIG. 7 (horizontal axis: samples, vertical axis: concentration of LRIG1).

Based on the results of FIG. 7, lung tumor xenograft samples #1 and #2 having high level of LRIG1 protein were selected, and the USP8 levels in the biological samples were measured by western blotting using anti-USP8 antibody (Cell Signaling,Danvers, MA, USA). The obtained results are shown in FIG. 8. As shown in FIG. 8, in lung tumor xenograft sample #1, the USP8 level is relatively high whereas in lung tumor xenograft sample #2, USP8 is nearly absent or present in low level.

Through the above step, lung tumor xenograft samples #1 and #2 which have high level of LRIG1 but different level of USP8 from each other. Lung tumor xenograft samples #1 and #2 were treated with L3-1Y/IgG2 antibody (5 mg/kg I.V. once/a week) and then ground to obtain proteins. The level of c-Met protein in the obtained proteins was measured using total c-Met ELISA kit (R&D systems). PBS treated group (group without antibody treatment) was used as a control.

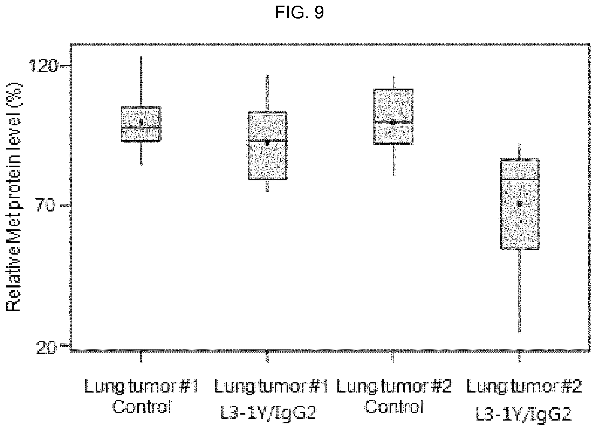

The obtained results are shown in FIG. 9. The c-Met level in the antibody treated sample is indicated as a percentage (%) of the c-Met level in the control. As shown in FIG. 9, in the xenograft sample #2 with low level of USP8, the c-Met level is decreased by treating with L3-1Y/IgG2 antibody, whereas in the xenograft sample #1 with high level of USP8, the c-Met level is not decreased by treating with L3-1Y/IgG2 antibody.

To confirm *in vivo* effect of the anti-c-Met antibody on the growth of patient derived lung tumor cells, tumor xenografting test was performed using male NRMI nu/nu mice (performed by Oncotest). The mice were acclimated for at least 1 week before tumor inoculation. Then, the mice were anesthetized with 1-2% (v/v) isofuran, and patient derived lung tumor cells (5x10⁶ cells) were injected to right flank of the mice. 7 days after, when the tumor cells grow to the average size of 50-250 mm³, the mice were grouped into follow groups: antibody L3-1Y/IgG2 (5 mg/kg I.V. once/a week) treated group, and vehicle (PBS 0.2 ml I.V. once/a week) treated group (control). Each group consisted of 10 mice. The tumor size and weight of mice were measured 2-3 times per a week during the experimentation period of 6 weeks in total. The tumor size (V) was calculated as follows: V(mm³)={long axis length (mm)x(short axis length (mm))²}/2.

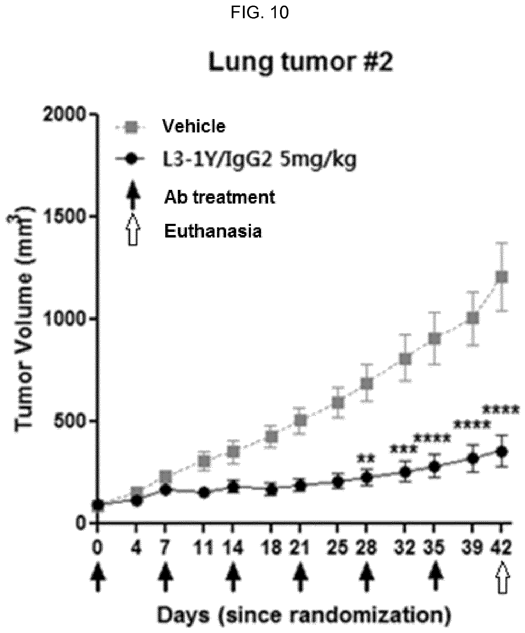

The obtained results are shown in FIG. 10. As shown in FIG. 10, similar to the results of decrease of c-Met, the inhibition effect by treatment of L3-1 Y/IgG2 antibody is observed in xenograft sample #2 (about 76% decrease).

From the above results, it can be revealed that L3-1Y/IgG2 antibody exhibits more excellent c-Met degradation effect in tumor having low level of USP8.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.
## Claims

1. *In vitro* method of selecting a subject for administration of an anti-c-Met antibody, comprising:
(i) determining the level of ubiquitin specific peptidase 8 (USP8), or the presence or absence of a mutation in an active site of USP8 in a biological sample from said subject; and
(ii) selecting the subject for administration of the anti-c-Met antibody if the score measured by immunohistochemical staining reflecting the absence or low level of USP8 is -, 0, or 1; or the mutation is present in an active site affecting its activity.

2. The method of claim 1, wherein the anti-c-Met antibody is an antibody or an antigen-binding fragment thereof comprising:
at least one heavy chain complementarity determining region (CDR) selected from the group consisting of (a) a CDR-H1 comprising the amino acid sequence of SEQ ID NO: 4; (b) a CDR-H2 comprising the amino acid sequence of SEQ ID NO: 5, SEQ ID NO: 2, or an amino acid sequence comprising 8-19 consecutive amino acids of SEQ ID NO: 2 including the 3^{rd} to 10^{th} positions of SEQ ID NO: 2; and (c) a CDR-H3 comprising the amino acid sequence of SEQ ID NO: 6, SEQ ID NO: 85, or an amino acid sequence comprising 6-13 consecutive amino acids of SEQ ID NO: 85 including the 1^{st} to 6^{th} positions of SEQ ID NO: 85, or a heavy chain variable region comprising the at least one heavy chain complementarity determining region;
at least one light chain complementarity determining region selected from the group consisting of (a) a CDR-L1 comprising the amino acid sequence of SEQ ID NO: 7, (b) a CDR-L2 comprising the amino acid sequence of SEQ ID NO: 8, and (c) a CDR-L3 comprising the amino acid sequence of SEQ ID NO: 9, SEQ ID NO: 86, or an amino acid sequence comprising 9-17 consecutive amino acids of SEQ ID NO: 89 including the 1^{st} to 9^{th} positions of SEQ ID NO: 89, or a light chain variable region comprising the at least one light chain complementarity determining region;
a combination of the at least one heavy chain complementarity determining region and the at least one light chain complementarity determining region; or
a combination of the heavy chain variable region and the light chain variable region.

3. A pharmaceutical composition for combination administration for use in a method for preventing or treating a cancer, comprising as active ingredients: i) an anti-c-Met antibody and ii) an inhibitor against USP8.

4. The pharmaceutical composition for use according to claim 3, wherein the anti-c-Met antibody and the inhibitor against USP8 are co-administered simultaneously or sequentially in any order.

5. The pharmaceutical composition for use according to claims 3 or 4, wherein the inhibitor against USP8 comprises at least one selected from the group consisting of:
a mutant USP8 comprising a mutation in an active site, a chemical inhibitor of USP8 or USP8 coding gene, an antibody against USP8, an aptamer against USP8 or USP8 coding gene, an siRNA against USP8 coding gene, a microRNA against USP8 coding gene, an shRNA against the USP8 coding gene, and a polynucleotide encoding a mutant USP8 comprising a mutation in an active site.

6. The pharmaceutical composition for use according to any one of claims 3-5, wherein the anti-c-Met antibody is an antibody or an antigen-binding fragment thereof and comprises:
at least one heavy chain complementarity determining region (CDR) selected from the group consisting of (a) a CDR-H1 comprising the amino acid sequence of SEQ ID NO: 4; (b) a CDR-H2 comprising the amino acid sequence of SEQ ID NO: 5, SEQ ID NO: 2, or an amino acid sequence comprising 8-19 consecutive amino acids of SEQ ID NO: 2 including the 3^{rd} to 10^{th} positions of SEQ ID NO: 2; and (c) a CDR-H3 comprising the amino acid sequence of SEQ ID NO: 6, SEQ ID NO: 85, or an amino acid sequence comprising 6-13 consecutive amino acids of SEQ ID NO: 85 including the 1^{st} to 6^{th} positions of SEQ ID NO: 85, or a heavy chain variable region comprising the at least one heavy chain complementarity determining region;
at least one light chain complementarity determining region selected from the group consisting of (a) a CDR-L1 comprising the amino acid sequence of SEQ ID NO: 7, (b) a CDR-L2 comprising the amino acid sequence of SEQ ID NO: 8, and (c) a CDR-L3 comprising the amino acid sequence of SEQ ID NO: 9, SEQ ID NO: 86, or an amino acid sequence comprising 9-17 consecutive amino acids of SEQ ID NO: 89 including the 1^{st} to 9^{th} positions of SEQ ID NO: 89, or a light chain variable region comprising the at least one light chain complementarity determining region;
a combination of the at least one heavy chain complementarity determining region and the at least one light chain complementarity determining region; or
a combination of the heavy chain variable region and the light chain variable region.

7. The pharmaceutical composition for use according to claim 6, wherein
the CDR-H1 comprises the amino acid sequence of SEQ ID NO: 1, SEQ ID NO: 22, SEQ ID NO: 23, or SEQ ID NO: 24,
the CDR-H2 comprises the amino acid sequence of SEQ ID NO: 2, SEQ ID NO: 25, or SEQ ID NO: 26,
the CDR-H3 comprises the amino acid sequence of SEQ ID NO: 3, SEQ ID NO: 27, SEQ ID NO: 28, or SEQ ID NO: 85,
the CDR-L1 comprises the amino acid sequence of SEQ ID NO: 10, SEQ ID NO: 29, SEQ ID NO: 30, SEQ ID NO: 31, SEQ ID NO: 32, SEQ ID NO: 33, or SEQ ID NO: 106,
the CDR-L2 comprises the amino acid sequence of SEQ ID NO: 11, SEQ ID NO: 34, SEQ ID NO: 35, or SEQ ID NO: 36, and
the CDR-L3 comprises the amino acid sequence of SEQ ID NO: 12, SEQ ID NO: 13, SEQ ID NO: 14, SEQ ID NO: 15, SEQ ID NO: 16, SEQ ID NO: 37, SEQ ID NO: 86, or SEQ ID NO: 89.

8. The pharmaceutical composition for use according to claim 6, wherein
the heavy chain variable region comprises an amino acid sequence selected from the group consisting of SEQ ID NOS: 17, 74, 87, 90, 91, 92, 93, and 94, and
the light chain variable region comprises an amino acid sequence selected from the group consisting of SEQ ID NOS: 18, 19, 20, 21, 75, 88, 95, 96, 97, 98, 99, and 107.

9. The pharmaceutical composition for use according to claim 6, wherein the anti-c-Met antibody comprises:
(i) a heavy chain comprising the amino acid sequence of SEQ ID NO: 62, the amino acid sequence from the 18^{th} to 462^{nd} positions of SEQ ID NO: 62, the amino acid sequence of SEQ ID NO: 64, the amino acid sequence from the 18^{th} to 461^{st} positions of SEQ ID NO: 64, the amino acid sequence of SEQ ID NO: 66, or the amino acid sequence from the 18^{th} to 460^{th} positions of SEQ ID NO: 66; and
(ii) a light chain comprising the amino acid sequence of SEQ ID NO: 68, the amino acid sequence from the 21^{st} to 240^{th} positions of SEQ ID NO: 68, the amino acid sequence of SEQ ID NO: 70, the amino acid sequence from the 21^{st} to 240^{th} positions of SEQ ID NO: 70, or the amino acid sequence of SEQ ID NO: 108.

## Patentansprüche

1. *In vitro* Verfahren zum Auswählen eines Probanden zur Verabreichung eines anti-c-Met-Antikörpers, umfassend:
(i) Bestimmen des Spiegels von Ubiquitin-spezifischer Peptidase 8 (USP8), oder des Vorhandenseins oder der Abwesenheit einer Mutation in einer aktiven Stelle von USP8 in einer biologischen Probe von dem Probanden; und
(ii) Auswählen des Probanden zur Verabreichung des anti-c-Met-Antikörpers, wenn der Wert, der durch immunhistochemische Anfärbung gemessen wird und die Abwesenheit oder einen niedrigen Spiegel von USP8 widerspiegelt, -, 0 oder 1 ist; oder
die Mutation in einer aktiven Stelle vorhanden ist, welche ihre Aktivität beeinflusst.

2. Verfahren nach Anspruch 1, wobei der anti-c-Met-Antikörper ein Antikörper oder ein Antigen-bindendes Fragment davon ist, umfassend:
wenigstens eine komplementaritätsbestimmende Region (CDR) der schweren Kette, ausgewählt aus der Gruppe bestehend aus (a) einer CDR-H1 umfassend die Aminosäuresequenz von SEQ ID NO: 4; (b) einer CDR-H2 umfassend die Aminosäuresequenz von SEQ ID NO: 5, SEQ ID NO: 2, oder eine Aminosäuresequenz umfassend 8-19 aufeinanderfolgende Aminosäuren von SEQ ID NO: 2 einschließlich der 3-ten bis 10-ten Positionen von SEQ ID NO: 2; und (c) einer CDR-H3 umfassend die Aminosäuresequenz von SEQ ID NO: 6, SEQ ID NO: 85, oder eine Aminosäuresequenz umfassend 6-13 aufeinanderfolgende Aminosäuren von SEQ ID NO: 85 einschließlich der 1-ten bis 6-ten Positionen von SEQ ID NO: 85, oder eine variable Region der schweren Kette umfassend die wenigstens eine komplementaritätsbestimmende Region der schweren Kette;
wenigstens eine komplementaritätsbestimmende Region der leichten Kette, ausgewählt aus der Gruppe bestehend aus (a) einer CDR-L1 umfassend die Aminosäuresequenz von SEQ ID NO: 7, (b) einer CDR-L2 umfassend die Aminosäuresequenz von SEQ ID NO: 8, und (c) einer CDR-L3 umfassend die Aminosäuresequenz von SEQ ID NO: 9, SEQ ID NO: 86, oder eine Aminosäuresequenz umfassend 9-17 aufeinanderfolgende Aminosäuren von SEQ ID NO: 89, einschließlich der 1-ten bis 9-ten Positionen von SEQ ID NO: 89, oder eine variable Region der leichten Kette umfassend die wenigstens eine komplementaritätsbestimmende Region der leichten Kette;
eine Kombination der wenigstens einen komplementaritätsbestimmenden Region der schweren Kette und der wenigstens einen komplementaritätsbestimmenden Region der leichten Kette; oder
eine Kombination der variablen Region der schweren Kette und der variablen Region der leichten Kette.

3. Pharmazeutische Zusammensetzung zur kombinierten Verabreichung zur Verwendung in einem Verfahren zum Verhüten oder Behandeln einer Krebserkrankung, umfassend als Wirkstoffe: i) einen anti-c-Met-Antikörper und ii) einen Inhibitor gegen USP8.

4. Pharmazeutische Zusammensetzung zur Verwendung gemäß Anspruch 3, wobei der anti-c-Met-Antikörper und der Inhibitor gegen USP8 gleichzeitig oder nacheinander in einer beliebigen Reihenfolge co-verabreicht werden.

5. Pharmazeutische Zusammensetzung zur Verwendung gemäß den Ansprüchen 3 oder 4, wobei der Inhibitor gegen USP8 wenigstens eines umfasst, ausgewählt aus der Gruppe bestehend aus:
einer mutierten USP8, die eine Mutation in einer aktiven Stelle umfasst, einem chemischen Inhibitor von USP8 oder USP8 codierendem Gen, einem Antikörper gegen USP8, einem Aptamer gegen USP8 oder USP8 codierendes Gen, einer siRNA gegen USP8 codierendes Gen, einer microRNA gegen USP8 codierendes Gen, einer shRNA gegen das USP8 codierende Gen, und einem Polynucleotid, das eine mutierte USP8 codiert, die eine Mutation in einer aktiven Stelle umfasst.

6. Pharmazeutische Zusammensetzung zur Verwendung gemäß einem der Ansprüche 3-5, wobei der anti-c-Met-Antikörper ein Antikörper oder ein Antigen-bindendes Fragment davon ist und umfasst:
wenigstens eine komplementaritätsbestimmende Region (CDR) der schweren Kette, ausgewählt aus der Gruppe bestehend aus (a) einer CDR-H1 umfassend die Aminosäuresequenz von SEQ ID NO: 4; (b) einer CDR-H2 umfassend die Aminosäuresequenz von SEQ ID NO: 5, SEQ ID NO: 2, oder eine Aminosäuresequenz umfassend 8-19 aufeinanderfolgende Aminosäuren von SEQ ID NO: 2 einschließlich der 3-ten bis 10-ten Positionen von SEQ ID NO: 2; und (c) einer CDR-H3 umfassend die Aminosäuresequenz von SEQ ID NO: 6, SEQ ID NO: 85, oder eine Aminosäuresequenz umfassend 6-13 aufeinanderfolgende Aminosäuren von SEQ ID NO: 85 einschließlich der 1-ten bis 6-ten Positionen von SEQ ID NO: 85, oder eine variable Region der schweren Kette umfassend die wenigstens eine komplementaritätsbestimmende Region der schweren Kette;
wenigstens eine komplementaritätsbestimmende Region der leichten Kette, ausgewählt aus der Gruppe bestehend aus (a) einer CDR-L1 umfassend die Aminosäuresequenz von SEQ ID NO: 7, (b) einer CDR-L2 umfassend die Aminosäuresequenz von SEQ ID NO: 8, und (c) einer CDR-L3 umfassend die Aminosäuresequenz von SEQ ID NO: 9, SEQ ID NO: 86, oder eine Aminosäuresequenz umfassend 9-17 aufeinanderfolgende Aminosäuren von SEQ ID NO: 89, einschließlich der 1-ten bis 9-ten Positionen von SEQ ID NO: 89, oder eine variable Region der leichten Kette umfassend die wenigstens eine komplementaritätsbestimmende Region der leichten Kette;
eine Kombination der wenigstens einen komplementaritätsbestimmenden Region der schweren Kette und der wenigstens einen komplementaritätsbestimmenden Region der leichten Kette; oder
eine Kombination der variablen Region der schweren Kette und der variablen Region der leichten Kette.

7. Pharmazeutische Zusammensetzung zur Verwendung gemäß Anspruch 6, wobei
die CDR-H1 die Aminosäuresequenz von SEQ ID NO: 1, SEQ ID NO: 22, SEQ ID NO: 23, oder SEQ ID NO: 24 umfasst,
die CDR-H2 die Aminosäuresequenz von SEQ ID NO: 2, SEQ ID NO: 25, oder SEQ ID NO: 26 umfasst,
die CDR-H3 die Aminosäuresequenz von SEQ ID NO: 3, SEQ ID NO: 27, SEQ ID NO: 28, oder SEQ ID NO: 85 umfasst,
die CDR-L1 die Aminosäuresequenz von SEQ ID NO: 10, SEQ ID NO: 29, SEQ ID NO: 30, SEQ ID NO: 31, SEQ ID NO: 32, SEQ ID NO: 33, oder SEQ ID NO: 106 umfasst,
die CDR-L2 die Aminosäuresequenz von SEQ ID NO: 11, SEQ ID NO: 34, SEQ ID NO: 35, oder SEQ ID NO: 36 umfasst, und
die CDR-L3 die Aminosäuresequenz von SEQ ID NO: 12, SEQ ID NO: 13, SEQ ID NO: 14, SEQ ID NO: 15, SEQ ID NO: 16, SEQ ID NO: 37, SEQ ID NO: 86, oder SEQ ID NO: 89 umfasst.

8. Pharmazeutische Zusammensetzung zur Verwendung gemäß Anspruch 6, wobei
die variable Region der schweren Kette eine Aminosäuresequenz, ausgewählt aus der Gruppe bestehend aus SEQ ID NOS: 17, 74, 87, 90, 91, 92, 93, und 94, umfasst, und
die variable Region der leichten Kette eine Aminosäuresequenz, ausgewählt aus der Gruppe bestehend aus SEQ ID NOS: 18, 19, 20, 21, 75, 88, 95, 96, 97, 98, 99, und 107, umfasst.

9. Pharmazeutische Zusammensetzung zur Verwendung gemäß Anspruch 6, wobei der anti-c-Met-Antikörper umfasst:
(i) eine schwere Kette umfassend die Aminosäuresequenz von SEQ ID NO: 62, die Aminosäuresequenz von den 18-ten bis 462-ten Positionen von SEQ ID NO: 62, die Aminosäuresequenz von SEQ ID NO: 64, die Aminosäuresequenz von den 18-ten bis 461-ten Positionen von SEQ ID NO: 64, die Aminosäuresequenz von SEQ ID NO: 66 oder die Aminosäuresequenz von den 18-ten bis 460-ten Positionen von SEQ ID NO: 66; und
(ii) eine leichte Kette umfassend die Aminosäuresequenz von SEQ ID NO: 68, die Aminosäuresequenz von den 21-ten bis 240-ten Positionen von SEQ ID NO: 68, die Aminosäuresequenz von SEQ ID NO: 70, die Aminosäuresequenz von den 21-ten bis 240-ten Positionen von SEQ ID NO: 70, oder die Aminosäuresequenz von SEQ ID NO: 108.

## Revendications

1. Procédé *in vitro* de sélection d'un sujet pour l'administration d'un anticorps dirigé contre c-Met, comprenant :
(i) la détermination du niveau de peptidase 8 spécifique à l'ubiquitine (USP8), ou la présence ou l'absence d'une mutation au niveau d'un site actif d'USP8 dans un échantillon biologique provenant dudit sujet ; et
(ii) la sélection du sujet pour l'administration de l'anticorps dirigé contre c-Met si le score mesuré par coloration immunohistochimique reflétant l'absence ou le niveau bas d'USP8 est -, 0 ou 1 ; ou
la mutation est présente au niveau d'un site actif et affecte son activité.

2. Procédé selon la revendication 1, dans lequel l'anticorps dirigé contre c-Met est un anticorps ou un fragment de celui-ci de liaison à un antigène comprenant :
au moins une région de détermination de complémentarité (CDR) de chaîne lourde choisie dans le groupe constitué de (a) une CDR-H1 comprenant la séquence d'acides aminés de la SEQ ID NO : 4 ; (b) une CDR-H2 comprenant la séquence d'acides aminés des SEQ ID NO : 5, SEQ ID NO : 2, ou une séquence d'acides aminés comprenant 8 à 19 acides aminés consécutifs de la SEQ ID NO : 2 incluant les positions 3 à 10 de la SEQ ID NO : 2 ; et (c) une CDR-H3 comprenant la séquence d'acides aminés des SEQ ID NO : 6, SEQ ID NO : 85, ou une séquence d'acides aminés comprenant 6 à 13 acides aminés consécutifs de la SEQ ID NO : 85 incluant les positions 1 à 6 de la SEQ ID NO : 85, ou une région variable de chaîne lourde comprenant ladite au moins une région de détermination de complémentarité de chaîne lourde ;
au moins une région de détermination de complémentarité (CDR) de chaîne légère choisie dans le groupe constitué de (a) une CDR-L1 comprenant la séquence d'acides aminés de la SEQ ID NO : 7 ; (b) une CDR-L2 comprenant la séquence d'acides aminés de la SEQ ID NO : 8 ; et (c) une CDR-L3 comprenant la séquence d'acides aminés des SEQ ID NO : 9, SEQ ID NO : 86, ou une séquence d'acides aminés comprenant 9 à 17 acides aminés consécutifs de la SEQ ID NO : 89 incluant les positions 1 à 9 de la SEQ ID NO : 89, ou une région variable de chaîne légère comprenant ladite au moins une région de détermination de complémentarité de chaîne légère ;
une combinaison de ladite au moins une région de détermination de la complémentarité de chaîne lourde et de ladite au moins une région de détermination de la complémentarité de chaîne légère ; ou
une combinaison de la région variable de chaîne lourde et de la région variable de chaîne légère.

3. Composition pharmaceutique pour administration de combinaison destinée à être utilisée dans un procédé pour prévenir ou traiter un cancer, comprenant, en tant que principes actifs : 1) un anticorps dirigé contre c-Met et ii) un inhibiteur d'USP8.

4. Composition pharmaceutique destinée à être utilisée selon la revendication 3, l'anticorps dirigé contre c-Met et l'inhibiteur d'USP8 étant co-administrés simultanément ou séquentiellement dans n'importe quel ordre.

5. Composition pharmaceutique destinée à être utilisée selon les revendications 3 ou 4, l'inhibiteur d'USP8 comprenant au moins un élément choisi dans le groupe constitué de :
une USP8 mutante comprenant une mutation au niveau d'un site actif, un inhibiteur chimique d'USP8 ou du gène codant pour USP8, un anticorps dirigé contre USP8, un aptamère dirigé contre USP8 ou le gène codant pour USP8, un petit ARN interférent contre USP8 ou le gène codant pour USP8, un microARN dirigé contre le gène codant pour USP8, un petit ARN en épingle à cheveux dirigé contre le gène codant pour USP8 et un polynucléotide codant pour une USP8 mutante comprenant une mutation au niveau d'un site actif.

6. Composition pharmaceutique destinée à être utilisée selon l'une quelconque des revendications 3 à 5, l'anticorps dirigé contre c-Met étant un anticorps ou un fragment de celui-ci de liaison à un antigène comprenant :
au moins une région de détermination de complémentarité (CDR) de chaîne lourde choisie dans le groupe constitué de (a) une CDR-H1 comprenant la séquence d'acides aminés de la SEQ ID NO : 4 ; (b) une CDR-H2 comprenant la séquence d'acides aminés des SEQ ID NO : 5, SEQ ID NO : 2, ou une séquence d'acides aminés comprenant 8 à 19 acides aminés consécutifs de la SEQ ID NO : 2 incluant les positions 3 à 10 de la SEQ ID NO : 2 ; et (c) une CDR-H3 comprenant la séquence d'acides aminés des SEQ ID NO : 6, SEQ ID NO : 85, ou une séquence d'acides aminés comprenant 6 à 13 acides aminés consécutifs de la SEQ ID NO : 85 incluant les positions 1 à 6 de la SEQ ID NO : 85, ou une région variable de chaîne lourde comprenant ladite au moins une région de détermination de complémentarité de chaîne lourde ;
au moins une région de détermination de complémentarité (CDR) de chaîne légère choisie dans le groupe constitué de (a) une CDR-L1 comprenant la séquence d'acides aminés de la SEQ ID NO : 7 ; (b) une CDR-L2 comprenant la séquence d'acides aminés de la SEQ ID NO : 8 ; et (c) une CDR-L3 comprenant la séquence d'acides aminés des SEQ ID NO : 9, SEQ ID NO : 86, ou une séquence d'acides aminés comprenant 9 à 17 acides aminés consécutifs de la SEQ ID NO : 89 incluant les positions 1 à 9 de la SEQ ID NO : 89, ou une région variable de chaîne légère comprenant ladite au moins une région de détermination de complémentarité de chaîne légère ;
une combinaison de ladite au moins une région de détermination de la complémentarité de chaîne lourde et de ladite au moins une région de détermination de la complémentarité de chaîne légère ; ou
une combinaison de la région variable de chaîne lourde et de la région variable de chaîne légère.

7. Composition pharmaceutique destinée à être utilisée selon la revendication 6, dans laquelle
la CDR-H1 comprend la séquence d'acides aminés de la SEQ ID NO : 1, la SEQ ID NO : 22, la SEQ ID NO : 23 ou la SEQ ID NO : 24,
la CDR-H2 comprend la séquence d'acides aminés de la SEQ ID NO : 2, la SEQ ID NO : 25 ou la SEQ ID NO : 26,
la CDR-H3 comprend la séquence d'acides aminés de la SEQ ID NO : 3, la SEQ ID NO : 27, la SEQ ID NO : 28 ou la SEQ ID NO : 85,
la CDR-L1 comprend la séquence d'acides aminés de la SEQ ID NO : 10, la SEQ ID NO : 29, la SEQ ID NO : 30, la SEQ ID NO : 31, la SEQ ID NO : 32, la SEQ ID NO : 33, ou la SEQ ID NO : 106,
la CDR-L2 comprend la séquence d'acides aminés de la SEQ ID NO : 11, la SEQ ID NO : 34, la SEQ ID NO : 35, ou la SEQ ID NO : 36, et
la CDR-L3 comprend la séquence d'acides aminés de la SEQ ID NO : 12, la SEQ ID NO : 13, la SEQ ID NO : 14, la SEQ ID NO : 15, la SEQ ID NO : 16, la SEQ ID NO : 37, la SEQ ID NO : 86, ou la SEQ ID NO : 89.

8. Composition pharmaceutique destinée à être utilisée selon la revendication 6, dans laquelle
la région variable de chaîne lourde comprend une séquence d'acides aminés choisie dans le groupe constitué des SEQ ID NO : 17, 74, 87, 90, 91, 92, 93 et 94, et
la région variable de chaîne légère comprend une séquence d'acides aminés choisie dans le groupe constitué des SEQ ID NO : 18, 19, 20, 21, 75, 88, 95, 96, 97, 98, 99 et 107.

9. Composition pharmaceutique destinée à être utilisée selon la revendication 6, dans laquelle l'anticorps dirigé contre c-Met comprend :
(i) une chaîne lourde comprenant la séquence d'acides aminés de la SEQ ID NO : 62, la séquence d'acides aminés des positions 18 à 462 de la SEQ ID NO : 62, la séquence d'acides aminés de la SEQ ID NO : 64, la séquence d'acides aminés des positions 18 à 461 de la SEQ ID NO : 64, la séquence d'acides aminés de la SEQ ID NO : 66, ou la séquence d'acides aminés des positions 18 à 460 de la SEQ ID NO : 66 ; et
(ii) une chaîne légère comprenant la séquence d'acides aminés de la SEQ ID NO : 68, la séquence d'acides aminés des positions 21 à 240 de la SEQ ID NO : 68, la séquence d'acides aminés de la SEQ ID NO : 70, la séquence d'acides aminés des positions 21 à 240 de SEQ ID NO : 70, ou la séquence d'acides aminés de la SEQ ID NO : 108.


<110> Samsung Electronics Co. Ltd
<120> Biomarker for identifying a subject for application of an anti-c-Met antibody
<130> EP94543FZSEpau
<140> not yet assigned
   <141> herewith
<150> KR 10-2013-0080281
   <151> 2013-07-09
<150> KR 10-2013-0130534
   <151> 2013-10-30
<160> 112
<170> KopatentIn 1.71
<210> 1
   <211> 5
   <212> PRT
   <213> Artificial Sequence
<220>
   <223> heavy chain CDR1 of AbF46
<400> 1
   Asp Tyr Tyr Met Ser
    1               5
<210> 2
   <211> 19
   <212> PRT
   <213> Artificial Sequence
<220>
   <223> heavy chain CDR2 of AbF46
<400> 2
   Phe Ile Arg Asn Lys Ala Asn Gly Tyr Thr Thr Glu Tyr Ser Ala Ser
    1               5                  10                  15
   Val Lys Gly
<210> 3
   <211> 6
   <212> PRT
   <213> Artificial Sequence
<220>
   <223> heavy chain CDR3 of AbF46
<400> 3
   Asp Asn Trp Phe Ala Tyr
   1 5
<210> 4
   <211> 6
   <212> PRT
   <213> Artificial Sequence
<220>
   <223> heavy chain CDR1 of c-Met antibody
<220>
   <221> VARIANT
   <222> (1)
   <223> X is Pro or Ser or absent
<220>
   <221> VARIANT
   <222> (2)
   <223> X is Glu or Asp
<400> 4
   Xaa Xaa Tyr Tyr Met Ser
   1 5
<210> 5
   <211> 8
   <212> PRT
   <213> Artificial Sequence
<220>
   <223> heavy chain CDR2 of c-Met antibody
<220>
   <221> VARIANT
   <222> (3)
   <223> X is Asn or Lys
<220>
   <221> VARIANT
   <222> (4)
   <223> X is Ala or Val
<220>
   <221> VARIANT
   <222> (7)
   <223> X is Asn or Thr
<400> 5
   Arg Asn Xaa Xaa Asn Gly Xaa Thr
   1 5
<210> 6
   <211> 6
   <212> PRT
   <213> Artificial Sequence
<220>
   <223> heavy chain CDR3 of c-Met antibody
<220>
   <221> VARIANT
   <222> (5)
   <223> X is Ser or Thr
<400> 6
   Asp Asn Trp Leu Xaa Tyr
   1 5
<210> 7
   <211> 17
   <212> PRT
   <213> Artificial Sequence
<220>
   <223> light chain CDR1 of c-Met antibody
<220>
   <221> VARIANT
   <222> (4)
   <223> X is His, Arg, Gln or Lys
<220>
   <221> VARIANT
   <222> (12)
   <223> X is His or Gln
<220>
   <221> VARIANT
   <222> (13)
   <223> X is Lys or Asn
<220>
   <221> VARIANT
   <222> (9)
   <223> X is Ser or Trp
<400> 7
   Lys Ser Ser Xaa Ser Leu Leu Ala Xaa Gly Asn Xaa Xaa Asn Tyr Leu
   1 5 10 15
   Ala
<210> 8
   <211> 7
   <212> PRT
   <213> Artificial Sequence
<220>
   <223> light chain CDR2 of c-Met antibody
<220>
   <221> VARIANT
   <222> (2)
   <223> X is Ala or Gly
<220>
   <221> VARIANT
   <222> (4)
   <223> X is Thr or Lys
<220>
   <221> VARIANT
   <222> (7)
   <223> X is Ser or Pro
<400> 8
   Trp Xaa Ser Xaa Arg Val Xaa
   1 5
<210> 9
   <211> 9
   <212> PRT
   <213> Artificial Sequence
<220>
   <223> light chain CDR3 of c-Met antibody
<220>
   <221> VARIANT
   <222> (1)
   <223> X is Gly, Ala or Gln
<220>
   <221> VARIANT
   <222> (6)
   <223> X is Arg, His, Ser, Ala, Gly or Lys
<220>
   <221> VARIANT
   <222> (8)
   <223> X is Leu, Tyr, Phe or Met
<400> 9
   Xaa Gln Ser Tyr Ser Xaa Pro Xaa Thr
   1 5
<210> 10
   <211> 17
   <212> PRT
   <213> Artificial Sequence
<220>
   <223> light chain CDR1 of AbF46
<400> 10
   Lys Ser Ser Gln Ser Leu Leu Ala Ser Gly Asn Gln Asn Asn Tyr Leu
   1 5 10 15
   Ala
<210> 11
   <211> 7
   <212> PRT
   <213> Artificial Sequence
<220>
   <223> light chain CDR2 of AbF46
<400> 11
   Trp Ala Ser Thr Arg Val Ser
   1 5
<210> 12
   <211> 9
   <212> PRT
   <213> Artificial Sequence
<220>
   <223> light chain CDR3 of AbF46
<400> 12
   Gln Gln Ser Tyr Ser Ala Pro Leu Thr
   1 5
<210> 13
   <211> 9
   <212> PRT
   <213> Artificial Sequence
<220>
   <223> CDR-L3 derived from L3-1 clone
<400> 13
   Gln Gln Ser Tyr Ser Arg Pro Tyr Thr
   1 5
<210> 14
   <211> 9
   <212> PRT
   <213> Artificial Sequence
<220>
   <223> CDR-L3 derived from L3-2 clone
<400> 14
   Gly Gln Ser Tyr Ser Arg Pro Leu Thr
   1 5
<210> 15
   <211> 9
   <212> PRT
   <213> Artificial Sequence
<220>
   <223> CDR-L3 derived from L3-3 clone
<400> 15
   Ala Gln Ser Tyr Ser His Pro Phe Ser
   1 5
<210> 16
   <211> 9
   <212> PRT
   <213> Artificial Sequence
<220>
   <223> CDR-L3 derived from L3-5 clone
<400> 16
   Gln Gln Ser Tyr Ser Arg Pro Phe Thr
   1 5
<210> 17
   <211> 117
   <212> PRT
   <213> Artificial Sequence
<220>
   <223> heavy chain variable region of anti c-Met humanized antibody(huAbF46-H4)
<400> 17
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Thr Asp Tyr
   20 25 30
   Tyr Met Ser Trp Val Arg Gln Ala Pro Gly Lys Gly Leu Glu Trp Leu
   35 40 45
   Gly Phe Ile Arg Asn Lys Ala Asn Gly Tyr Thr Thr Glu Tyr Ser Ala
   50 55 60
   Ser Val Lys Gly Arg Phe Thr Ile Ser Arg Asp Asn Ser Lys Asn Thr
   65 70 75 80
   Leu Tyr Leu Gln Met Asn Ser Leu Arg Ala Glu Asp Thr Ala Val Tyr
   85 90 95
   Tyr Cys Ala Arg Asp Asn Trp Phe Ala Tyr Trp Gly Gln Gly Thr Leu
   100 105 110
   Val Thr Val Ser Ser
   115
<210> 18
   <211> 114
   <212> PRT
   <213> Artificial Sequence
<220>
   <223> light chain variable region of anti c-Met humanized antibody(huAbF46-H4)
<400> 18
   Asp Ile Gln Met Thr Gln Ser Pro Ser Ser Leu Ser Ala Ser Val Gly
   1               5                   10                  15
   Asp Arg Val Thr Ile Thr Cys Lys Ser Ser Gln Ser Leu Leu Ala Ser
               20                  25                  30
   Gly Asn Gln Asn Asn Tyr Leu Ala Trp His Gln Gln Lys Pro Gly Lys
           35                  40                  45
   Ala Pro Lys Met Leu Ile Ile Trp Ala Ser Thr Arg Val Ser Gly Val
       50                  55                  60
   Pro Ser Arg Phe Ser Gly Ser Gly Ser Gly Thr Asp Phe Thr Leu Thr
   65                  70                  75                  80
   Ile Ser Ser Leu Gln Pro Glu Asp Phe Ala Thr Tyr Tyr Cys Gln Gln
                   85                  90                  95
   Ser Tyr Ser Arg Pro Tyr Thr Phe Gly Gln Gly Thr Lys Val Glu Ile
               100                 105                 110
   Lys Arg
<210> 19
   <211> 114
   <212> PRT
   <213> Artificial Sequence
<220>
   <223> light chain variable region of anti c-Met humanized antibody(huAbF46-H4)
<400> 19
   Asp Ile Gln Met Thr Gln Ser Pro Ser Ser Leu Ser Ala Ser Val Gly
   1               5                   10                  15
   Asp Arg Val Thr Ile Thr Cys Lys Ser Ser Gln Ser Leu Leu Ala Ser
               20                  25                  30
   Gly Asn Gln Asn Asn Tyr Leu Ala Trp His Gln Gln Lys Pro Gly Lys
           35                  40                  45
   Ala Pro Lys Met Leu Ile Ile Trp Ala Ser Thr Arg Val Ser Gly Val
        50                  55                  60
   Pro Ser Arg Phe Ser Gly Ser Gly Ser Gly Thr Asp Phe Thr Leu Thr
    65                  70                  75                  80
   Ile Ser Ser Leu Gln Pro Glu Asp Phe Ala Thr Tyr Tyr Cys Gly Gln
                    85                  90                  95
   Ser Tyr Ser Arg Pro Leu Thr Phe Gly Gln Gly Thr Lys Val Glu Ile
               100                 105                 110
   Lys Arg
<210> 20
   <211> 114
   <212> PRT
   <213> Artificial Sequence
<220>
   <223> light chain variable region of anti c-Met humanized antibody(huAbF46-H4)
<400> 20
   Asp Ile Gln Met Thr Gln Ser Pro Ser Ser Leu Ser Ala Ser Val Gly
     1               5                  10                  15
   Asp Arg Val Thr Ile Thr Cys Lys Ser Ser Gln Ser Leu Leu Ala Ser
                20                  25                  30
   Gly Asn Gln Asn Asn Tyr Leu Ala Trp His Gln Gln Lys Pro Gly Lys
            35                  40                  45
   Ala Pro Lys Met Leu Ile Ile Trp Ala Ser Thr Arg Val Ser Gly Val
        50                  55                  60
   Pro Ser Arg Phe Ser Gly Ser Gly Ser Gly Thr Asp Phe Thr Leu Thr
    65                  70                  75                  80
   Ile Ser Ser Leu Gln Pro Glu Asp Phe Ala Thr Tyr Tyr Cys Ala Gln
                    85                  90                  95
   Ser Tyr Ser His Pro Phe Ser Phe Gly Gln Gly Thr Lys Val Glu Ile
               100                 105                 110
   Lys Arg
<210> 21
   <211> 114
   <212> PRT
   <213> Artificial Sequence
<220>
   <223> light chain variable region of anti c-Met humanized antibody(huAbF46-H4)
<400> 21
   Asp Ile Gln Met Thr Gln Ser Pro Ser Ser Leu Ser Ala Ser Val Gly
   1 5 10 15
   Asp Arg Val Thr Ile Thr Cys Lys Ser Ser Gln Ser Leu Leu Ala Ser
   20 25 30
   Gly Asn Gln Asn Asn Tyr Leu Ala Trp His Gln Gln Lys Pro Gly Lys
   35 40 45
   Ala Pro Lys Met Leu Ile Ile Trp Ala Ser Thr Arg Val Ser Gly Val
   50 55 60
   Pro Ser Arg Phe Ser Gly Ser Gly Ser Gly Thr Asp Phe Thr Leu Thr
   65 70 75 80
   Ile Ser Ser Leu Gln Pro Glu Asp Phe Ala Thr Tyr Tyr Cys Gln Gln
   85 90 95
   Ser Tyr Ser Arg Pro Phe Thr Phe Gly Gln Gly Thr Lys Val Glu Ile
   100 105 110
   Lys Arg
<210> 22
   <211> 6
   <212> PRT
   <213> Artificial Sequence
<220>
   <223> CDR-H1 derived from H11-4 clone
<400> 22
   Pro Glu Tyr Tyr Met Ser
   1 5
<210> 23
   <211> 6
   <212> PRT
   <213> Artificial Sequence
<220>
   <223> CDR-H1 derived from YC151 clone
<400> 23
   Pro Asp Tyr Tyr Met Ser
   1 5
<210> 24
   <211> 6
   <212> PRT
   <213> Artificial Sequence
<220>
   <223> CDR-H1 derived from YC193 clone
<400> 24
   Ser Asp Tyr Tyr Met Ser
   1 5
<210> 25
   <211> 8
   <212> PRT
   <213> Artificial Sequence
<220>
   <223> CDR-H2 derived from YC244 clone
<400> 25
   Arg Asn Asn Ala Asn Gly Asn Thr
   1 5
<210> 26
   <211> 8
   <212> PRT
   <213> Artificial Sequence
<220>
   <223> CDR-H2 derived from YC321 clone
<400> 26
   Arg Asn Lys Val Asn Gly Tyr Thr
   1 5
<210> 27
   <211> 6
   <212> PRT
   <213> Artificial Sequence
<220>
   <223> CDR-H3 derived from YC354 clone
<400> 27
   Asp Asn Trp Leu Ser Tyr
   1 5
<210> 28
   <211> 6
   <212> PRT
   <213> Artificial Sequence
<220>
   <223> CDR-H3 derived from YC374 clone
<400> 28
   Asp Asn Trp Leu Thr Tyr
   1 5
<210> 29
   <211> 17
   <212> PRT
   <213> Artificial Sequence
<220>
   <223> CDR-L1 derived from L1-1 clone
<400> 29
   Lys Ser Ser His Ser Leu Leu Ala Ser Gly Asn Gln Asn Asn Tyr Leu
   1               5                   10                  15
   Ala
<210> 30
   <211> 17
   <212> PRT
   <213> Artificial Sequence
<220>
   <223> CDR-L1 derived from L1-3 clone
<400> 30
   Lys Ser Ser Arg Ser Leu Leu Ser Ser Gly Asn His Lys Asn Tyr Leu
   1               5                   10                  15
   Ala
<210> 31
   <211> 17
   <212> PRT
   <213> Artificial Sequence
<220>
   <223> CDR-L1 derived from L1-4 clone
<400> 31
   Lys Ser Ser Lys Ser Leu Leu Ala Ser Gly Asn Gln Asn Asn Tyr Leu
   1               5                   10                  15
   Ala
<210> 32
   <211> 17
   <212> PRT
   <213> Artificial Sequence
<220>
   <223> CDR-L1 derived from L1-12 clone
<400> 32
   Lys Ser Ser Arg Ser Leu Leu Ala Ser Gly Asn Gln Asn Asn Tyr Leu
   1               5                   10                  15
   Ala
<210> 33
   <211> 17
   <212> PRT
   <213> Artificial Sequence
<220>
   <223> CDR-L1 derived from L1-22 clone
<400> 33
   Lys Ser Ser His Ser Leu Leu Ala Ser Gly Asn Gln Asn Asn Tyr Leu
   1 5 10 15
   Ala
<210> 34
   <211> 7
   <212> PRT
   <213> Artificial Sequence
<220>
   <223> CDR-L2 derived from L2-9 clone
<400> 34
   Trp Ala Ser Lys Arg Val Ser
   1 5
<210> 35
   <211> 7
   <212> PRT
   <213> Artificial Sequence
<220>
   <223> CDR-L2 derived from L2-12 clone
<400> 35
   Trp Gly Ser Thr Arg Val Ser
   1 5
<210> 36
   <211> 7
   <212> PRT
   <213> Artificial Sequence
<220>
   <223> CDR-L2 derived from L2-16 clone
<400> 36
   Trp Gly Ser Thr Arg Val Pro
   1 5
<210> 37
   <211> 9
   <212> PRT
   <213> Artificial Sequence
<220>
   <223> CDR-L3 derived from L3-32 clone
<400> 37
   Gln Gln Ser Tyr Ser Lys Pro Phe Thr
   1 5
<210> 38
   <211> 1416
   <212> DNA
   <213> Artificial Sequence
<220>
   <223> nucleotide sequence of heavy chain of chAbF46
<220>
   <221> misc_feature
   <222> (1)..(6)
   <223> EcoRI restriction site
<220>
   <221> misc_feature
   <222> (7)..(66)
   <223> signal sequence
<220>
   <221> misc_feature
   <222> (67)..(417)
   <223> VH - heavy chain variable region
<220>
   <221> misc_feature
   <222> (418)..(423)
   <223> NdeI restriction site
<220>
   <221> misc_feature
   <222> (418)..(1407)
   <223> CH - heavy chain constant region
<220>
   <221> misc_feature
   <222> (1408)..(1410)
   <223> TGA - stop sodon
<220>
   <221> misc_feature
   <222> (1411)..(1416)
   <223> Xhol restriction site
<400> 38
   gaattcgccg ccaccatgga atggagctgg gtttttctcg taacactttt aaatggtatc      60
   cagtgtgagg tgaagctggt ggagtctgga ggaggcttgg tacagcctgg gggttctctg     120
   agactctcct gtgcaacttc tgggttcacc ttcactgatt actacatgag ctgggtccgc     180
   cagcctccag gaaaggcact tgagtggttg ggttttatta gaaacaaagc taatggttac     240
   acaacagagt acagtgcatc tgtgaagggt cggttcacca tctccagaga taattcccaa     300
   agcatcctct atcttcaaat ggacaccctg agagctgagg acagtgccac ttattactgt     360
   gcaagagata actggtttgc ttactggggc caagggactc tggtcactgt ctctgcagct     420
   agcaccaagg gcccatcggt cttccccctg gcaccctcct ccaagagcac ctctgggggc     480
   acagcggccc tgggctgcct ggtcaaggac tacttccccg aaccggtgac ggtgtcgtgg     540
   aactcaggcg ccctgaccag cggcgtgcac accttcccgg ctgtcctaca gtcctcagga     600
   ctctactccc tcagcagcgt ggtgaccgtg ccctccagca gcttgggcac ccagacctac     660
   atctgcaacg tgaatcacaa gcccagcaac accaaggtgg acaagaaagt tgagcccaaa     720
   tcttgtgaca aaactcacac atgcccaccg tgcccagcac ctgaactcct ggggggaccg     780
   tcagtcttcc tcttcccccc aaaacccaag gacaccctca tgatctcccg gacccctgag     840
   gtcacatgcg tggtggtgga cgtgagccac gaagaccctg aggtcaagtt caactggtac     900
   gtggacggcg tggaggtgca taatgccaag acaaagccgc gggaggagca gtacaacagc     960
   acgtaccgtg tggtcagcgt cctcaccgtc ctgcaccagg actggctgaa tggcaaggag    1020
   tacaagtgca aggtctccaa caaagccctc ccagccccca tcgagaaaac catctccaaa    1080
   gccaaagggc agccccgaga accacaggtg tacaccctgc ccccatcccg ggaggagatg    1140
   accaagaacc aggtcagcct gacctgcctg gtcaaaggct tctatcccag cgacatcgcc    1200
   gtggagtggg agagcaatgg gcagccggag aacaactaca agaccacgcc tcccgtgctg    1260
   gactccgacg gctccttctt cctctacagc aagctcaccg tggacaagag caggtggcag    1320
   caggggaacg tcttctcatg ctccgtgatg catgaggctc tgcacaacca ctacacgcag    1380
   aagagcctct ccctgtctcc gggtaaatga ctcgag                              1416
<210> 39
   <211> 759
   <212> DNA
   <213> Artificial Sequence
<220>
   <223> nucleotide sequence of light chain of chAbF46
<220>
   <221> misc_difference
   <222> (1)..(6)
   <223> EcoRI restriction site
<220>
   <221> misc_difference
   <222> (7)..(90)
   <223> signal sequence
<220>
   <221> misc_difference
   <222> (91)..(432)
   <223> VL - light chain variable region
<220>
   <221> misc_difference
   <222> (430)..(435)
   <223> BsiWI restriction site
<220>
   <221> misc_difference
   <222> (433)..(750)
   <223> CL - light chain constant region
<220>
   <221> misc_difference
   <222> (751)..(753)
   <223> stop codon
<220>
   <221> misc_difference
   <222> (754)..(759)
   <223> Xhol restriction site
<400> 39
   gaattcacta gtgattaatt cgccgccacc atggattcac aggcccaggt cctcatgttg        60
   ctgctgctat cggtatctgg tacctgtgga gacattttga tgacccagtc tccatcctcc       120
   ctgactgtgt cagcaggaga gaaggtcact atgagctgca agtccagtca gagtctttta       180
   gctagtggca accaaaataa ctacttggcc tggcaccagc agaaaccagg acgatctcct       240
   aaaatgctga taatttgggc atccactagg gtatctggag tccctgatcg cttcataggc       300
   agtggatctg ggacggattt cactctgacc atcaacagtg tgcaggctga agatctggct       360
   gtttattact gtcagcagtc ctacagcgct ccgctcacgt tcggtgctgg gaccaagctg       420
   gagctgaaac gtacggtggc tgcaccatct gtcttcatct tcccgccatc tgatgagcag       480
   ttgaaatctg gaactgcctc tgttgtgtgc ctgctgaata acttctatcc cagagaggcc       540
   aaagtacagt ggaaggtgga taacgccctc caatcgggta actcccagga gagtgtcaca       600
   gagcaggaca gcaaggacag cacctacagc ctcagcagca ccctgacgct gagcaaagca       660
   gactacgaga aacacaaagt ctacgcctgc gaagtcaccc atcagggcct gagctcgccc       720
   gtcacaaaga gcttcaacag gggagagtgt tgactcgag                              759
<210> 40
   <211> 447
   <212> PRT
   <213> Artificial Sequence
<220>
   <223> amino acid sequence of H1-heavy
<400> 40
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Thr Asp Tyr
               20                  25                  30
   Tyr Met Ser Trp Val Arg Gln Ala Pro Gly Lys Gly Leu Glu Trp Leu
           35                  40                  45
   Gly Phe Ile Arg Asn Lys Ala Asn Gly Tyr Thr Thr Glu Tyr Ser Ala
       50                  55                  60
   Ser Val Lys Gly Arg Phe Thr Ile Ser Arg Asp Asn Ser Lys Asn Ser
   65                  70                  75                  80
   Leu Tyr Leu Gln Met Asn Ser Leu Lys Thr Glu Asp Thr Ala Val Tyr
                   85                  90                  95
   Tyr Cys Ala Arg Asp Asn Trp Phe Ala Tyr Trp Gly Gln Gly Thr Leu
               100                 105                 110
   Val Thr Val Ser Ser Ala Ser Thr Lys Gly Pro Ser Val Phe Pro Leu
           115                 120                 125
   Ala Pro Ser Ser Lys Ser Thr Ser Gly Gly Thr Ala Ala Leu Gly Cys
       130                 135                 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ala Val Leu Gln Ser
                   165                 170                 175
   Ser Gly Leu Tyr Ser Leu Ser Ser Val Val Thr Val Pro Ser Ser Ser
               180                 185                 190
   Leu Gly Thr Gln Thr Tyr Ile Cys Asn Val Asn His Lys Pro Ser Asn
           195                 200                 205
   Thr Lys Val Asp Lys Lys Val Glu Pro Lys Ser Cys Asp Lys Thr His
       210                 215                 220
   Thr Cys Pro Pro Cys Pro Ala Pro Glu Leu Leu Gly Gly Pro Ser Val
   225                 230                 235                 240
   Phe Leu Phe Pro Pro Lys Pro Lys Asp Thr Leu Met Ile Ser Arg Thr
                   245                 250                 255
   Pro Glu Val Thr Cys Val Val Val Asp Val Ser His Glu Asp Pro Glu
               260                 265                 270
   Val Lys Phe Asn Trp Tyr Val Asp Gly Val Glu Val His Asn Ala Lys
   275 280 285
   Thr Lys Pro Arg Glu Glu Gln Tyr Asn Ser Thr Tyr Arg Val Val Ser
   290 295 300
   Val Leu Thr Val Leu His Gln Asp Trp Leu Asn Gly Lys Glu Tyr Lys
   305 310 315 320
   Cys Lys Val Ser Asn Lys Ala Leu Pro Ala Pro Ile Glu Lys Thr Ile
   325 330 335
   Ser Lys Ala Lys Gly Gln Pro Arg Glu Pro Gln Val Tyr Thr Leu Pro
   340 345 350
   Pro Ser Arg Glu Glu Met Thr Lys Asn Gln Val Ser Leu Thr Cys Leu
   355 360 365
   Val Lys Gly Phe Tyr Pro Ser Asp Ile Ala Val Glu Trp Glu Ser Asn
   370 375 380
   Gly Gln Pro Glu Asn Asn Tyr Lys Thr Thr Pro Pro Val Leu Asp Ser
   385 390 395 400
   Asp Gly Ser Phe Phe Leu Tyr Ser Lys Leu Thr Val Asp Lys Ser Arg
   405 410 415
   Trp Gln Gln Gly Asn Val Phe Ser Cys Ser Val Met His Glu Ala Leu
   420 425 430
   His Asn His Tyr Thr Gln Lys Ser Leu Ser Leu Ser Pro Gly Lys
   435 440 445
<210> 41
   <211> 447
   <212> PRT
   <213> Artificial Sequence
<220>
   <223> amino acid sequence of H3-heavy
<400> 41
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Thr Asp Tyr
   20 25 30
   Tyr Met Ser Trp Val Arg Gln Ala Pro Gly Lys Gly Leu Glu Trp Leu
   35 40 45
   Gly Phe Ile Arg Asn Lys Ala Asn Gly Tyr Thr Thr Glu Tyr Ser Ala
   50 55 60
   Ser Val Lys Gly Arg Phe Thr Ile Ser Arg Asp Asn Ser Lys Asn Ser
   65 70 75 80
   Leu Tyr Leu Gln Met Asn Ser Leu Arg Ala Glu Asp Thr Ala Val Tyr
                   85                  90                  95
   Tyr Cys Ala Arg Asp Asn Trp Phe Ala Tyr Trp Gly Gln Gly Thr Leu
               100                 105                 110
   Val Thr Val Ser Ser Ala Ser Thr Lys Gly Pro Ser Val Phe Pro Leu
           115                 120                 125
   Ala Pro Ser Ser Lys Ser Thr Ser Gly Gly Thr Ala Ala Leu Gly Cys
       130                 135                 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ala Val Leu Gln Ser
                   165                 170                 175
   Ser Gly Leu Tyr Ser Leu Ser Ser Val Val Thr Val Pro Ser Ser Ser
               180                 185                 190
   Leu Gly Thr Gln Thr Tyr Ile Cys Asn Val Asn His Lys Pro Ser Asn
           195                 200                 205
   Thr Lys Val Asp Lys Lys Val Glu Pro Lys Ser Cys Asp Lys Thr His
       210                 215                 220
   Thr Cys Pro Pro Cys Pro Ala Pro Glu Leu Leu Gly Gly Pro Ser Val
   225                 230                 235                 240
   Phe Leu Phe Pro Pro Lys Pro Lys Asp Thr Leu Met Ile Ser Arg Thr
                   245                 250                 255
   Pro Glu Val Thr Cys Val Val Val Asp Val Ser His Glu Asp Pro Glu
               260                 265                 270
   Val Lys Phe Asn Trp Tyr Val Asp Gly Val Glu Val His Asn Ala Lys
           275                 280                 285
   Thr Lys Pro Arg Glu Glu Gln Tyr Asn Ser Thr Tyr Arg Val Val Ser
       290                 295                 300
   Val Leu Thr Val Leu His Gln Asp Trp Leu Asn Gly Lys Glu Tyr Lys
   305                 310                 315                 320
   Cys Lys Val Ser Asn Lys Ala Leu Pro Ala Pro Ile Glu Lys Thr Ile
                   325                 330                 335
   Ser Lys Ala Lys Gly Gln Pro Arg Glu Pro Gln Val Tyr Thr Leu Pro
               340                 345                 350
   Pro Ser Arg Glu Glu Met Thr Lys Asn Gln Val Ser Leu Thr Cys Leu
           355                 360                 365
   Val Lys Gly Phe Tyr Pro Ser Asp Ile Ala Val Glu Trp Glu Ser Asn
       370                 375                 380
   Gly Gln Pro Glu Asn Asn Tyr Lys Thr Thr Pro Pro Val Leu Asp Ser
   385                 390                 395                 400
   Asp Gly Ser Phe Phe Leu Tyr Ser Lys Leu Thr Val Asp Lys Ser Arg
                   405                 410                 415
   Trp Gln Gln Gly Asn Val Phe Ser Cys Ser Val Met His Glu Ala Leu
   420 425 430
   His Asn His Tyr Thr Gln Lys Ser Leu Ser Leu Ser Pro Gly Lys
   435 440 445
<210> 42
   <211> 447
   <212> PRT
   <213> Artificial Sequence
<220>
   <223> amino acid sequence of H4-heavy
<400> 42
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Thr Asp Tyr
   20 25 30
   Tyr Met Ser Trp Val Arg Gln Ala Pro Gly Lys Gly Leu Glu Trp Leu
   35 40 45
   Gly Phe Ile Arg Asn Lys Ala Asn Gly Tyr Thr Thr Glu Tyr Ser Ala
   50 55 60
   Ser Val Lys Gly Arg Phe Thr Ile Ser Arg Asp Asn Ser Lys Asn Thr
   65 70 75 80
   Leu Tyr Leu Gln Met Asn Ser Leu Arg Ala Glu Asp Thr Ala Val Tyr
   85 90 95
   Tyr Cys Ala Arg Asp Asn Trp Phe Ala Tyr Trp Gly Gln Gly Thr Leu
   100 105 110
   Val Thr Val Ser Ser Ala Ser Thr Lys Gly Pro Ser Val Phe Pro Leu
   115 120 125
   Ala Pro Ser Ser Lys Ser Thr Ser Gly Gly Thr Ala Ala Leu Gly Cys
   130 135 140
   Leu Val Lys Asp Tyr Phe Pro Glu Pro Val Thr Val Ser Trp Asn Ser
   145 150 155 160
   Gly Ala Leu Thr Ser Gly Val His Thr Phe Pro Ala Val Leu Gln Ser
   165 170 175
   Ser Gly Leu Tyr Ser Leu Ser Ser Val Val Thr Val Pro Ser Ser Ser
   180 185 190
   Leu Gly Thr Gln Thr Tyr Ile Cys Asn Val Asn His Lys Pro Ser Asn
   195 200 205
   Thr Lys Val Asp Lys Lys Val Glu Pro Lys Ser Cys Asp Lys Thr His
   210 215 220
   Thr Cys Pro Pro Cys Pro Ala Pro Glu Leu Leu Gly Gly Pro Ser Val
   225 230 235 240
   Phe Leu Phe Pro Pro Lys Pro Lys Asp Thr Leu Met Ile Ser Arg Thr
                   245                 250                 255
   Pro Glu Val Thr Cys Val Val Val Asp Val Ser His Glu Asp Pro Glu
               260                 265                 270
   Val Lys Phe Asn Trp Tyr Val Asp Gly Val Glu Val His Asn Ala Lys
           275                 280                 285
   Thr Lys Pro Arg Glu Glu Gln Tyr Asn Ser Thr Tyr Arg Val Val Ser
       290                 295                 300
   Val Leu Thr Val Leu His Gln Asp Trp Leu Asn Gly Lys Glu Tyr Lys
   305                 310                 315                 320
   Cys Lys Val Ser Asn Lys Ala Leu Pro Ala Pro Ile Glu Lys Thr Ile
                   325                 330                 335
   Ser Lys Ala Lys Gly Gln Pro Arg Glu Pro Gln Val Tyr Thr Leu Pro
               340                 345                 350
   Pro Ser Arg Glu Glu Met Thr Lys Asn Gln Val Ser Leu Thr Cys Leu
           355                 360                 365
   Val Lys Gly Phe Tyr Pro Ser Asp Ile Ala Val Glu Trp Glu Ser Asn
       370                 375                 380
   Gly Gln Pro Glu Asn Asn Tyr Lys Thr Thr Pro Pro Val Leu Asp Ser
   385                 390                 395                 400
   Asp Gly Ser Phe Phe Leu Tyr Ser Lys Leu Thr Val Asp Lys Ser Arg
                   405                 410                 415
   Trp Gln Gln Gly Asn Val Phe Ser Cys Ser Val Met His Glu Ala Leu
               420                 425                 430
   His Asn His Tyr Thr Gln Lys Ser Leu Ser Leu Ser Pro Gly Lys
           435                 440                 445
<210> 43
   <211> 220
   <212> PRT
   <213> Artificial Sequence
<220>
   <223> amino acid sequence of H1-light
<400> 43
   Asp Ile Val Met Thr Gln Ser Pro Asp Ser Leu Ala Val Ser Leu Gly
   1               5                   10                  15
   Glu Arg Ala Thr Ile Asn Cys Lys Ser Ser Gln Ser Leu Leu Ala Ser
               20                  25                  30
   Gly Asn Gln Asn Asn Tyr Leu Ala Trp His Gln Gln Lys Pro Gly Gln
           35                  40                  45
   Pro Pro Lys Met Leu Ile Ile Trp Ala Ser Thr Arg Val Ser Gly Val
       50                  55                  60
   Pro Asp Arg Phe Ser Gly Ser Gly Ser Gly Thr Asp Phe Thr Leu Thr
   65                  70                  75                  80
   Ile Ser Ser Leu Gln Ala Glu Asp Val Ala Val Tyr Tyr Cys Gln Gln
                   85                  90                  95
   Ser Tyr Ser Ala Pro Leu Thr Phe Gly Gly Gly Thr Lys Val Glu Ile
               100                 105                 110
   Lys Arg Thr Val Ala Ala Pro Ser Val Phe Ile Phe Pro Pro Ser Asp
           115                 120                 125
   Glu Gln Leu Lys Ser Gly Thr Ala Ser Val Val Cys Leu Leu Asn Asn
       130                 135                 140
   Phe Tyr Pro Arg Glu Ala Lys Val Gln Trp Lys Val Asp Asn Ala Leu
   145                 150                 155                 160
   Gln Ser Gly Asn Ser Gln Glu Ser Val Thr Glu Gln Asp Ser Lys Asp
                   165                 170                 175
   Ser Thr Tyr Ser Leu Ser Ser Thr Leu Thr Leu Ser Lys Ala Asp Tyr
               180                 185                 190
   Glu Lys His Lys Val Tyr Ala Cys Glu Val Thr His Gln Gly Leu Ser
           195                 200                 205
   Ser Pro Val Thr Lys Ser Phe Asn Arg Gly Glu Cys
       210                 215                 220
<210> 44
   <211> 220
   <212> PRT
   <213> Artificial Sequence
<220>
   <223> amino acid sequence of H2-light
<400> 44
   Asp Ile Val Met Thr Gln Thr Pro Leu Ser Leu Pro Val Thr Pro Gly
   1               5                   10                  15
   Glu Pro Ala Ser Ile Ser Cys Lys Ser Ser Gln Ser Leu Leu Ala Ser
               20                  25                  30
   Gly Asn Gln Asn Asn Tyr Leu Ala Trp His Leu Gln Lys Pro Gly Gln
           35                  40                  45
   Ser Pro Gln Met Leu Ile Ile Trp Ala Ser Thr Arg Val Ser Gly Val
       50                  55                  60
   Pro Asp Arg Phe Ser Gly Ser Gly Ser Gly Thr Asp Phe Thr Leu Lys
   65                  70                  75                  80
   Ile Ser Arg Val Glu Ala Glu Asp Val Gly Val Tyr Tyr Cys Gln Gln
                   85                  90                  95
   Ser Tyr Ser Ala Pro Leu Thr Phe Gly Gln Gly Thr Lys Leu Glu Leu
               100                 105                 110
   Lys Arg Thr Val Ala Ala Pro Ser Val Phe Ile Phe Pro Pro Ser Asp
           115                 120                 125
   Glu Gln Leu Lys Ser Gly Thr Ala Ser Val Val Cys Leu Leu Asn Asn
       130                 135                 140
   Phe Tyr Pro Arg Glu Ala Lys Val Gln Trp Lys Val Asp Asn Ala Leu
   145                 150                 155                 160
   Gln Ser Gly Asn Ser Gln Glu Ser Val Thr Glu Gln Asp Ser Lys Asp
                   165                 170                 175
   Ser Thr Tyr Ser Leu Ser Ser Thr Leu Thr Leu Ser Lys Ala Asp Tyr
               180                 185                 190
   Glu Lys His Lys Val Tyr Ala Cys Glu Val Thr His Gln Gly Leu Ser
           195                 200                 205
   Ser Pro Val Thr Lys Ser Phe Asn Arg Gly Glu Cys
       210                 215                 220
<210> 45
   <211> 220
   <212> PRT
   <213> Artificial Sequence
<220>
   <223> amino acid sequence of H3-light
<400> 45
   Asp Ile Val Met Thr Gln Ser Pro Asp Ser Leu Ala Val Ser Leu Gly
   1               5                   10                  15
   Glu Arg Ala Thr Ile Asn Cys Lys Ser Ser Gln Ser Leu Leu Ala Ser
               20                  25                  30
   Gly Asn Gln Asn Asn Tyr Leu Ala Trp Tyr Gln Gln Lys Pro Gly Gln
           35                  40                  45
   Pro Pro Lys Leu Leu Ile Ile Trp Ala Ser Thr Arg Val Ser Gly Val
       50                  55                  60
   Pro Asp Arg Phe Ser Gly Ser Gly Ser Gly Thr Asp Phe Thr Leu Thr
   65                  70                  75                  80
   Ile Ser Ser Leu Gln Ala Glu Asp Val Ala Val Tyr Tyr Cys Gln Gln
                   85                  90                  95
   Ser Tyr Ser Ala Pro Leu Thr Phe Gly Gly Gly Thr Lys Val Glu Ile
               100                 105                 110
   Lys Arg Thr Val Ala Ala Pro Ser Val Phe Ile Phe Pro Pro Ser Asp
           115                 120                 125
   Glu Gln Leu Lys Ser Gly Thr Ala Ser Val Val Cys Leu Leu Asn Asn
       130                 135                 140
   Phe Tyr Pro Arg Glu Ala Lys Val Gln Trp Lys Val Asp Asn Ala Leu
   145                 150                 155                 160
   Gln Ser Gly Asn Ser Gln Glu Ser Val Thr Glu Gln Asp Ser Lys Asp
                   165                 170                 175
   Ser Thr Tyr Ser Leu Ser Ser Thr Leu Thr Leu Ser Lys Ala Asp Tyr
               180                 185                 190
   Glu Lys His Lys Val Tyr Ala Cys Glu Val Thr His Gln Gly Leu Ser
           195                 200                 205
   Ser Pro Val Thr Lys Ser Phe Asn Arg Gly Glu Cys
       210                 215                 220
<210> 46
   <211> 219
   <212> PRT
   <213> Artificial Sequence
<220>
   <223> amino acid sequence of H4-light
<400> 46
   Asp Ile Gln Met Thr Gln Ser Pro Ser Ser Leu Ser Ala Ser Val Gly
    1               5                  10                  15
   Asp Arg Val Thr Ile Thr Cys Lys Ser Ser Gln Ser Leu Leu Ala Ser
               20                  25                  30
   Gly Asn Gln Asn Asn Tyr Leu Ala Trp His Gln Gln Lys Pro Gly Lys
           35                  40                  45
   Ala Pro Lys Met Leu Ile Ile Trp Ala Ser Thr Arg Val Ser Gly Val
       50                  55                  60
   Pro Ser Arg Phe Ser Gly Ser Gly Ser Gly Thr Asp Phe Thr Leu Thr
    65                  70                  75                  80
   Ile Ser Ser Leu Gln Pro Glu Asp Phe Ala Thr Tyr Tyr Cys Gln Gln
                   85                  90                  95
   Ser Tyr Ser Ala Pro Leu Thr Phe Gly Gln Gly Thr Lys Val Glu Ile
               100                 105                 110
   Lys Arg Thr Val Ala Ala Pro Ser Val Phe Ile Phe Pro Pro Ser Asp
           115                 120                 125
   Glu Gln Leu Lys Ser Gly Thr Ala Ser Val Val Cys Leu Leu Asn Asn
       130                 135                 140
   Phe Tyr Pro Arg Glu Ala Lys Val Gln Trp Lys Val Asp Asn Ala Leu
   145                 150                 155                 160
   Gln Ser Gly Asn Ser Gln Glu Ser Val Thr Glu Gln Asp Ser Lys Asp
                   165                 170                 175
   Ser Thr Tyr Ser Leu Ser Ser Thr Leu Thr Leu Ser Lys Ala Asp Tyr
               180                 185                 190
   Glu Lys His Lys Val Tyr Ala Cys Glu Val Thr His Gln Gly Leu Ser
           195                 200                 205
   Ser Pro Val Thr Lys Ser Phe Asn Arg Gly Glu
       210                 215
<210> 47
   <211> 1350
   <212> DNA
   <213> Artificial Sequence
<220>
   <223> nucleotide sequence of H1-heavy
<400> 47
   gaggtgcagc tggtggagtc tgggggaggc ttggtccagc ctggagggtc cctgagactc 60
   tcctgtgcag cctctggatt caccttcact gactactaca tgagctgggt ccgccaggct 120
   ccagggaagg ggctggagtg gttgggcttt attagaaaca aagctaacgg ttacaccaca 180
   gaatacagtg cgtctgtgaa aggcagattc accatctcaa gagataattc aaagaactca 240
   ctgtatctgc aaatgaacag cctgaaaacc gaggacacgg ccgtgtatta ctgtgctaga 300
   gataactggt ttgcttactg gggtcaagga accctggtca ccgtctcctc ggctagcacc 360
   aagggcccat cggtcttccc cctggcaccc tcctccaaga gcacctctgg gggcacagcg 420
   gccctgggct gcctggtcaa ggactacttc cccgaaccgg tgacggtgtc gtggaactca 480
   ggcgccctga ccagcggcgt gcacaccttc ccggctgtcc tacagtcctc aggactctac 540
   tccctcagca gcgtggtgac cgtgccctcc agcagcttgg gcacccagac ctacatctgc 600
   aacgtgaatc acaagcccag caacaccaag gtggacaaga aagttgagcc caaatcttgt 660
   gacaaaactc acacatgccc accgtgccca gcacctgaac tcctgggggg accgtcagtc 720
   ttcctcttcc ccccaaaacc caaggacacc ctcatgatct cccggacccc tgaggtcaca 780
   tgcgtggtgg tggacgtgag ccacgaagac cctgaggtca agttcaactg gtacgtggac 840
   ggcgtggagg tgcataatgc caagacaaag ccgcgggagg agcagtacaa cagcacgtac 900
   cgtgtggtca gcgtcctcac cgtcctgcac caggactggc tgaatggcaa ggagtacaag 960
   tgcaaggtct ccaacaaagc cctcccagcc cccatcgaga aaaccatctc caaagccaaa 1020
   gggcagcccc gagaaccaca ggtgtacacc ctgcccccat cccgggagga gatgaccaag 1080
   aaccaggtca gcctgacctg cctggtcaaa ggcttctatc ccagcgacat cgccgtggag 1140
   tgggagagca atgggcagcc ggagaacaac tacaagacca cgcctcccgt gctggactcc 1200
   gacggctcct tcttcctcta cagcaagctc accgtggaca agagcaggtg gcagcagggg 1260
   aacgtcttct catgctccgt gatgcatgag gctctgcaca accactacac gcagaagagc 1320
   ctctccctgt ctccgggtaa atgactcgag 1350
<210> 48
   <211> 1350
   <212> DNA
   <213> Artificial Sequence
<220>
   <223> nucleotide sequence of H3-heavy
<400> 48
   gaggtgcagc tggtggagtc tgggggaggc ttggtccagc ctggagggtc cctgagactc        60
   tcctgtgcag cctctggatt caccttcact gactactaca tgagctgggt ccgccaggct       120
   ccagggaagg ggctggagtg gttgggcttt attagaaaca aagctaacgg ttacaccaca       180
   gaatacagtg cgtctgtgaa aggcagattc accatctcaa gagataattc aaagaactca       240
   ctgtatctgc aaatgaacag cctgcgtgct gaggacacgg ccgtgtatta ctgtgctaga       300
   gataactggt ttgcttactg gggtcaagga accctggtca ccgtctcctc ggctagcacc       360
   aagggcccat cggtcttccc cctggcaccc tcctccaaga gcacctctgg gggcacagcg       420
   gccctgggct gcctggtcaa ggactacttc cccgaaccgg tgacggtgtc gtggaactca       480
   ggcgccctga ccagcggcgt gcacaccttc ccggctgtcc tacagtcctc aggactctac       540
   tccctcagca gcgtggtgac cgtgccctcc agcagcttgg gcacccagac ctacatctgc       600
   aacgtgaatc acaagcccag caacaccaag gtggacaaga aagttgagcc caaatcttgt       660
   gacaaaactc acacatgccc accgtgccca gcacctgaac tcctgggggg accgtcagtc       720
   ttcctcttcc ccccaaaacc caaggacacc ctcatgatct cccggacccc tgaggtcaca       780
   tgcgtggtgg tggacgtgag ccacgaagac cctgaggtca agttcaactg gtacgtggac       840
   ggcgtggagg tgcataatgc caagacaaag ccgcgggagg agcagtacaa cagcacgtac       900
   cgtgtggtca gcgtcctcac cgtcctgcac caggactggc tgaatggcaa ggagtacaag       960
   tgcaaggtct ccaacaaagc cctcccagcc cccatcgaga aaaccatctc caaagccaaa      1020
   gggcagcccc gagaaccaca ggtgtacacc ctgcccccat cccgggagga gatgaccaag      1080
   aaccaggtca gcctgacctg cctggtcaaa ggcttctatc ccagcgacat cgccgtggag      1140
   tgggagagca atgggcagcc ggagaacaac tacaagacca cgcctcccgt gctggactcc      1200
   gacggctcct tcttcctcta cagcaagctc accgtggaca agagcaggtg gcagcagggg      1260
   aacgtcttct catgctccgt gatgcatgag gctctgcaca accactacac gcagaagagc      1320
   ctctccctgt ctccgggtaa atgactcgag                                        1350
<210> 49
   <211> 1350
   <212> DNA
   <213> Artificial Sequence
<220>
   <223> nucleotide sequence of H4-heavy
<400> 49
   gaggttcagc tggtggagtc tggcggtggc ctggtgcagc cagggggctc actccgtttg          60
   tcctgtgcag cttctggctt caccttcact gattactaca tgagctgggt gcgtcaggcc         120
   ccgggtaagg gcctggaatg gttgggtttt attagaaaca aagctaatgg ttacacaaca         180
   gagtacagtg catctgtgaa gggtcgtttc actataagca gagataattc caaaaacaca         240
   ctgtacctgc agatgaacag cctgcgtgct gaggacactg ccgtctatta ttgtgctaga         300
   gataactggt ttgcttactg gggccaaggg actctggtca ccgtctcctc ggctagcacc         360
   aagggcccat cggtcttccc cctggcaccc tcctccaaga gcacctctgg gggcacagcg         420
   gccctgggct gcctggtcaa ggactacttc cccgaaccgg tgacggtgtc gtggaactca         480
   ggcgccctga ccagcggcgt gcacaccttc ccggctgtcc tacagtcctc aggactctac         540
   tccctcagca gcgtggtgac cgtgccctcc agcagcttgg gcacccagac ctacatctgc         600
   aacgtgaatc acaagcccag caacaccaag gtggacaaga aagttgagcc caaatcttgt         660
   gacaaaactc acacatgccc accgtgccca gcacctgaac tcctgggggg accgtcagtc         720
   ttcctcttcc ccccaaaacc caaggacacc ctcatgatct cccggacccc tgaggtcaca         780
   tgcgtggtgg tggacgtgag ccacgaagac cctgaggtca agttcaactg gtacgtggac         840
   ggcgtggagg tgcataatgc caagacaaag ccgcgggagg agcagtacaa cagcacgtac         900
   cgtgtggtca gcgtcctcac cgtcctgcac caggactggc tgaatggcaa ggagtacaag         960
   tgcaaggtct ccaacaaagc cctcccagcc cccatcgaga aaaccatctc caaagccaaa        1020
   gggcagcccc gagaaccaca ggtgtacacc ctgcccccat cccgggagga gatgaccaag        1080
   aaccaggtca gcctgacctg cctggtcaaa ggcttctatc ccagcgacat cgccgtggag        1140
   tgggagagca atgggcagcc ggagaacaac tacaagacca cgcctcccgt gctggactcc        1200
   gacggctcct tcttcctcta cagcaagctc accgtggaca agagcaggtg gcagcagggg        1260
   aacgtcttct catgctccgt gatgcatgag gctctgcaca accactacac gcagaagagc        1320
   ctctccctgt ctccgggtaa atgactcgag                                         1350
<210> 50
   <211> 669
   <212> DNA
   <213> Artificial Sequence
<220>
   <223> nucleotide sequence of H1-light
<400> 50
   gacatcgtga tgacccagtc tccagactcc ctggctgtgt ctctgggcga gagggccacc 60
   atcaactgca agtccagcca gagtctttta gctagcggca accaaaataa ctacttagct 120
   tggcaccagc agaaaccagg acagcctcct aagatgctca ttatttgggc atctacccgg 180
   gtatccgggg tccctgaccg attcagtggc agcgggtctg ggacagattt cactctcacc 240
   atcagcagcc tgcaggctga agatgtggca gtttattact gtcagcaatc ctatagtgct 300
   cctctcacgt tcggaggcgg taccaaggtg gagatcaaac gtacggtggc tgcaccatct 360
   gtcttcatct tcccgccatc tgatgagcag ttgaaatctg gaactgcctc tgttgtgtgc 420
   ctgctgaata acttctatcc cagagaggcc aaagtacagt ggaaggtgga taacgccctc 480
   caatcgggta actcccagga gagtgtcaca gagcaggaca gcaaggacag cacctacagc 540
   ctcagcagca ccctgacgct gagcaaagca gactacgaga aacacaaagt ctacgcctgc 600
   gaagtcaccc atcagggcct gagctcgccc gtcacaaaga gcttcaacag gggagagtgt 660
   tgactcgag 669
<210> 51
   <211> 669
   <212> DNA
   <213> Artificial Sequence
<220>
   <223> nucleotide sequence of H2-light
<400> 51
   gatattgtga tgacccagac tccactctcc ctgcccgtca cccctggaga gccggcctcc 60
   atctcctgca agtccagtca gagtctttta gctagtggca accaaaataa ctacttggcc 120
   tggcacctgc agaagccagg gcagtctcca cagatgctga tcatttgggc atccactagg 180
   gtatctggag tcccagacag gttcagtggc agtgggtcag gcactgattt cacactgaaa 240
   atcagcaggg tggaggctga ggatgttgga gtttattact gccagcagtc ctacagcgct 300
   ccgctcacgt tcggacaggg taccaagctg gagctcaaac gtacggtggc tgcaccatct 360
   gtcttcatct tcccgccatc tgatgagcag ttgaaatctg gaactgcctc tgttgtgtgc 420
   ctgctgaata acttctatcc cagagaggcc aaagtacagt ggaaggtgga taacgccctc 480
   caatcgggta actcccagga gagtgtcaca gagcaggaca gcaaggacag cacctacagc 540
   ctcagcagca ccctgacgct gagcaaagca gactacgaga aacacaaagt ctacgcctgc 600
   gaagtcaccc atcagggcct gagctcgccc gtcacaaaga gcttcaacag gggagagtgt 660
   tgactcgag 669
<210> 52
   <211> 669
   <212> DNA
   <213> Artificial Sequence
<220>
   <223> nucleotide sequence of H3-light
<400> 52
   gacatcgtga tgacccagtc tccagactcc ctggctgtgt ctctgggcga gagggccacc        60
   atcaactgca agtccagcca gagtctttta gctagcggca accaaaataa ctacttagct       120
   tggtaccagc agaaaccagg acagcctcct aagctgctca ttatttgggc atctacccgg       180
   gtatccgggg tccctgaccg attcagtggc agcgggtctg ggacagattt cactctcacc       240
   atcagcagcc tgcaggctga agatgtggca gtttattact gtcagcaatc ctatagtgct       300
   cctctcacgt tcggaggcgg taccaaggtg gagatcaaac gtacggtggc tgcaccatct       360
   gtcttcatct tcccgccatc tgatgagcag ttgaaatctg gaactgcctc tgttgtgtgc       420
   ctgctgaata acttctatcc cagagaggcc aaagtacagt ggaaggtgga taacgccctc       480
   caatcgggta actcccagga gagtgtcaca gagcaggaca gcaaggacag cacctacagc       540
   ctcagcagca ccctgacgct gagcaaagca gactacgaga aacacaaagt ctacgcctgc       600
   gaagtcaccc atcagggcct gagctcgccc gtcacaaaga gcttcaacag gggagagtgt       660
   tgactcgag                                                               669
<210> 53
   <211> 669
   <212> DNA
   <213> Artificial Sequence
<220>
   <223> nucleotide sequence of H4-light
<400> 53
   gatatccaga tgacccagtc cccgagctcc ctgtccgcct ctgtgggcga tagggtcacc 60
   atcacctgca agtccagtca gagtctttta gctagtggca accaaaataa ctacttggcc 120
   tggcaccaac agaaaccagg aaaagctccg aaaatgctga ttatttgggc atccactagg 180
   gtatctggag tcccttctcg cttctctgga tccgggtctg ggacggattt cactctgacc 240
   atcagcagtc tgcagccgga agacttcgca acttattact gtcagcagtc ctacagcgct 300
   ccgctcacgt tcggacaggg taccaaggtg gagatcaaac gtacggtggc tgcaccatct 360
   gtcttcatct tcccgccatc tgatgagcag ttgaaatctg gaactgcctc tgttgtgtgc 420
   ctgctgaata acttctatcc cagagaggcc aaagtacagt ggaaggtgga taacgccctc 480
   caatcgggta actcccagga gagtgtcaca gagcaggaca gcaaggacag cacctacagc 540
   ctcagcagca ccctgacgct gagcaaagca gactacgaga aacacaaagt ctacgcctgc 600
   gaagtcaccc atcagggcct gagctcgccc gtcacaaaga gcttcaacag gggagagtgt 660
   tgactcgag 669
<210> 54
   <211> 23
   <212> PRT
   <213> Artificial Sequence
<220>
   <223> linker between VH and VL
<400> 54
   Gly Leu Gly Gly Leu Gly Gly Gly Gly Ser Gly Gly Gly Gly Ser Gly
   1 5 10 15
   Gly Ser Ser Gly Val Gly Ser
   20
<210> 55
   <211> 1088
   <212> DNA
   <213> Artificial Sequence
<220>
   <223> polynucleotide encoding scFv of huAbF46 antibody
<400> 55
   gctagcgttt tagcagaagt tcaattggtt gaatctggtg gtggtttggt tcaaccaggt 60
   ggttctttga gattgtcttg tgctgcttct ggttttactt tcaccgatta ttacatgtcc 120
   tgggttagac aagctccagg taaaggtttg gaatggttgg gtttcattag aaacaaggct 180
   aacggttaca ctaccgaata ttctgcttct gttaagggta gattcaccat ttctagagac 240
   aactctaaga acaccttgta cttgcaaatg aactccttga gagctgaaga tactgctgtt 300
   tattactgcg ctagagataa ttggtttgct tattggggtc aaggtacttt ggttactgtt 360
   tcttctggcc tcgggggcct cggaggagga ggtagtggcg gaggaggctc cggtggatcc 420
   agcggtgtgg gttccgatat tcaaatgacc caatctccat cttctttgtc tgcttcagtt 480
   ggtgatagag ttaccattac ttgtaagtcc tcccaatctt tgttggcttc tggtaatcag 540
   aacaattact tggcttggca tcaacaaaaa ccaggtaaag ctccaaagat gttgattatt 600
   tgggcttcta ccagagtttc tggtgttcca tctagatttt ctggttctgg ttccggtact 660
   gattttactt tgaccatttc atccttgcaa ccagaagatt tcgctactta ctactgtcaa 720
   caatcttact ctgctccatt gacttttggt caaggtacaa aggtcgaaat caagagagaa 780
   ttcggtaagc ctatccctaa ccctctcctc ggtctcgatt ctacgggtgg tggtggatct 840
   ggtggtggtg gttctggtgg tggtggttct caggaactga caactatatg cgagcaaatc 900
   ccctcaccaa ctttagaatc gacgccgtac tctttgtcaa cgactactat tttggccaac 960
   gggaaggcaa tgcaaggagt ttttgaatat tacaaatcag taacgtttgt cagtaattgc 1020
   ggttctcacc cctcaacaac tagcaaaggc agccccataa acacacagta tgttttttga 1080
   gtttaaac 1088
<210> 56
   <211> 5597
   <212> DNA
   <213> Artificial Sequence
<220>
   <223> expression vector including polynucleotide encoding scFv of huAbF46 antibody
<220>
   <221> misc_difference
   <222> (573)..(578)
   <223> NheI restriction site
<220>
   <221> misc_difference
   <222> (588)..(938)
   <223> huAbF46 VH
<220>
   <221> misc_difference
   <222> (939)..(1007)
   <223> linker
<220>
   <221> misc_difference
   <222> (1008)..(1349)
   <223> huAbF46 VL
<220>
   <221> misc_difference
   <222> (1350)..(1355)
   <223> EcoRI restriction site
<220>
   <221> misc_difference
   <222> (1356)..(1397)
   <223> V5 epitope
<220>
   <221> misc_difference
   <222> (1398)..(1442)
   <223> (G4S)3 linker
<220>
   <221> misc_difference
   <222> (1443)..(1649)
   <223> Aga2
<220>
   <221> misc_difference
   <222> (1650)..(1652)
   <223> TGA(stop codon)
<220>
   <221> misc_difference
   <222> (1653)..(1660)
   <223> PmeI restriction site
<400> 56
   acggattaga agccgccgag cgggtgacag ccctccgaag gaagactctc ctccgtgcgt 60
   cctcgtcttc accggtcgcg ttcctgaaac gcagatgtgc ctcgcgccgc actgctccga 120
   acaataaaga ttctacaata ctagctttta tggttatgaa gaggaaaaat tggcagtaac 180
   ctggccccac aaaccttcaa atgaacgaat caaattaaca accataggat gataatgcga 240
   ttagtttttt agccttattt ctggggtaat taatcagcga agcgatgatt tttgatctat 300
   taacagatat ataaatgcaa aaactgcata accactttaa ctaatacttt caacattttc 360
   ggtttgtatt acttcttatt caaatgtaat aaaagtatca acaaaaaatt gttaatatac 420
   ctctatactt taacgtcaag gagaaaaaac cccggatcgg actactagca gctgtaatac 480
   gactcactat agggaatatt aagctaattc tacttcatac attttcaatt aagatgcagt 540
   tacttcgctg tttttcaata ttttctgtta ttgctagcgt tttagcagaa gttcaattgg 600
   ttgaatctgg tggtggtttg gttcaaccag gtggttcttt gagattgtct tgtgctgctt 660
   ctggttttac tttcaccgat tattacatgt cctgggttag acaagctcca ggtaaaggtt 720
   tggaatggtt gggtttcatt agaaacaagg ctaacggtta cactaccgaa tattctgctt 780
   ctgttaaggg tagattcacc atttctagag acaactctaa gaacaccttg tacttgcaaa 840
   tgaactcctt gagagctgaa gatactgctg tttattactg cgctagagat aattggtttg 900
   cttattgggg tcaaggtact ttggttactg tttcttctgg cctcgggggc ctcggaggag 960
   gaggtagtgg cggaggaggc tccggtggat ccagcggtgt gggttccgat attcaaatga 1020
   cccaatctcc atcttctttg tctgcttcag ttggtgatag agttaccatt acttgtaagt 1080
   cctcccaatc tttgttggct tctggtaatc agaacaatta cttggcttgg catcaacaaa 1140
   aaccaggtaa agctccaaag atgttgatta tttgggcttc taccagagtt tctggtgttc 1200
   catctagatt ttctggttct ggttccggta ctgattttac tttgaccatt tcatccttgc 1260
   aaccagaaga tttcgctact tactactgtc aacaatctta ctctgctcca ttgacttttg 1320
   gtcaaggtac aaaggtcgaa atcaagagag aattcggtaa gcctatccct aaccctctcc 1380
   tcggtctcga ttctacgggt ggtggtggat ctggtggtgg tggttctggt ggtggtggtt 1440
   ctcaggaact gacaactata tgcgagcaaa tcccctcacc aactttagaa tcgacgccgt 1500
   actctttgtc aacgactact attttggcca acgggaaggc aatgcaagga gtttttgaat 1560
   attacaaatc agtaacgttt gtcagtaatt gcggttctca cccctcaaca actagcaaag 1620
   gcagccccat aaacacacag tatgtttttt gagtttaaac ccgctgatct gataacaaca 1680
   gtgtagatgt aacaaaatcg actttgttcc cactgtactt ttagctcgta caaaatacaa 1740
   tatacttttc atttctccgt aaacaacatg ttttcccatg taatatcctt ttctattttt 1800
   cgttccgtta ccaactttac acatacttta tatagctatt cacttctata cactaaaaaa 1860
   ctaagacaat tttaattttg ctgcctgcca tatttcaatt tgttataaat tcctataatt 1920
   tatcctatta gtagctaaaa aaagatgaat gtgaatcgaa tcctaagaga attgggcaag 1980
   tgcacaaaca atacttaaat aaatactact cagtaataac ctatttctta gcatttttga 2040
   cgaaatttgc tattttgtta gagtctttta caccatttgt ctccacacct ccgcttacat 2100
   caacaccaat aacgccattt aatctaagcg catcaccaac attttctggc gtcagtccac 2160
   cagctaacat aaaatgtaag ctctcggggc tctcttgcct tccaacccag tcagaaatcg 2220
   agttccaatc caaaagttca cctgtcccac ctgcttctga atcaaacaag ggaataaacg 2280
   aatgaggttt ctgtgaagct gcactgagta gtatgttgca gtcttttgga aatacgagtc 2340
   ttttaataac tggcaaaccg aggaactctt ggtattcttg ccacgactca tctccgtgca 2400
   gttggacgat atcaatgccg taatcattga ccagagccaa aacatcctcc ttaggttgat 2460
   tacgaaacac gccaaccaag tatttcggag tgcctgaact atttttatat gcttttacaa 2520
   gacttgaaat tttccttgca ataaccgggt caattgttct ctttctattg ggcacacata 2580
   taatacccag caagtcagca tcggaatcta gagcacattc tgcggcctct gtgctctgca 2640
   agccgcaaac tttcaccaat ggaccagaac tacctgtgaa attaataaca gacatactcc 2700
   aagctgcctt tgtgtgctta atcacgtata ctcacgtgct caatagtcac caatgccctc 2760
   cctcttggcc ctctcctttt cttttttcga ccgaatttct tgaagacgaa agggcctcgt 2820
   gatacgccta tttttatagg ttaatgtcat gataataatg gtttcttagg acggatcgct 2880
   tgcctgtaac ttacacgcgc ctcgtatctt ttaatgatgg aataatttgg gaatttactc 2940
   tgtgtttatt tatttttatg ttttgtattt ggattttaga aagtaaataa agaaggtaga 3000
   agagttacgg aatgaagaaa aaaaaataaa caaaggttta aaaaatttca acaaaaagcg 3060
   tactttacat atatatttat tagacaagaa aagcagatta aatagatata cattcgatta 3120
   acgataagta aaatgtaaaa tcacaggatt ttcgtgtgtg gtcttctaca cagacaagat 3180
   gaaacaattc ggcattaata cctgagagca ggaagagcaa gataaaaggt agtatttgtt 3240
   ggcgatcccc ctagagtctt ttacatcttc ggaaaacaaa aactattttt tctttaattt 3300
   ctttttttac tttctatttt taatttatat atttatatta aaaaatttaa attataatta 3360
   tttttatagc acgtgatgaa aaggacccag gtggcacttt tcggggaaat gtgcgcggaa 3420
   cccctatttg tttatttttc taaatacatt caaatatgta tccgctcatg agacaataac 3480
   cctgataaat gcttcaataa tattgaaaaa ggaagagtat gagtattcaa catttccgtg 3540
   tcgcccttat tccctttttt gcggcatttt gccttcctgt ttttgctcac ccagaaacgc 3600
   tggtgaaagt aaaagatgct gaagatcagt tgggtgcacg agtgggttac atcgaactgg 3660
   atctcaacag cggtaagatc cttgagagtt ttcgccccga agaacgtttt ccaatgatga 3720
   gcacttttaa agttctgcta tgtggcgcgg tattatcccg tgttgacgcc gggcaagagc 3780
   aactcggtcg ccgcatacac tattctcaga atgacttggt tgagtactca ccagtcacag 3840
   aaaagcatct tacggatggc atgacagtaa gagaattatg cagtgctgcc ataaccatga 3900
   gtgataacac tgcggccaac ttacttctga caacgatcgg aggaccgaag gagctaaccg 3960
   cttttttgca caacatgggg gatcatgtaa ctcgccttga tcgttgggaa ccggagctga 4020
   atgaagccat accaaacgac gagcgtgaca ccacgatgcc tgtagcaatg gcaacaacgt 4080
   tgcgcaaact attaactggc gaactactta ctctagcttc ccggcaacaa ttaatagact 4140
   ggatggaggc ggataaagtt gcaggaccac ttctgcgctc ggcccttccg gctggctggt 4200
   ttattgctga taaatctgga gccggtgagc gtgggtctcg cggtatcatt gcagcactgg 4260
   ggccagatgg taagccctcc cgtatcgtag ttatctacac gacggggagt caggcaacta 4320
   tggatgaacg aaatagacag atcgctgaga taggtgcctc actgattaag cattggtaac 4380
   tgtcagacca agtttactca tatatacttt agattgattt aaaacttcat ttttaattta 4440
   aaaggatcta ggtgaagatc ctttttgata atctcatgac caaaatccct taacgtgagt 4500
   tttcgttcca ctgagcgtca gaccccgtag aaaagatcaa aggatcttct tgagatcctt 4560
   tttttctgcg cgtaatctgc tgcttgcaaa caaaaaaacc accgctacca gcggtggttt 4620
   gtttgccgga tcaagagcta ccaactcttt ttccgaaggt aactggcttc agcagagcgc 4680
   agataccaaa tactgtcctt ctagtgtagc cgtagttagg ccaccacttc aagaactctg 4740
   tagcaccgcc tacatacctc gctctgctaa tcctgttacc agtggctgct gccagtggcg 4800
   ataagtcgtg tcttaccggg ttggactcaa gacgatagtt accggataag gcgcagcggt 4860
   cgggctgaac ggggggttcg tgcacacagc ccagcttgga gcgaacgacc tacaccgaac 4920
   tgagatacct acagcgtgag cattgagaaa gcgccacgct tcccgaaggg agaaaggcgg 4980
   acaggtatcc ggtaagcggc agggtcggaa caggagagcg cacgagggag cttccagggg 5040
   ggaacgcctg gtatctttat agtcctgtcg ggtttcgcca cctctgactt gagcgtcgat 5100
   ttttgtgatg ctcgtcaggg gggccgagcc tatggaaaaa cgccagcaac gcggcctttt 5160
   tacggttcct ggccttttgc tggccttttg ctcacatgtt ctttcctgcg ttatcccctg 5220
   attctgtgga taaccgtatt accgcctttg agtgagctga taccgctcgc cgcagccgaa 5280
   cgaccgagcg cagcgagtca gtgagcgagg aagcggaaga gcgcccaata cgcaaaccgc 5340
   ctctccccgc gcgttggccg attcattaat gcagctggca cgacaggttt cccgactgga 5400
   aagcgggcag tgagcgcaac gcaattaatg tgagttacct cactcattag gcaccccagg 5460
   ctttacactt tatgcttccg gctcctatgt tgtgtggaat tgtgagcgga taacaatttc 5520
   acacaggaaa cagctatgac catgattacg ccaagctcgg aattaaccct cactaaaggg 5580
   aacaaaagct ggctagt 5597
<210> 57
   <211> 13
   <212> PRT
   <213> Artificial Sequence
<220>
   <223> U6-HC7 hinge
<400> 57
   Glu Pro Lys Ser Cys Asp Cys His Cys Pro Pro Cys Pro
   1 5 10
<210> 58
   <211> 435
   <212> DNA
   <213> Artificial Sequence
<220>
   <223> polynucleotide encoding CDR-L3 derived from L3-1 clone
<400> 58
   gaattcacta gtgattaatt cgccgccacc atggattcac aggcccaggt cctcatgttg 60
   ctgctgctat cggtatctgg tacctgtgga gatatccaga tgacccagtc cccgagctcc 120
   ctgtccgcct ctgtgggcga tagggtcacc atcacctgca agtccagtca gagtctttta 180
   gctagtggca accaaaataa ctacttggcc tggcaccaac agaaaccagg aaaagctccg 240
   aaaatgctga ttatttgggc atccactagg gtatctggag tcccttctcg cttctctgga 300
   tccgggtctg ggacggattt cactctgacc atcagcagtc tgcagccgga agacttcgca 360
   acttattact gtcagcagtc ctacagccgc ccgtacacgt tcggacaggg taccaaggtg 420
   gagatcaaac gtacg 435
<210> 59
   <211> 435
   <212> DNA
   <213> Artificial Sequence
<220>
   <223> polynucleotide encoding CDR-L3 derived from L3-2 clone
<400> 59
   gaattcacta gtgattaatt cgccgccacc atggattcac aggcccaggt cctcatgttg 60
   ctgctgctat cggtatctgg tacctgtgga gatatccaga tgacccagtc cccgagctcc 120
   ctgtccgcct ctgtgggcga tagggtcacc atcacctgca agtccagtca gagtctttta 180
   gctagtggca accaaaataa ctacttggcc tggcaccaac agaaaccagg aaaagctccg 240
   aaaatgctga ttatttgggc atccactagg gtatctggag tcccttctcg cttctctgga 300
   tccgggtctg ggacggattt cactctgacc atcagcagtc tgcagccgga agacttcgca 360
   acttattact gtgggcagtc ctacagccgt ccgctcacgt tcggacaggg taccaaggtg 420
   gagatcaaac gtacg 435
<210> 60
   <211> 435
   <212> DNA
   <213> Artificial Sequence
<220>
   <223> polynucleotide encoding CDR-L3 derived from L3-3 clone
<400> 60
   gaattcacta gtgattaatt cgccgccacc atggattcac aggcccaggt cctcatgttg 60
   ctgctgctat cggtatctgg tacctgtgga gatatccaga tgacccagtc cccgagctcc 120
   ctgtccgcct ctgtgggcga tagggtcacc atcacctgca agtccagtca gagtctttta 180
   gctagtggca accaaaataa ctacttggcc tggcaccaac agaaaccagg aaaagctccg 240
   aaaatgctga ttatttgggc atccactagg gtatctggag tcccttctcg cttctctgga 300
   tccgggtctg ggacggattt cactctgacc atcagcagtc tgcagccgga agacttcgca 360
   acttattact gtgcacagtc ctacagccat ccgttctctt tcggacaggg taccaaggtg 420
   gagatcaaac gtacg 435
<210> 61
   <211> 435
   <212> DNA
   <213> Artificial Sequence
<220>
   <223> polynucleotide encoding CDR-L3 derived from L3-5 clone
<400> 61
   gaattcacta gtgattaatt cgccgccacc atggattcac aggcccaggt cctcatgttg      60
   ctgctgctat cggtatctgg tacctgtgga gatatccaga tgacccagtc cccgagctcc     120
   ctgtccgcct ctgtgggcga tagggtcacc atcacctgca agtccagtca gagtctttta     180
   gctagtggca accaaaataa ctacttggcc tggcaccaac agaaaccagg aaaagctccg     240
   aaaatgctga ttatttgggc atccactagg gtatctggag tcccttctcg cttctctgga     300
   tccgggtctg ggacggattt cactctgacc atcagcagtc tgcagccgga agacttcgca     360
   acttattact gtcagcagtc ctacagccgc ccgtttacgt tcggacaggg taccaaggtg     420
   gagatcaaac gtacg                                                      435
<210> 62
   <211> 462
   <212> PRT
   <213> Artificial Sequence
<220>
   <223> polypeptide consisting of heavy chain of huAbF46-H4-A1, U6-HC7 hinge and constant region of human IgG1
<400> 62
   Met Glu Trp Ser Trp Val Phe Leu Val Thr Leu Leu Asn Gly Ile Gln
     1               5                  10                  15
   Cys Glu Val Gln Leu Val Glu Ser Gly Gly Gly Leu Val Gln Pro Gly
                20                  25                  30
   Gly Ser Leu Arg Leu Ser Cys Ala Ala Ser Gly Phe Thr Phe Thr Asp
            35                  40                  45
   Tyr Tyr Met Ser Trp Val Arg Gln Ala Pro Gly Lys Gly Leu Glu Trp
        50                  55                  60
   Leu Gly Phe Ile Arg Asn Lys Ala Asn Gly Tyr Thr Thr Glu Tyr Ser
    65                  70                  75                  80
   Ala Ser Val Lys Gly Arg Phe Thr Ile Ser Arg Asp Asn Ser Lys Asn
                    85                  90                  95
   Thr Leu Tyr Leu Gln Met Asn Ser Leu Arg Ala Glu Asp Thr Ala Val
               100                 105                 110
   Tyr Tyr Cys Ala Arg Asp Asn Trp Phe Ala Tyr Trp Gly Gln Gly Thr
           115                 120                 125
   Leu Val Thr Val Ser Ser Ala Ser Thr Lys Gly Pro Ser Val Phe Pro
       130                 135                 140
   Leu Ala Pro Ser Ser Lys Ser Thr Ser Gly Gly Thr Ala Ala Leu Gly
   145                 150                 155                 160
   Cys Leu Val Lys Asp Tyr Phe Pro Glu Pro Val Thr Val Ser Trp Asn
                   165                 170                 175
   Ser Gly Ala Leu Thr Ser Gly Val His Thr Phe Pro Ala Val Leu Gln
               180                 185                 190
   Ser Ser Gly Leu Tyr Ser Leu Ser Ser Val Val Thr Val Pro Ser Ser
           195                 200                 205
   Ser Leu Gly Thr Gln Thr Tyr Ile Cys Asn Val Asn His Lys Pro Ser
       210                 215                 220
   Asn Thr Lys Val Asp Lys Lys Val Glu Pro Lys Ser Cys Asp Cys His
   225                 230                 235                 240
   Cys Pro Pro Cys Pro Ala Pro Glu Leu Leu Gly Gly Pro Ser Val Phe
                   245                 250                 255
   Leu Phe Pro Pro Lys Pro Lys Asp Thr Leu Met Ile Ser Arg Thr Pro
               260                 265                 270
   Glu Val Thr Cys Val Val Val Asp Val Ser His Glu Asp Pro Glu Val
           275                 280                 285
   Lys Phe Asn Trp Tyr Val Asp Gly Val Glu Val His Asn Ala Lys Thr
       290                 295                 300
   Lys Pro Arg Glu Glu Gln Tyr Asn Ser Thr Tyr Arg Val Val Ser Val
   305                 310                 315                 320
   Leu Thr Val Leu His Gln Asp Trp Leu Asn Gly Lys Glu Tyr Lys Cys
                   325                 330                 335
   Lys Val Ser Asn Lys Ala Leu Pro Ala Pro Ile Glu Lys Thr Ile Ser
               340                 345                 350
   Lys Ala Lys Gly Gln Pro Arg Glu Pro Gln Val Tyr Thr Leu Pro Pro
           355                 360                 365
   Ser Arg Glu Glu Met Thr Lys Asn Gln Val Ser Leu Thr Cys Leu Val
       370                 375                 380
   Lys Gly Phe Tyr Pro Ser Asp Ile Ala Val Glu Trp Glu Ser Asn Gly
   385                 390                 395                 400
   Gln Pro Glu Asn Asn Tyr Lys Thr Thr Pro Pro Val Leu Asp Ser Asp
                   405                 410                 415
   Gly Ser Phe Phe Leu Tyr Ser Lys Leu Thr Val Asp Lys Ser Arg Trp
               420                 425                 430
   Gln Gln Gly Asn Val Phe Ser Cys Ser Val Met His Glu Ala Leu His
           435                 440                 445
   Asn His Tyr Thr Gln Lys Ser Leu Ser Leu Ser Pro Gly Lys
       450                 455                 460
<210> 63
   <211> 1410
   <212> DNA
   <213> Artificial Sequence
<220>
   <223> polynucleotide encoding polypeptide consisting of heavy chain of huAbF46-H4-A1, U6-HC7 hinge and constant region of human IgG1
<400> 63
   gaattcgccg ccaccatgga atggagctgg gtttttctcg taacactttt aaatggtatc        60
   cagtgtgagg ttcagctggt ggagtctggc ggtggcctgg tgcagccagg gggctcactc       120
   cgtttgtcct gtgcagcttc tggcttcacc ttcactgatt actacatgag ctgggtgcgt       180
   caggccccgg gtaagggcct ggaatggttg ggttttatta gaaacaaagc taatggttac       240
   acaacagagt acagtgcatc tgtgaagggt cgtttcacta taagcagaga taattccaaa       300
   aacacactgt acctgcagat gaacagcctg cgtgctgagg acactgccgt ctattattgt        360
   gctagagata actggtttgc ttactggggc caagggactc tggtcaccgt ctcctcggct        420
   agcaccaagg gcccatcggt cttccccctg gcaccctcct ccaagagcac ctctgggggc        480
   acagcggccc tgggctgcct ggtcaaggac tacttccccg aaccggtgac ggtgtcgtgg        540
   aactcaggcg ccctgaccag cggcgtgcac accttcccgg ctgtcctaca gtcctcagga        600
   ctctactccc tcagcagcgt ggtgaccgtg ccctccagca gcttgggcac ccagacctac        660
   atctgcaacg tgaatcacaa gcccagcaac accaaggtgg acaagaaagt tgagcccaaa        720
   agctgcgatt gccactgtcc tccatgtcca gcacctgaac tcctgggggg accgtcagtc        780
   ttcctcttcc ccccaaaacc caaggacacc ctcatgatct cccggacccc tgaggtcaca        840
   tgcgtggtgg tggacgtgag ccacgaagac cctgaggtca agttcaactg gtacgtggac        900
   ggcgtggagg tgcataatgc caagacaaag ccgcgggagg agcagtacaa cagcacgtac        960
   cgtgtggtca gcgtcctcac cgtcctgcac caggactggc tgaatggcaa ggagtacaag       1020
   tgcaaggtct ccaacaaagc cctcccagcc cccatcgaga aaaccatctc caaagccaaa       1080
   gggcagcccc gagaaccaca ggtgtacacc ctgcccccat cccgggagga gatgaccaag       1140
   aaccaggtca gcctgacctg cctggtcaaa ggcttctatc ccagcgacat cgccgtggag       1200
   tgggagagca atgggcagcc ggagaacaac tacaagacca cgcctcccgt gctggactcc       1260
   gacggctcct tcttcctcta cagcaagctc accgtggaca agagcaggtg gcagcagggg       1320
   aacgtcttct catgctccgt gatgcatgag gctctgcaca accactacac gcagaagagc       1380
   ctctccctgt ctccgggtaa atgactcgag                                        1410
<210> 64
   <211> 461
   <212> PRT
   <213> Artificial Sequence
<220>
   <223> polypeptide consisting of heavy chain of huAbF46-H4-A1, human IgG2 hinge and constant region of human IgG1
<400> 64
   Met Glu Trp Ser Trp Val Phe Leu Val Thr Leu Leu Asn Gly Ile Gln
   1               5                   10                  15
   Cys Glu Val Gln Leu Val Glu Ser Gly Gly Gly Leu Val Gln Pro Gly
               20                  25                  30
   Gly Ser Leu Arg Leu Ser Cys Ala Ala Ser Gly Phe Thr Phe Thr Asp
           35                  40                  45
   Tyr Tyr Met Ser Trp Val Arg Gln Ala Pro Gly Lys Gly Leu Glu Trp
       50                  55                  60
   Leu Gly Phe Ile Arg Asn Lys Ala Asn Gly Tyr Thr Thr Glu Tyr Ser
    65                  70                  75                  80
   Ala Ser Val Lys Gly Arg Phe Thr Ile Ser Arg Asp Asn Ser Lys Asn
                    85                  90                  95
   Thr Leu Tyr Leu Gln Met Asn Ser Leu Arg Ala Glu Asp Thr Ala Val
               100                 105                 110
   Tyr Tyr Cys Ala Arg Asp Asn Trp Phe Ala Tyr Trp Gly Gln Gly Thr
           115                 120                 125
   Leu Val Thr Val Ser Ser Ala Ser Thr Lys Gly Pro Ser Val Phe Pro
       130                 135                 140
   Leu Ala Pro Ser Ser Lys Ser Thr Ser Gly Gly Thr Ala Ala Leu Gly
   145                 150                 155                 160
   Cys Leu Val Lys Asp Tyr Phe Pro Glu Pro Val Thr Val Ser Trp Asn
                   165                 170                 175
   Ser Gly Ala Leu Thr Ser Gly Val His Thr Phe Pro Ala Val Leu Gln
               180                 185                 190
   Ser Ser Gly Leu Tyr Ser Leu Ser Ser Val Val Thr Val Pro Ser Ser
           195                 200                 205
   Ser Leu Gly Thr Gln Thr Tyr Ile Cys Asn Val Asn His Lys Pro Ser
       210                 215                 220
   Asn Thr Lys Val Asp Lys Lys Val Glu Arg Lys Cys Cys Val Glu Cys
   225                 230                 235                 240
   Pro Pro Cys Pro Ala Pro Glu Leu Leu Gly Gly Pro Ser Val Phe Leu
                   245                 250                 255
   Phe Pro Pro Lys Pro Lys Asp Thr Leu Met Ile Ser Arg Thr Pro Glu
               260                 265                 270
   Val Thr Cys Val Val Val Asp Val Ser His Glu Asp Pro Glu Val Lys
           275                 280                 285
   Phe Asn Trp Tyr Val Asp Gly Val Glu Val His Asn Ala Lys Thr Lys
       290                 295                 300
   Pro Arg Glu Glu Gln Tyr Asn Ser Thr Tyr Arg Val Val Ser Val Leu
   305                 310                 315                 320
   Thr Val Leu His Gln Asp Trp Leu Asn Gly Lys Glu Tyr Lys Cys Lys
                   325                 330                 335
   Val Ser Asn Lys Ala Leu Pro Ala Pro Ile Glu Lys Thr Ile Ser Lys
               340                 345                 350
   Ala Lys Gly Gln Pro Arg Glu Pro Gln Val Tyr Thr Leu Pro Pro Ser
           355                 360                 365
   Arg Glu Glu Met Thr Lys Asn Gln Val Ser Leu Thr Cys Leu Val Lys
       370                 375                 380
   Gly Phe Tyr Pro Ser Asp Ile Ala Val Glu Trp Glu Ser Asn Gly Gln
   385                 390                 395                 400
   Pro Glu Asn Asn Tyr Lys Thr Thr Pro Pro Val Leu Asp Ser Asp Gly
                   405                 410                 415
   Ser Phe Phe Leu Tyr Ser Lys Leu Thr Val Asp Lys Ser Arg Trp Gln
               420                 425                 430
   Gln Gly Asn Val Phe Ser Cys Ser Val Met His Glu Ala Leu His Asn
           435                 440                 445
   His Tyr Thr Gln Lys Ser Leu Ser Leu Ser Pro Gly Lys
       450                 455                 460
<210> 65
   <211> 1407
   <212> DNA
   <213> Artificial Sequence
<220>
   <223> polynucleotide encoding polypeptide consisting of heavy chain of huAbF46-H4-A1, human IgG2 hinge and constant region of human IgG1
<400> 65
   gaattcgccg ccaccatgga atggagctgg gtttttctcg taacactttt aaatggtatc      60
   cagtgtgagg ttcagctggt ggagtctggc ggtggcctgg tgcagccagg gggctcactc     120
   cgtttgtcct gtgcagcttc tggcttcacc ttcactgatt actacatgag ctgggtgcgt     180
   caggccccgg gtaagggcct ggaatggttg ggttttatta gaaacaaagc taatggttac     240
   acaacagagt acagtgcatc tgtgaagggt cgtttcacta taagcagaga taattccaaa     300
   aacacactgt acctgcagat gaacagcctg cgtgctgagg acactgccgt ctattattgt     360
   gctagagata actggtttgc ttactggggc caagggactc tggtcaccgt ctcctcggct     420
   agcaccaagg gcccatcggt cttccccctg gcaccctcct ccaagagcac ctctgggggc     480
   acagcggccc tgggctgcct ggtcaaggac tacttccccg aaccggtgac ggtgtcgtgg     540
   aactcaggcg ccctgaccag cggcgtgcac accttcccgg ctgtcctaca gtcctcagga     600
   ctctactccc tcagcagcgt ggtgaccgtg ccctccagca gcttgggcac ccagacctac     660
   atctgcaacg tgaatcacaa gcccagcaac accaaggtgg acaagaaagt tgagaggaag     720
   tgctgtgtgg agtgcccccc ctgcccagca cctgaactcc tggggggacc gtcagtcttc     780
   ctcttccccc caaaacccaa ggacaccctc atgatctccc ggacccctga ggtcacatgc     840
   gtggtggtgg acgtgagcca cgaagaccct gaggtcaagt tcaactggta cgtggacggc     900
   gtggaggtgc ataatgccaa gacaaagccg cgggaggagc agtacaacag cacgtaccgt     960
   gtggtcagcg tcctcaccgt cctgcaccag gactggctga atggcaagga gtacaagtgc    1020
   aaggtctcca acaaagccct cccagccccc atcgagaaaa ccatctccaa agccaaaggg    1080
   cagccccgag aaccacaggt gtacaccctg cccccatccc gggaggagat gaccaagaac    1140
   caggtcagcc tgacctgcct ggtcaaaggc ttctatccca gcgacatcgc cgtggagtgg 1200
   gagagcaatg ggcagccgga gaacaactac aagaccacgc ctcccgtgct ggactccgac 1260
   ggctccttct tcctctacag caagctcacc gtggacaaga gcaggtggca gcaggggaac 1320
   gtcttctcat gctccgtgat gcatgaggct ctgcacaacc actacacgca gaagagcctc 1380
   tccctgtctc cgggtaaatg actcgag 1407
<210> 66
   <211> 460
   <212> PRT
   <213> Artificial Sequence
<220>
   <223> polypeptide consisting of heavy chain of huAbF46-H4-A1, human IgG2 hinge and constant region of human IgG2
<400> 66
   Met Glu Trp Ser Trp Val Phe Leu Val Thr Leu Leu Asn Gly Ile Gln
     1               5                  10                  15
   Cys Glu Val Gln Leu Val Glu Ser Gly Gly Gly Leu Val Gln Pro Gly
                20                  25                  30
   Gly Ser Leu Arg Leu Ser Cys Ala Ala Ser Gly Phe Thr Phe Thr Asp
            35                  40                  45
   Tyr Tyr Met Ser Trp Val Arg Gln Ala Pro Gly Lys Gly Leu Glu Trp
        50                  55                  60
   Leu Gly Phe Ile Arg Asn Lys Ala Asn Gly Tyr Thr Thr Glu Tyr Ser
    65                  70                  75                  80
   Ala Ser Val Lys Gly Arg Phe Thr Ile Ser Arg Asp Asn Ser Lys Asn
                    85                  90                  95
   Thr Leu Tyr Leu Gln Met Asn Ser Leu Arg Ala Glu Asp Thr Ala Val
               100                 105                 110
   Tyr Tyr Cys Ala Arg Asp Asn Trp Phe Ala Tyr Trp Gly Gln Gly Thr
           115                 120                 125
   Leu Val Thr Val Ser Ser Ala Ser Thr Lys Gly Pro Ser Val Phe Pro
       130                 135                 140
   Leu Ala Pro Cys Ser Arg Ser Thr Ser Glu Ser Thr Ala Ala Leu Gly
   145                 150                 155                 160
   Cys Leu Val Lys Asp Tyr Phe Pro Glu Pro Val Thr Val Ser Trp Asn
                   165                 170                 175
   Ser Gly Ala Leu Thr Ser Gly Val His Thr Phe Pro Ala Val Leu Gln
               180                 185                 190
   Ser Ser Gly Leu Tyr Ser Leu Ser Ser Val Val Thr Val Pro Ser Ser
           195                 200                 205
   Asn Phe Gly Thr Gln Thr Tyr Thr Cys Asn Val Asp His Lys Pro Ser
       210                 215                 220
   Asn Thr Lys Val Asp Lys Thr Val Glu Arg Lys Cys Cys Val Glu Cys
   225                 230                 235                 240
   Pro Pro Cys Pro Ala Pro Pro Val Ala Gly Pro Ser Val Phe Leu Phe
                   245                 250                 255
   Pro Pro Lys Pro Lys Asp Thr Leu Met Ile Ser Arg Thr Pro Glu Val
               260                 265                 270
   Thr Cys Val Val Val Asp Val Ser His Glu Asp Pro Glu Val Gln Phe
           275                 280                 285
   Asn Trp Tyr Val Asp Gly Val Glu Val His Asn Ala Lys Thr Lys Pro
       290                 295                 300
   Arg Glu Glu Gln Phe Asn Ser Thr Phe Arg Val Val Ser Val Leu Thr
   305                 310                 315                 320
   Val Val His Gln Asp Trp Leu Asn Gly Lys Glu Tyr Lys Cys Lys Val
                   325                 330                 335
   Ser Asn Lys Gly Leu Pro Ala Pro Ile Glu Lys Thr Ile Ser Lys Thr
               340                 345                 350
   Lys Gly Gln Pro Arg Glu Pro Gln Val Tyr Thr Leu Pro Pro Ser Arg
           355                 360                 365
   Glu Glu Met Thr Lys Asn Gln Val Ser Leu Thr Cys Leu Val Lys Gly
       370                 375                 380
   Phe Tyr Pro Ser Asp Ile Ala Val Glu Trp Glu Ser Asn Gly Gln Pro
   385                 390                 395                 400
   Glu Asn Asn Tyr Lys Thr Thr Pro Pro Met Leu Asp Ser Asp Gly Ser
                   405                 410                 415
   Phe Phe Leu Tyr Ser Lys Leu Thr Val Asp Lys Ser Arg Trp Gln Gln
               420                 425                 430
   Gly Asn Val Phe Ser Cys Ser Val Met His Glu Ala Leu His Asn His
           435                 440                 445
   Tyr Thr Gln Lys Ser Leu Ser Leu Ser Pro Gly Lys
       450                 455                 460
<210> 67
   <211> 1404
   <212> DNA
   <213> Artificial Sequence
<220>
   <223> polynucleotide encoding polypeptide consisting of heavy chain of huAbF46-H4-A1, human IgG2 hinge and constant region of human IgG2
<400> 67
   gaattcgccg ccaccatgga atggagctgg gtttttctcg taacactttt aaatggtatc      60
   cagtgtgagg ttcagctggt ggagtctggc ggtggcctgg tgcagccagg gggctcactc     120
   cgtttgtcct gtgcagcttc tggcttcacc ttcactgatt actacatgag ctgggtgcgt     180
   caggccccgg gtaagggcct ggaatggttg ggttttatta gaaacaaagc taatggttac     240
   acaacagagt acagtgcatc tgtgaagggt cgtttcacta taagcagaga taattccaaa     300
   aacacactgt acctgcagat gaacagcctg cgtgctgagg acactgccgt ctattattgt     360
   gctagagata actggtttgc ttactggggc caagggactc tggtcaccgt ctcctcggct     420
   agcaccaagg gcccatcggt cttccccctg gcgccctgct ccaggagcac ctccgagagc     480
   acagcggccc tgggctgcct ggtcaaggac tacttccccg aaccggtgac ggtgtcgtgg     540
   aactcaggcg ctctgaccag cggcgtgcac accttcccag ctgtcctaca gtcctcagga     600
   ctctactccc tcagcagcgt ggtgaccgtg ccctccagca acttcggcac ccagacctac     660
   acctgcaacg tagatcacaa gcccagcaac accaaggtgg acaagacagt tgagcgcaaa     720
   tgttgtgtcg agtgcccacc gtgcccagca ccacctgtgg caggaccgtc agtcttcctc     780
   ttccccccaa aacccaagga caccctcatg atctcccgga cccctgaggt cacgtgcgtg     840
   gtggtggacg tgagccacga agaccccgag gtccagttca actggtacgt ggacggcgtg     900
   gaggtgcata atgccaagac aaagccacgg gaggagcagt tcaacagcac gttccgtgtg     960
   gtcagcgtcc tcaccgttgt gcaccaggac tggctgaacg gcaaggagta caagtgcaag    1020
   gtctccaaca aaggcctccc agcccccatc gagaaaacca tctccaaaac caaagggcag    1080
   ccccgagaac cacaggtgta caccctgccc ccatcccggg aggagatgac caagaaccag    1140
   gtcagcctga cctgcctggt caaaggcttc taccccagcg acatcgccgt ggagtgggag    1200
   agcaatgggc agccggagaa caactacaag accacgcctc ccatgctgga ctccgacggc    1260
   tccttcttcc tctacagcaa gctcaccgtg gacaagagca ggtggcagca ggggaacgtc    1320
   ttctcatgct ccgtgatgca tgaggctctg cacaaccact acacgcagaa gagcctctcc    1380
   ctgtctccgg gtaaatgact cgag                                           1404
<210> 68
   <211> 240
   <212> PRT
   <213> Artificial Sequence
<220>
   <223> polypeptide consisting of light chain of huAbF46-H4-A1(H36Y) and human kappa constant region
<400> 68
<210> 69
   <211> 758
   <212> DNA
   <213> Artificial Sequence
<220>
   <223> polynucleotide encoding polypeptide consisting of light chain of huAbF46-H4-A1(H36Y) and human kappa constant region
<400> 69
   aattcactag tgattaattc gccgccacca tggattcaca ggcccaggtc ctcatgttgc 60
   tgctgctatc ggtatctggt acctgtggag atatccagat gacccagtcc ccgagctccc 120
   tgtccgcctc tgtgggcgat agggtcacca tcacctgcaa gtccagtcag agtcttttag 180
   ctagtggcaa ccaaaataac tacttggcct ggtaccaaca gaaaccagga aaagctccga 240
   aaatgctgat tatttgggca tccactaggg tatctggagt cccttctcgc ttctctggat 300
   ccgggtctgg gacggatttc actctgacca tcagcagtct gcagccggaa gacttcgcaa 360
   cttattactg tcagcagtcc tacagccgcc cgtacacgtt cggacagggt accaaggtgg 420
   agatcaaacg tacggtggct gcaccatctg tcttcatctt cccgccatct gatgagcagt 480
   tgaaatctgg aactgcctct gttgtgtgcc tgctgaataa cttctatccc agagaggcca 540
   aagtacagtg gaaggtggat aacgccctcc aatcgggtaa ctcccaggag agtgtcacag 600
   agcaggacag caaggacagc acctacagcc tcagcagcac cctgacgctg agcaaagcag 660
   actacgagaa acacaaagtc tacgcctgcg aagtcaccca tcagggcctg agctcgcccg 720
   tcacaaagag cttcaacagg ggagagtgtt gactcgag 758
<210> 70
   <211> 240
   <212> PRT
   <213> Artificial Sequence
<220>
   <223> polypeptide consisting of light chain of huAbF46-H4-A1 and human kappa constant region
<400> 70
   Met Asp Ser Gln Ala Gln Val Leu Met Leu Leu Leu Leu Ser Val Ser
   1               5                   10                  15
   Gly Thr Cys Gly Asp Ile Gln Met Thr Gln Ser Pro Ser Ser Leu Ser
               20                  25                  30
   Ala Ser Val Gly Asp Arg Val Thr Ile Thr Cys Lys Ser Ser Gln Ser
           35                  40                  45
   Leu Leu Ala Ser Gly Asn Gln Asn Asn His Leu Ala Trp Tyr Gln Gln
       50                  55                  60
   Lys Pro Gly Lys Ala Pro Lys Met Leu Ile Ile Trp Ala Ser Thr Arg
   65                  70                  75                  80
   Val Ser Gly Val Pro Ser Arg Phe Ser Gly Ser Gly Ser Gly Thr Asp
                   85                  90                  95
   Phe Thr Leu Thr Ile Ser Ser Leu Gln Pro Glu Asp Phe Ala Thr Tyr
               100                 105                 110
   Tyr Cys Gln Gln Ser Tyr Ser Arg Pro Tyr Thr Phe Gly Gln Gly Thr
           115                 120                 125
   Lys Val Glu Ile Lys Arg Thr Val Ala Ala Pro Ser Val Phe Ile Phe
       130                 135                 140
   Pro Pro Ser Asp Glu Gln Leu Lys Ser Gly Thr Ala Ser Val Val Cys
   145                 150                 155                 160
   Leu Leu Asn Asn Phe Tyr Pro Arg Glu Ala Lys Val Gln Trp Lys Val
                   165                 170                 175
   Asp Asn Ala Leu Gln Ser Gly Asn Ser Gln Glu Ser Val Thr Glu Gln
               180                 185                 190
   Asp Ser Lys Asp Ser Thr Tyr Ser Leu Ser Ser Thr Leu Thr Leu Ser
           195                 200                 205
   Lys Ala Asp Tyr Glu Lys His Lys Val Tyr Ala Cys Glu Val Thr His
       210                 215                 220
   Gln Gly Leu Ser Ser Pro Val Thr Lys Ser Phe Asn Arg Gly Glu Cys
   225                 230                 235                 240
<210> 71
   <211> 19
   <212> PRT
   <213> Artificial Sequence
<220>
   <223> epitope in SEMA domain of c-Met
<400> 71
   Phe Ser Pro Gln Ile Glu Glu Pro Ser Gln Cys Pro Asp Cys Val Val
   1               5                   10                  15
   Ser Ala Leu
<210> 72
   <211> 10
   <212> PRT
   <213> Artificial Sequence
<220>
   <223> epitope in SEMA domain of c-Met
<400> 72
   Pro Gln Ile Glu Glu Pro Ser Gln Cys Pro
   1 5 10
<210> 73
   <211> 5
   <212> PRT
   <213> Artificial Sequence
<220>
   <223> epitope in SEMA domain of c-Met
<400> 73
   Glu Glu Pro Ser Gln
   1 5
<210> 74
   <211> 117
   <212> PRT
   <213> Artificial Sequence
<220>
   <223> heavy chain variable region of anti-c-Met antibody (AbF46 or huAbF46-H1)
<400> 74
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Thr Asp Tyr
   20 25 30
   Tyr Met Ser Trp Val Arg Gln Ala Pro Gly Lys Gly Leu Glu Trp Leu
   35 40 45
   Gly Phe Ile Arg Asn Lys Ala Asn Gly Tyr Thr Thr Glu Tyr Ser Ala
   50 55 60
   Ser Val Lys Gly Arg Phe Thr Ile Ser Arg Asp Asn Ser Lys Asn Ser
   65 70 75 80
   Leu Tyr Leu Gln Met Asn Ser Leu Lys Thr Glu Asp Thr Ala Val Tyr
   85 90 95
   Tyr Cys Ala Arg Asp Asn Trp Phe Ala Tyr Trp Gly Gln Gly Thr Leu
   100 105 110
   Val Thr Val Ser Ser
   115
<210> 75
   <211> 114
   <212> PRT
   <213> Artificial Sequence
<220>
   <223> light chain variable region of anti-c-Met antibody (AbF46 or huAbF46-H1)
<400> 75
   Asp Ile Val Met Thr Gln Ser Pro Asp Ser Leu Ala Val Ser Leu Gly
   1               5                   10                  15
   Glu Arg Ala Thr Ile Asn Cys Lys Ser Ser Gln Ser Leu Leu Ala Ser
               20                  25                  30
   Gly Asn Gln Asn Asn Tyr Leu Ala Trp His Gln Gln Lys Pro Gly Gln
           35                  40                  45
   Pro Pro Lys Met Leu Ile Ile Trp Ala Ser Thr Arg Val Ser Gly Val
       50                  55                  60
   Pro Asp Arg Phe Ser Gly Ser Gly Ser Gly Thr Asp Phe Thr Leu Thr
   65                  70                  75                  80
   Ile Ser Ser Leu Gln Ala Glu Asp Val Ala Val Tyr Tyr Cys Gln Gln
                   85                  90                  95
   Ser Tyr Ser Ala Pro Leu Thr Phe Gly Gly Gly Thr Lys Val Glu Ile
               100                 105                 110
   Lys Arg
<210> 76
   <211> 1416
   <212> DNA
   <213> Artificial Sequence
<220>
   <223> nucleotide sequence of heavy chain of nti-c-Met antibody (AbF46 or huAbF46-H1)
<220>
   <221> misc_feature
   <222> (1)..(6)
   <223> EcoRI restriction site
<220>
   <221> misc_feature
   <222> (7)..(66)
   <223> signal sequence
<220>
   <221> misc_feature
   <222> (67)..(417)
   <223> VH - heavy chain variable region
<220>
   <221> misc_feature
   <222> (418)..(423)
   <223> NdeI restriction site
<220>
   <221> misc_feature
   <222> (418)..(1407)
   <223> CH - heavy chain constant region
<220>
   <221> misc_feature
   <222> (1408)..(1410)
   <223> TGA - stop sodon
<220>
   <221> misc_feature
   <222> (1411)..(1416)
   <223> Xhol restriction site
<400> 76
   gaattcgccg ccaccatgga atggagctgg gtttttctcg taacactttt aaatggtatc 60
   cagtgtgagg tgaagctggt ggagtctgga ggaggcttgg tacagcctgg gggttctctg 120
   agactctcct gtgcaacttc tgggttcacc ttcactgatt actacatgag ctgggtccgc 180
   cagcctccag gaaaggcact tgagtggttg ggttttatta gaaacaaagc taatggttac 240
   acaacagagt acagtgcatc tgtgaagggt cggttcacca tctccagaga taattcccaa 300
   agcatcctct atcttcaaat ggacaccctg agagctgagg acagtgccac ttattactgt 360
   gcaagagata actggtttgc ttactggggc caagggactc tggtcactgt ctctgcagct 420
   agcaccaagg gcccatcggt cttccccctg gcaccctcct ccaagagcac ctctgggggc 480
   acagcggccc tgggctgcct ggtcaaggac tacttccccg aaccggtgac ggtgtcgtgg 540
   aactcaggcg ccctgaccag cggcgtgcac accttcccgg ctgtcctaca gtcctcagga 600
   ctctactccc tcagcagcgt ggtgaccgtg ccctccagca gcttgggcac ccagacctac 660
   atctgcaacg tgaatcacaa gcccagcaac accaaggtgg acaagaaagt tgagcccaaa 720
   tcttgtgaca aaactcacac atgcccaccg tgcccagcac ctgaactcct ggggggaccg 780
   tcagtcttcc tcttcccccc aaaacccaag gacaccctca tgatctcccg gacccctgag 840
   gtcacatgcg tggtggtgga cgtgagccac gaagaccctg aggtcaagtt caactggtac 900
   gtggacggcg tggaggtgca taatgccaag acaaagccgc gggaggagca gtacaacagc 960
   acgtaccgtg tggtcagcgt cctcaccgtc ctgcaccagg actggctgaa tggcaaggag 1020
   tacaagtgca aggtctccaa caaagccctc ccagccccca tcgagaaaac catctccaaa 1080
   gccaaagggc agccccgaga accacaggtg tacaccctgc ccccatcccg ggaggagatg 1140
   accaagaacc aggtcagcct gacctgcctg gtcaaaggct tctatcccag cgacatcgcc 1200
   gtggagtggg agagcaatgg gcagccggag aacaactaca agaccacgcc tcccgtgctg 1260
   gactccgacg gctccttctt cctctacagc aagctcaccg tggacaagag caggtggcag 1320
   caggggaacg tcttctcatg ctccgtgatg catgaggctc tgcacaacca ctacacgcag 1380
   aagagcctct ccctgtctcc gggtaaatga ctcgag 1416
<210> 77
   <211> 759
   <212> DNA
   <213> Artificial Sequence
<220>
   <223> nucleotide sequence of light chain of anti-c-Met antibody (AbF46 or huAbF46-H1)
<220>
   <221> misc_difference
   <222> (1)..(6)
   <223> EcoRI restriction site
<220>
   <221> misc_difference
   <222> (7)..(90)
   <223> signal sequence
<220>
   <221> misc_difference
   <222> (91)..(432)
   <223> VL - light chain variable region
<220>
   <221> misc_difference
   <222> (430)..(435)
   <223> BsiWI restriction site
<220>
   <221> misc_difference
   <222> (433)..(750)
   <223> CL - light chain constant region
<220>
   <221> misc_difference
   <222> (751)..(753)
   <223> stop codon
<220>
   <221> misc_difference
   <222> (754)..(759)
   <223> Xhol restriction site
<400> 77
   gaattcacta gtgattaatt cgccgccacc atggattcac aggcccaggt cctcatgttg      60
   ctgctgctat cggtatctgg tacctgtgga gacattttga tgacccagtc tccatcctcc     120
   ctgactgtgt cagcaggaga gaaggtcact atgagctgca agtccagtca gagtctttta     180
   gctagtggca accaaaataa ctacttggcc tggcaccagc agaaaccagg acgatctcct     240
   aaaatgctga taatttgggc atccactagg gtatctggag tccctgatcg cttcataggc     300
   agtggatctg ggacggattt cactctgacc atcaacagtg tgcaggctga agatctggct     360
   gtttattact gtcagcagtc ctacagcgct ccgctcacgt tcggtgctgg gaccaagctg     420
   gagctgaaac gtacggtggc tgcaccatct gtcttcatct tcccgccatc tgatgagcag     480
   ttgaaatctg gaactgcctc tgttgtgtgc ctgctgaata acttctatcc cagagaggcc     540
   aaagtacagt ggaaggtgga taacgccctc caatcgggta actcccagga gagtgtcaca     600
   gagcaggaca gcaaggacag cacctacagc ctcagcagca ccctgacgct gagcaaagca     660
   gactacgaga aacacaaagt ctacgcctgc gaagtcaccc atcagggcct gagctcgccc     720
   gtcacaaaga gcttcaacag gggagagtgt tgactcgag                            759
<210> 78
   <211> 4170
   <212> DNA
   <213> Artificial Sequence
<220>
   <223> polynucleotide encoding c-Met protein
<400> 78
   atgaaggccc ccgctgtgct tgcacctggc atcctcgtgc tcctgtttac cttggtgcag 60
   aggagcaatg gggagtgtaa agaggcacta gcaaagtccg agatgaatgt gaatatgaag 120
   tatcagcttc ccaacttcac cgcggaaaca cccatccaga atgtcattct acatgagcat 180
   cacattttcc ttggtgccac taactacatt tatgttttaa atgaggaaga ccttcagaag 240
   gttgctgagt acaagactgg gcctgtgctg gaacacccag attgtttccc atgtcaggac 300
   tgcagcagca aagccaattt atcaggaggt gtttggaaag ataacatcaa catggctcta 360
   gttgtcgaca cctactatga tgatcaactc attagctgtg gcagcgtcaa cagagggacc 420
   tgccagcgac atgtctttcc ccacaatcat actgctgaca tacagtcgga ggttcactgc 480
   atattctccc cacagataga agagcccagc cagtgtcctg actgtgtggt gagcgccctg 540
   ggagccaaag tcctttcatc tgtaaaggac cggttcatca acttctttgt aggcaatacc 600
   ataaattctt cttatttccc agatcatcca ttgcattcga tatcagtgag aaggctaaag 660
   gaaacgaaag atggttttat gtttttgacg gaccagtcct acattgatgt tttacctgag 720
   ttcagagatt cttaccccat taagtatgtc catgcctttg aaagcaacaa ttttatttac 780
   ttcttgacgg tccaaaggga aactctagat gctcagactt ttcacacaag aataatcagg 840
   ttctgttcca taaactctgg attgcattcc tacatggaaa tgcctctgga gtgtattctc 900
   acagaaaaga gaaaaaagag atccacaaag aaggaagtgt ttaatatact tcaggctgcg 960
   tatgtcagca agcctggggc ccagcttgct agacaaatag gagccagcct gaatgatgac 1020
   attcttttcg gggtgttcgc acaaagcaag ccagattctg ccgaaccaat ggatcgatct 1080
   gccatgtgtg cattccctat caaatatgtc aacgacttct tcaacaagat cgtcaacaaa 1140
   aacaatgtga gatgtctcca gcatttttac ggacccaatc atgagcactg ctttaatagg 1200
   acacttctga gaaattcatc aggctgtgaa gcgcgccgtg atgaatatcg aacagagttt 1260
   accacagctt tgcagcgcgt tgacttattc atgggtcaat tcagcgaagt cctcttaaca 1320
   tctatatcca ccttcattaa aggagacctc accatagcta atcttgggac atcagagggt 1380
   cgcttcatgc aggttgtggt ttctcgatca ggaccatcaa cccctcatgt gaattttctc 1440
   ctggactccc atccagtgtc tccagaagtg attgtggagc atacattaaa ccaaaatggc 1500
   tacacactgg ttatcactgg gaagaagatc acgaagatcc cattgaatgg cttgggctgc 1560
   agacatttcc agtcctgcag tcaatgcctc tctgccccac cctttgttca gtgtggctgg 1620
   tgccacgaca aatgtgtgcg atcggaggaa tgcctgagcg ggacatggac tcaacagatc 1680
   tgtctgcctg caatctacaa ggttttccca aatagtgcac cccttgaagg agggacaagg 1740
   ctgaccatat gtggctggga ctttggattt cggaggaata ataaatttga tttaaagaaa 1800
   actagagttc tccttggaaa tgagagctgc accttgactt taagtgagag cacgatgaat 1860
   acattgaaat gcacagttgg tcctgccatg aataagcatt tcaatatgtc cataattatt 1920
   tcaaatggcc acgggacaac acaatacagt acattctcct atgtggatcc tgtaataaca 1980
   agtatttcgc cgaaatacgg tcctatggct ggtggcactt tacttacttt aactggaaat 2040
   tacctaaaca gtgggaattc tagacacatt tcaattggtg gaaaaacatg tactttaaaa 2100
   agtgtgtcaa acagtattct tgaatgttat accccagccc aaaccatttc aactgagttt 2160
   gctgttaaat tgaaaattga cttagccaac cgagagacaa gcatcttcag ttaccgtgaa 2220
   gatcccattg tctatgaaat tcatccaacc aaatctttta ttagtggtgg gagcacaata 2280
   acaggtgttg ggaaaaacct gaattcagtt agtgtcccga gaatggtcat aaatgtgcat 2340
   gaagcaggaa ggaactttac agtggcatgt caacatcgct ctaattcaga gataatctgt 2400
   tgtaccactc cttccctgca acagctgaat ctgcaactcc ccctgaaaac caaagccttt 2460
   ttcatgttag atgggatcct ttccaaatac tttgatctca tttatgtaca taatcctgtg 2520
   tttaagcctt ttgaaaagcc agtgatgatc tcaatgggca atgaaaatgt actggaaatt 2580
   aagggaaatg atattgaccc tgaagcagtt aaaggtgaag tgttaaaagt tggaaataag 2640
   agctgtgaga atatacactt acattctgaa gccgttttat gcacggtccc caatgacctg 2700
   ctgaaattga acagcgagct aaatatagag tggaagcaag caatttcttc aaccgtcctt 2760
   ggaaaagtaa tagttcaacc agatcagaat ttcacaggat tgattgctgg tgttgtctca 2820
   atatcaacag cactgttatt actacttggg tttttcctgt ggctgaaaaa gagaaagcaa 2880
   attaaagatc tgggcagtga attagttcgc tacgatgcaa gagtacacac tcctcatttg 2940
   gataggcttg taagtgcccg aagtgtaagc ccaactacag aaatggtttc aaatgaatct 3000
   gtagactacc gagctacttt tccagaagat cagtttccta attcatctca gaacggttca 3060
   tgccgacaag tgcagtatcc tctgacagac atgtccccca tcctaactag tggggactct 3120
   gatatatcca gtccattact gcaaaatact gtccacattg acctcagtgc tctaaatcca 3180
   gagctggtcc aggcagtgca gcatgtagtg attgggccca gtagcctgat tgtgcatttc 3240
   aatgaagtca taggaagagg gcattttggt tgtgtatatc atgggacttt gttggacaat 3300
   gatggcaaga aaattcactg tgctgtgaaa tccttgaaca gaatcactga cataggagaa 3360
   gtttcccaat ttctgaccga gggaatcatc atgaaagatt ttagtcatcc caatgtcctc 3420
   tcgctcctgg gaatctgcct gcgaagtgaa gggtctccgc tggtggtcct accatacatg 3480
   aaacatggag atcttcgaaa tttcattcga aatgagactc ataatccaac tgtaaaagat 3540
   cttattggct ttggtcttca agtagccaaa ggcatgaaat atcttgcaag caaaaagttt 3600
   gtccacagag acttggctgc aagaaactgt atgctggatg aaaaattcac agtcaaggtt 3660
   gctgattttg gtcttgccag agacatgtat gataaagaat actatagtgt acacaacaaa 3720
   acaggtgcaa agctgccagt gaagtggatg gctttggaaa gtctgcaaac tcaaaagttt 3780
   accaccaagt cagatgtgtg gtcctttggc gtgctcctct gggagctgat gacaagagga 3840
   gccccacctt atcctgacgt aaacaccttt gatataactg tttacttgtt gcaagggaga 3900
   agactcctac aacccgaata ctgcccagac cccttatatg aagtaatgct aaaatgctgg 3960
   caccctaaag ccgaaatgcg cccatccttt tctgaactgg tgtcccggat atcagcgatc 4020
   ttctctactt tcattgggga gcactatgtc catgtgaacg ctacttatgt gaacgtaaaa 4080
   tgtgtcgctc cgtatccttc tctgttgtca tcagaagata acgctgatga tgaggtggac 4140
   acacgaccag cctccttctg ggagacatca 4170
<210> 79
   <211> 444
   <212> PRT
   <213> Artificial Sequence
<220>
   <223> SEMA domain of c-Met
<400> 79
   Leu His Glu His His Ile Phe Leu Gly Ala Thr Asn Tyr Ile Tyr Val
   1               5                   10                  15
   Leu Asn Glu Glu Asp Leu Gln Lys Val Ala Glu Tyr Lys Thr Gly Pro
               20                  25                  30
   Val Leu Glu His Pro Asp Cys Phe Pro Cys Gln Asp Cys Ser Ser Lys
           35                  40                  45
   Ala Asn Leu Ser Gly Gly Val Trp Lys Asp Asn Ile Asn Met Ala Leu
       50                  55                  60
   Val Val Asp Thr Tyr Tyr Asp Asp Gln Leu Ile Ser Cys Gly Ser Val
   65                  70                  75                      80
   Asn Arg Gly Thr Cys Gln Arg His Val Phe Pro His Asn His Thr Ala
                   85                  90                  95
   Asp Ile Gln Ser Glu Val His Cys Ile Phe Ser Pro Gln Ile Glu Glu
               100                 105                 110
   Pro Ser Gln Cys Pro Asp Cys Val Val Ser Ala Leu Gly Ala Lys Val
           115                 120                 125
   Leu Ser Ser Val Lys Asp Arg Phe Ile Asn Phe Phe Val Gly Asn Thr
       130                 135                 140
   Ile Asn Ser Ser Tyr Phe Pro Asp His Pro Leu His Ser Ile Ser Val
   145                 150                 155                 160
   Arg Arg Leu Lys Glu Thr Lys Asp Gly Phe Met Phe Leu Thr Asp Gln
                   165                 170                 175
   Ser Tyr Ile Asp Val Leu Pro Glu Phe Arg Asp Ser Tyr Pro Ile Lys
               180                 185                 190
   Tyr Val His Ala Phe Glu Ser Asn Asn Phe Ile Tyr Phe Leu Thr Val
           195                 200                 205
   Gln Arg Glu Thr Leu Asp Ala Gln Thr Phe His Thr Arg Ile Ile Arg
       210                 215                 220
   Phe Cys Ser Ile Asn Ser Gly Leu His Ser Tyr Met Glu Met Pro Leu
   225                 230                 235                 240
   Glu Cys Ile Leu Thr Glu Lys Arg Lys Lys Arg Ser Thr Lys Lys Glu
                   245                 250                 255
   Val Phe Asn Ile Leu Gln Ala Ala Tyr Val Ser Lys Pro Gly Ala Gln
               260                 265                 270
   Leu Ala Arg Gln Ile Gly Ala Ser Leu Asn Asp Asp Ile Leu Phe Gly
           275                 280                 285
   Val Phe Ala Gln Ser Lys Pro Asp Ser Ala Glu Pro Met Asp Arg Ser
       290                 295                 300
   Ala Met Cys Ala Phe Pro Ile Lys Tyr Val Asn Asp Phe Phe Asn Lys
   305                 310                 315                 320
   Ile Val Asn Lys Asn Asn Val Arg Cys Leu Gln His Phe Tyr Gly Pro
                   325                 330                 335
   Asn His Glu His Cys Phe Asn Arg Thr Leu Leu Arg Asn Ser Ser Gly
               340                 345                 350
   Cys Glu Ala Arg Arg Asp Glu Tyr Arg Thr Glu Phe Thr Thr Ala Leu
           355                 360                 365
   Gln Arg Val Asp Leu Phe Met Gly Gln Phe Ser Glu Val Leu Leu Thr
       370                 375                 380
   Ser Ile Ser Thr Phe Ile Lys Gly Asp Leu Thr Ile Ala Asn Leu Gly
   385                 390                 395                 400
   Thr Ser Glu Gly Arg Phe Met Gln Val Val Val Ser Arg Ser Gly Pro
                   405                 410                 415
   Ser Thr Pro His Val Asn Phe Leu Leu Asp Ser His Pro Val Ser Pro
               420                 425                 430
   Glu Val Ile Val Glu His Thr Leu Asn Gln Asn Gly
           435                 440
<210> 80
   <211> 451
   <212> PRT
   <213> Artificial Sequence
<220>
   <223> PSI-IPT domain of c-Met
<400> 80
   Tyr Thr Leu Val Ile Thr Gly Lys Lys Ile Thr Lys Ile Pro Leu Asn
    1               5                  10                  15
   Gly Leu Gly Cys Arg His Phe Gln Ser Cys Ser Gln Cys Leu Ser Ala
                   20                  25                  30
   Pro Pro Phe Val Gln Cys Gly Trp Cys His Asp Lys Cys Val Arg Ser
           35                  40                  45
   Glu Glu Cys Leu Ser Gly Thr Trp Thr Gln Gln Ile Cys Leu Pro Ala
       50                  55                  60
   Ile Tyr Lys Val Phe Pro Asn Ser Ala Pro Leu Glu Gly Gly Thr Arg
   65                  70                  75                  80
   Leu Thr Ile Cys Gly Trp Asp Phe Gly Phe Arg Arg Asn Asn Lys Phe
                   85                  90                  95
   Asp Leu Lys Lys Thr Arg Val Leu Leu Gly Asn Glu Ser Cys Thr Leu
               100                 105                 110
   Thr Leu Ser Glu Ser Thr Met Asn Thr Leu Lys Cys Thr Val Gly Pro
           115                 120                 125
   Ala Met Asn Lys His Phe Asn Met Ser Ile Ile Ile Ser Asn Gly His
       130                 135                 140
   Gly Thr Thr Gln Tyr Ser Thr Phe Ser Tyr Val Asp Pro Val Ile Thr
   145                 150                 155                 160
   Ser Ile Ser Pro Lys Tyr Gly Pro Met Ala Gly Gly Thr Leu Leu Thr
                   165                 170                 175
   Leu Thr Gly Asn Tyr Leu Asn Ser Gly Asn Ser Arg His Ile Ser Ile
               180                 185                 190
   Gly Gly Lys Thr Cys Thr Leu Lys Ser Val Ser Asn Ser Ile Leu Glu
           195                 200                 205
   Cys Tyr Thr Pro Ala Gln Thr Ile Ser Thr Glu Phe Ala Val Lys Leu
       210                 215                 220
   Lys Ile Asp Leu Ala Asn Arg Glu Thr Ser Ile Phe Ser Tyr Arg Glu
   225                 230                 235                 240
   Asp Pro Ile Val Tyr Glu Ile His Pro Thr Lys Ser Phe Ile Ser Thr
                   245                 250                 255
   Trp Trp Lys Glu Pro Leu Asn Ile Val Ser Phe Leu Phe Cys Phe Ala
               260                 265                 270
   Ser Gly Gly Ser Thr Ile Thr Gly Val Gly Lys Asn Leu Asn Ser Val
           275                 280                 285
   Ser Val Pro Arg Met Val Ile Asn Val His Glu Ala Gly Arg Asn Phe
       290                 295                 300
   Thr Val Ala Cys Gln His Arg Ser Asn Ser Glu Ile Ile Cys Cys Thr
   305                 310                 315                 320
   Thr Pro Ser Leu Gln Gln Leu Asn Leu Gln Leu Pro Leu Lys Thr Lys
                   325                 330                 335
   Ala Phe Phe Met Leu Asp Gly Ile Leu Ser Lys Tyr Phe Asp Leu Ile
               340                 345                 350
   Tyr Val His Asn Pro Val Phe Lys Pro Phe Glu Lys Pro Val Met Ile
               355                 360                 365
   Ser Met Gly Asn Glu Asn Val Leu Glu Ile Lys Gly Asn Asp Ile Asp
       370                 375                 380
   Pro Glu Ala Val Lys Gly Glu Val Leu Lys Val Gly Asn Lys Ser Cys
   385                 390                 395                 400
   Glu Asn Ile His Leu His Ser Glu Ala Val Leu Cys Thr Val Pro Asn
                   405                 410                 415
   Asp Leu Leu Lys Leu Asn Ser Glu Leu Asn Ile Glu Trp Lys Gln Ala
               420                 425                 430
   Ile Ser Ser Thr Val Leu Gly Lys Val Ile Val Gln Pro Asp Gln Asn
           435                 440                 445
   Phe Thr Gly
       450
<210> 81
   <211> 313
   <212> PRT
   <213> Artificial Sequence
<220>
   <223> TyrKc domain of c-Met
<400> 81
   Val His Phe Asn Glu Val Ile Gly Arg Gly His Phe Gly Cys Val Tyr
   1 5 10 15
   His Gly Thr Leu Leu Asp Asn Asp Gly Lys Lys Ile His Cys Ala Val
   20 25 30
   Lys Ser Leu Asn Arg Ile Thr Asp Ile Gly Glu Val Ser Gln Phe Leu
   35 40 45
   Thr Glu Gly Ile Ile Met Lys Asp Phe Ser His Pro Asn Val Leu Ser
   50 55 60
   Leu Leu Gly Ile Cys Leu Arg Ser Glu Gly Ser Pro Leu Val Val Leu
   65 70 75 80
   Pro Tyr Met Lys His Gly Asp Leu Arg Asn Phe Ile Arg Asn Glu Thr
   85 90 95
   His Asn Pro Thr Val Lys Asp Leu Ile Gly Phe Gly Leu Gln Val Ala
   100 105 110
   Lys Gly Met Lys Tyr Leu Ala Ser Lys Lys Phe Val His Arg Asp Leu
   115 120 125
   Ala Ala Arg Asn Cys Met Leu Asp Glu Lys Phe Thr Val Lys Val Ala
   130 135 140
   Asp Phe Gly Leu Ala Arg Asp Met Tyr Asp Lys Glu Tyr Tyr Ser Val
   145 150 155 160
   His Asn Lys Thr Gly Ala Lys Leu Pro Val Lys Trp Met Ala Leu Glu
   165 170 175
   Ser Leu Gln Thr Gln Lys Phe Thr Thr Lys Ser Asp Val Trp Ser Phe
   180 185 190
   Gly Val Leu Leu Trp Glu Leu Met Thr Arg Gly Ala Pro Pro Tyr Pro
   195 200 205
   Asp Val Asn Thr Phe Asp Ile Thr Val Tyr Leu Leu Gln Gly Arg Arg
   210 215 220
   Leu Leu Gln Pro Glu Tyr Cys Pro Asp Pro Leu Tyr Glu Val Met Leu
   225 230 235 240
   Lys Cys Trp His Pro Lys Ala Glu Met Arg Pro Ser Phe Ser Glu Leu
   245 250 255
   Val Ser Arg Ile Ser Ala Ile Phe Ser Thr Phe Ile Gly Glu His Tyr
   260 265 270
   Val His Val Asn Ala Thr Tyr Val Asn Val Lys Cys Val Ala Pro Tyr
   275 280 285
   Pro Ser Leu Leu Ser Ser Glu Asp Asn Ala Asp Asp Glu Val Asp Thr
   290 295 300
   Arg Pro Ala Ser Phe Trp Glu Thr Ser
   305 310
<210> 82
   <211> 1332
   <212> DNA
   <213> Artificial Sequence
<220>
   <223> polynucleotide encoding SEMA domain of c-Met
<400> 82
   ctacatgagc atcacatttt ccttggtgcc actaactaca tttatgtttt aaatgaggaa     60
   gaccttcaga aggttgctga gtacaagact gggcctgtgc tggaacaccc agattgtttc    120
   ccatgtcagg actgcagcag caaagccaat ttatcaggag gtgtttggaa agataacatc    180
   aacatggctc tagttgtcga cacctactat gatgatcaac tcattagctg tggcagcgtc    240
   aacagaggga cctgccagcg acatgtcttt ccccacaatc atactgctga catacagtcg    300
   gaggttcact gcatattctc cccacagata gaagagccca gccagtgtcc tgactgtgtg    360
   gtgagcgccc tgggagccaa agtcctttca tctgtaaagg accggttcat caacttcttt    420
   gtaggcaata ccataaattc ttcttatttc ccagatcatc cattgcattc gatatcagtg    480
   agaaggctaa aggaaacgaa agatggtttt atgtttttga cggaccagtc ctacattgat    540
   gttttacctg agttcagaga ttcttacccc attaagtatg tccatgcctt tgaaagcaac    600
   aattttattt acttcttgac ggtccaaagg gaaactctag atgctcagac ttttcacaca    660
   agaataatca ggttctgttc cataaactct ggattgcatt cctacatgga aatgcctctg    720
   gagtgtattc tcacagaaaa gagaaaaaag agatccacaa agaaggaagt gtttaatata    780
   cttcaggctg cgtatgtcag caagcctggg gcccagcttg ctagacaaat aggagccagc    840
   ctgaatgatg acattctttt cggggtgttc gcacaaagca agccagattc tgccgaacca    900
   atggatcgat ctgccatgtg tgcattccct atcaaatatg tcaacgactt cttcaacaag    960
   atcgtcaaca aaaacaatgt gagatgtctc cagcattttt acggacccaa tcatgagcac   1020
   tgctttaata ggacacttct gagaaattca tcaggctgtg aagcgcgccg tgatgaatat   1080
   cgaacagagt ttaccacagc tttgcagcgc gttgacttat tcatgggtca attcagcgaa   1140
   gtcctcttaa catctatatc caccttcatt aaaggagacc tcaccatagc taatcttggg   1200
   acatcagagg gtcgcttcat gcaggttgtg gtttctcgat caggaccatc aacccctcat   1260
   gtgaattttc tcctggactc ccatccagtg tctccagaag tgattgtgga gcatacatta   1320
   aaccaaaatg gc                                                       1332
<210> 83
   <211> 1299
   <212> DNA
   <213> Artificial Sequence
<220>
   <223> polynucleotide encoding PSI-IPT domain of c-Met
<400> 83
   tacacactgg ttatcactgg gaagaagatc acgaagatcc cattgaatgg cttgggctgc      60
   agacatttcc agtcctgcag tcaatgcctc tctgccccac cctttgttca gtgtggctgg     120
   tgccacgaca aatgtgtgcg atcggaggaa tgcctgagcg ggacatggac tcaacagatc     180
   tgtctgcctg caatctacaa ggttttccca aatagtgcac cccttgaagg agggacaagg     240
   ctgaccatat gtggctggga ctttggattt cggaggaata ataaatttga tttaaagaaa     300
   actagagttc tccttggaaa tgagagctgc accttgactt taagtgagag cacgatgaat     360
   acattgaaat gcacagttgg tcctgccatg aataagcatt tcaatatgtc cataattatt     420
   tcaaatggcc acgggacaac acaatacagt acattctcct atgtggatcc tgtaataaca     480
   agtatttcgc cgaaatacgg tcctatggct ggtggcactt tacttacttt aactggaaat     540
   tacctaaaca gtgggaattc tagacacatt tcaattggtg gaaaaacatg tactttaaaa     600
   agtgtgtcaa acagtattct tgaatgttat accccagccc aaaccatttc aactgagttt     660
   gctgttaaat tgaaaattga cttagccaac cgagagacaa gcatcttcag ttaccgtgaa     720
   gatcccattg tctatgaaat tcatccaacc aaatctttta ttagtggtgg gagcacaata     780
   acaggtgttg ggaaaaacct gaattcagtt agtgtcccga gaatggtcat aaatgtgcat     840
   gaagcaggaa ggaactttac agtggcatgt caacatcgct ctaattcaga gataatctgt     900
   tgtaccactc cttccctgca acagctgaat ctgcaactcc ccctgaaaac caaagccttt     960
   ttcatgttag atgggatcct ttccaaatac tttgatctca tttatgtaca taatcctgtg    1020
   tttaagcctt ttgaaaagcc agtgatgatc tcaatgggca atgaaaatgt actggaaatt    1080
   aagggaaatg atattgaccc tgaagcagtt aaaggtgaag tgttaaaagt tggaaataag    1140
   agctgtgaga atatacactt acattctgaa gccgttttat gcacggtccc caatgacctg    1200
   ctgaaattga acagcgagct aaatatagag tggaagcaag caatttcttc aaccgtcctt    1260
   ggaaaagtaa tagttcaacc agatcagaat ttcacagga                           1299
<210> 84
   <211> 939
   <212> DNA
   <213> Artificial Sequence
<220>
   <223> polynucleotide encoding TyrKc domain of c-Met
<400> 84
   gtgcatttca atgaagtcat aggaagaggg cattttggtt gtgtatatca tgggactttg      60
   ttggacaatg atggcaagaa aattcactgt gctgtgaaat ccttgaacag aatcactgac     120
   ataggagaag tttcccaatt tctgaccgag ggaatcatca tgaaagattt tagtcatccc     180
   aatgtcctct cgctcctggg aatctgcctg cgaagtgaag ggtctccgct ggtggtccta     240
   ccatacatga aacatggaga tcttcgaaat ttcattcgaa atgagactca taatccaact     300
   gtaaaagatc ttattggctt tggtcttcaa gtagccaaag gcatgaaata tcttgcaagc     360
   aaaaagtttg tccacagaga cttggctgca agaaactgta tgctggatga aaaattcaca     420
   gtcaaggttg ctgattttgg tcttgccaga gacatgtatg ataaagaata ctatagtgta     480
   cacaacaaaa caggtgcaaa gctgccagtg aagtggatgg ctttggaaag tctgcaaact     540
   caaaagttta ccaccaagtc agatgtgtgg tcctttggcg tgctcctctg ggagctgatg     600
   acaagaggag ccccacctta tcctgacgta aacacctttg atataactgt ttacttgttg     660
   caagggagaa gactcctaca acccgaatac tgcccagacc ccttatatga agtaatgcta     720
   aaatgctggc accctaaagc cgaaatgcgc ccatcctttt ctgaactggt gtcccggata     780
   tcagcgatct tctctacttt cattggggag cactatgtcc atgtgaacgc tacttatgtg     840
   aacgtaaaat gtgtcgctcc gtatccttct ctgttgtcat cagaagataa cgctgatgat     900
   gaggtggaca cacgaccagc ctccttctgg gagacatca                            939
<210> 85
   <211> 13
   <212> PRT
   <213> Artificial Sequence
<220>
   <223> heavy chain CDR3 of anti-c-Met antibody
<400> 85
   Asp Asn Trp Phe Ala Tyr Trp Gly Gln Gly Thr Leu Val
   1               5                   10
<210> 86
   <211> 10
   <212> PRT
   <213> Artificial Sequence
<220>
   <223> light chain CDR3 of anti-c-Met antibody
<400> 86
   Leu Thr Phe Gly Ala Gly Thr Lys Leu Glu
   1               5                   10
<210> 87
   <211> 117
   <212> PRT
   <213> Artificial Sequence
<220>
   <223> heavy chain variable region of monoclonal antibody AbF46
<400> 87
   Glu Val Lys Leu Val Glu Ser Gly Gly Gly Leu Val Gln Pro Gly Gly
   1               5                   10                  15
   Ser Leu Arg Leu Ser Cys Ala Thr Ser Gly Phe Thr Phe Thr Asp Tyr
               20                  25                  30
   Tyr Met Ser Trp Val Arg Gln Pro Pro Gly Lys Ala Leu Glu Trp Leu
           35                  40                  45
   Gly Phe Ile Arg Asn Lys Ala Asn Gly Tyr Thr Thr Glu Tyr Ser Ala
       50                  55                  60
   Ser Val Lys Gly Arg Phe Thr Ile Ser Arg Asp Asn Ser Gln Ser Ile
   65                  70                  75                  80
   Leu Tyr Leu Gln Met Asp Thr Leu Arg Ala Glu Asp Ser Ala Thr Tyr
                   85                  90                  95
   Tyr Cys Ala Arg Asp Asn Trp Phe Ala Tyr Trp Gly Gln Gly Thr Leu
               100                 105                 110
   Val Thr Val Ser Ala
           115
<210> 88
   <211> 114
   <212> PRT
   <213> Artificial Sequence
<220>
   <223> light chain variable region of anti-c-Met antibody
<400> 88
   Asp Ile Leu Met Thr Gln Ser Pro Ser Ser Leu Thr Val Ser Ala Gly
   1 5 10 15
   Glu Lys Val Thr Met Ser Cys Lys Ser Ser Gln Ser Leu Leu Ala Ser
   20 25 30
   Gly Asn Gln Asn Asn Tyr Leu Ala Trp His Gln Gln Lys Pro Gly Arg
   35 40 45
   Ser Pro Lys Met Leu Ile Ile Trp Ala Ser Thr Arg Val Ser Gly Val
   50 55 60
   Pro Asp Arg Phe Ile Gly Ser Gly Ser Gly Thr Asp Phe Thr Leu Thr
   65 70 75 80
   Ile Asn Ser Val Gln Ala Glu Asp Leu Ala Val Tyr Tyr Cys Gln Gln
   85 90 95
   Ser Tyr Ser Ala Pro Leu Thr Phe Gly Ala Gly Thr Lys Leu Glu Leu
   100 105 110
   Lys Arg
<210> 89
   <211> 17
   <212> PRT
   <213> Artificial Sequence
<220>
   <223> light chain CDR3 of anti-c-Met antibody
<400> 89
   Gln Gln Ser Tyr Ser Ala Pro Leu Thr Phe Gly Ala Gly Thr Lys Leu
   1 5 10 15
   Glu
<210> 90
   <211> 117
   <212> PRT
   <213> Artificial Sequence
<220>
   <223> heavy chain variable region of AT-VH1
<400> 90
   Glu Val Lys Leu Val Glu Ser Gly Gly Gly Leu Val Gln Pro Gly Gly
   1 5 10 15
   Ser Leu Arg Leu Ser Cys Ala Thr Ser Gly Phe Thr Phe Thr Asp Tyr
   20 25 30
   Tyr Met Ser Trp Val Arg Gln Pro Pro Gly Lys Gly Leu Glu Trp Leu
           35                  40                  45
   Gly Phe Ile Arg Asn Lys Ala Asn Gly Tyr Thr Thr Glu Tyr Ser Ala
       50                  55                  60
   Ser Val Lys Gly Arg Phe Thr Ile Ser Arg Asp Asn Ser Lys Ser Thr
   65                  70                  75                  80
   Leu Tyr Leu Gln Met Asn Ser Leu Arg Ala Glu Asp Ser Ala Thr Tyr
                   85                  90                  95
   Tyr Cys Ala Arg Asp Asn Trp Phe Ala Tyr Trp Gly Gln Gly Thr Leu
               100                 105                 110
   Val Thr Val Ser Ser
           115
<210> 91
   <211> 117
   <212> PRT
   <213> Artificial Sequence
<220>
   <223> heavy chain variable region of AT-VH2
<400> 91
   Glu Val Lys Leu Val Glu Ser Gly Gly Gly Leu Val Gln Pro Gly Gly
   1               5                   10                  15
   Ser Leu Arg Leu Ser Cys Ala Thr Ser Gly Phe Thr Phe Thr Asp Tyr
               20                  25                  30
   Tyr Met Ser Trp Val Arg Gln Pro Pro Gly Lys Gly Leu Glu Trp Leu
           35                  40                  45
   Gly Phe Ile Arg Asn Lys Ala Asn Gly Tyr Thr Thr Glu Tyr Ser Ala
       50                  55                  60
   Ser Val Lys Gly Arg Phe Thr Ile Ser Arg Asp Asn Ser Lys Ser Thr
   65                  70                  75                  80
   Leu Tyr Leu Gln Met Asn Ser Leu Arg Ala Glu Asp Thr Ala Thr Tyr
                   85                  90                  95
   Tyr Cys Ala Arg Asp Asn Trp Phe Ala Tyr Trp Gly Gln Gly Thr Leu
               100                 105                 110
   Val Thr Val Ser Ser
           115
<210> 92
   <211> 117
   <212> PRT
   <213> Artificial Sequence
<220>
   <223> heavy chain variable region of AT-VH3
<210> 93
   <211> 117
   <212> PRT
   <213> Artificial Sequence
<220>
   <223> heavy chain variable region of AT-VH4
<400> 93
   Glu Val Gln Leu Val Glu Ser Gly Gly Gly Leu Val Gln Pro Gly Gly
     1               5                  10                  15
   Ser Leu Arg Leu Ser Cys Ala Thr Ser Gly Phe Thr Phe Thr Asp Tyr
                20                  25                  30
   Tyr Met Ser Trp Val Arg Gln Pro Pro Gly Lys Gly Leu Glu Trp Leu
            35                  40                  45
   Gly Phe Ile Arg Asn Lys Ala Asn Gly Tyr Thr Thr Glu Tyr Ser Ala
        50                  55                  60
   Ser Val Lys Gly Arg Phe Thr Ile Ser Arg Asp Asn Ser Lys Asn Thr
    65                  70                  75                  80
   Leu Tyr Leu Gln Met Asn Ser Leu Arg Ala Glu Asp Thr Ala Thr Tyr
                    85                  90                  95
   Tyr Cys Ala Arg Asp Asn Trp Phe Ala Tyr Trp Gly Gln Gly Thr Leu
               100                 105                 110
   Val Thr Val Ser Ser
           115
<210> 94
   <211> 117
   <212> PRT
   <213> Artificial Sequence
<220>
   <223> heavy chain variable region of AT-VH5
<400> 94
   Glu Val Gln Leu Val Glu Ser Gly Gly Gly Leu Val Gln Pro Gly Gly
   1               5                   10                  15
   Ser Leu Arg Leu Ser Cys Ala Thr Ser Gly Phe Thr Phe Thr Asp Tyr
               20                  25                  30
   Tyr Met Ser Trp Val Arg Gln Pro Pro Gly Lys Gly Leu Glu Trp Leu
           35                  40                  45
   Gly Phe Ile Arg Asn Lys Ala Asn Gly Tyr Thr Thr Glu Tyr Ser Ala
       50                  55                  60
   Ser Val Lys Gly Arg Phe Thr Ile Ser Arg Asp Asn Ser Lys Asn Thr
   65                  70                  75                  80
   Leu Tyr Leu Gln Met Asn Ser Leu Arg Ala Glu Asp Thr Ala Val Tyr
                   85                  90                  95
   Tyr Cys Ala Arg Asp Asn Trp Phe Ala Tyr Trp Gly Gln Gly Thr Leu
               100                 105                 110
   Val Thr Val Ser Ser
           115
<210> 95
   <211> 114
   <212> PRT
   <213> Artificial Sequence
<220>
   <223> light chain variable region of anti c-Met humanized antibody(huAbF46-H4)
<400> 95
   Asp Ile Gln Met Thr Gln Ser Pro Ser Ser Leu Ser Ala Ser Val Gly
   1 5 10 15
   Asp Arg Val Thr Ile Thr Cys Lys Ser Ser Gln Ser Leu Leu Ala Ser
   20 25 30
   Gly Asn Gln Asn Asn Tyr Leu Ala Trp His Gln Gln Lys Pro Gly Lys
   35 40 45
   Ala Pro Lys Met Leu Ile Ile Trp Ala Ser Thr Arg Val Ser Gly Val
   50 55 60
   Pro Ser Arg Phe Ser Gly Ser Gly Ser Gly Thr Asp Phe Thr Leu Thr
   65 70 75 80
   Ile Ser Ser Leu Gln Pro Glu Asp Phe Ala Thr Tyr Tyr Cys Gln Gln
   85 90 95
   Ser Tyr Ser Ala Pro Leu Thr Phe Gly Gln Gly Thr Lys Val Glu Ile
   100 105 110
   Lys Arg
<210> 96
   <211> 113
   <212> PRT
   <213> Artificial Sequence
<220>
   <223> light chain variable region of AT-Vk1
<400> 96
   Asp Ile Leu Met Thr Gln Ser Pro Ser Ser Leu Thr Ala Ser Val Gly
   1 5 10 15
   Asp Arg Val Thr Met Thr Cys Lys Ser Ser Gln Ser Leu Leu Ala Ser
   20 25 30
   Gly Asn Gln Asn Asn Tyr Leu Ala Trp His Gln Gln Lys Pro Gly Lys
   35 40 45
   Ala Pro Lys Met Leu Ile Ile Trp Ala Ser Thr Arg Val Ser Gly Val
   50 55 60
   Pro Asp Arg Phe Ile Gly Ser Gly Ser Gly Thr Asp Phe Thr Leu Thr
   65 70 75 80
   Ile Ser Ser Leu Gln Ala Glu Asp Val Ala Val Tyr Tyr Cys Gln Gln
   85 90 95
   Ser Tyr Ser Ala Pro Leu Thr Phe Gly Gln Gly Thr Lys Leu Glu Ile
   100 105 110
   Lys
<210> 97
   <211> 113
   <212> PRT
   <213> Artificial Sequence
<220>
   <223> light chain variable region of AT-Vk2
<400> 97
   Asp Ile Leu Met Thr Gln Ser Pro Ser Ser Leu Ser Ala Ser Val Gly
   1               5                   10                  15
   Asp Arg Val Thr Ile Thr Cys Lys Ser Ser Gln Ser Leu Leu Ala Ser
               20                  25                  30
   Gly Asn Gln Asn Asn Tyr Leu Ala Trp His Gln Gln Lys Pro Gly Lys
           35                  40                  45
   Ala Pro Lys Met Leu Ile Ile Trp Ala Ser Thr Arg Val Ser Gly Val
       50                  55                  60
   Pro Asp Arg Phe Ile Gly Ser Gly Ser Gly Thr Asp Phe Thr Leu Thr
   65                  70                  75                  80
   Ile Ser Ser Leu Gln Ala Glu Asp Val Ala Val Tyr Tyr Cys Gln Gln
                   85                  90                  95
   Ser Tyr Ser Ala Pro Leu Thr Phe Gly Gln Gly Thr Lys Leu Glu Ile
               100                 105                 110
   Lys
<210> 98
   <211> 113
   <212> PRT
   <213> Artificial Sequence
<220>
   <223> light chain variable region of AT-Vk3
<400> 98
   Asp Ile Gln Met Thr Gln Ser Pro Ser Ser Leu Ser Ala Ser Val Gly
   1 5 10 15
   Asp Arg Val Thr Ile Thr Cys Lys Ser Ser Gln Ser Leu Leu Ala Ser
   20 25 30
   Gly Asn Gln Asn Asn Tyr Leu Ala Trp His Gln Gln Lys Pro Gly Lys
   35 40 45
   Ala Pro Lys Met Leu Ile Ile Trp Ala Ser Thr Arg Val Ser Gly Val
   50 55 60
   Pro Asp Arg Phe Ile Gly Ser Gly Ser Gly Thr Asp Phe Thr Leu Thr
   65 70 75 80
   Ile Ser Ser Leu Gln Ala Glu Asp Val Ala Val Tyr Tyr Cys Gln Gln
   85 90 95
   Ser Tyr Ser Ala Pro Leu Thr Phe Gly Gln Gly Thr Lys Leu Glu Ile
   100 105 110
   Lys
<210> 99
   <211> 113
   <212> PRT
   <213> Artificial Sequence
<220>
   <223> light chain variable region of AT-Vk4
<400> 99
   Asp Ile Gln Met Thr Gln Ser Pro Ser Ser Leu Ser Ala Ser Val Gly
   1 5 10 15
   Asp Arg Val Thr Ile Thr Cys Lys Ser Ser Gln Ser Leu Leu Ala Ser
   20 25 30
   Gly Asn Gln Asn Asn Tyr Leu Ala Trp His Gln Gln Lys Pro Gly Lys
   35 40 45
   Ala Pro Lys Met Leu Ile Ile Trp Ala Ser Thr Arg Val Ser Gly Val
   50 55 60
   Pro Asp Arg Phe Ser Gly Ser Gly Ser Gly Thr Asp Phe Thr Leu Thr
   65 70 75 80
   Ile Ser Ser Leu Gln Ala Glu Asp Val Ala Val Tyr Tyr Cys Gln Gln
   85 90 95
   Ser Tyr Ser Ala Pro Leu Thr Phe Gly Gln Gly Thr Lys Leu Glu Ile
   100 105 110
   Lys
<210> 100
   <211> 13
   <212> PRT
   <213> Artificial Sequence
<220>
   <223> modified hinge region(U7-HC6)
<400> 100
   Glu Pro Ser Cys Asp Lys His Cys Cys Pro Pro Cys Pro
   1 5 10
<210> 101
   <211> 13
   <212> PRT
   <213> Artificial Sequence
<220>
   <223> modified hinge region(U6-HC7)
<400> 101
   Glu Pro Lys Ser Cys Asp Cys His Cys Pro Pro Cys Pro
   1 5 10
<210> 102
   <211> 12
   <212> PRT
   <213> Artificial Sequence
<220>
   <223> modified hinge region(U3-HC9)
<400> 102
   Glu Arg Lys Cys Cys Val Glu Cys Pro Pro Cys Pro
   1 5 10
<210> 103
   <211> 14
   <212> PRT
   <213> Artificial Sequence
<220>
   <223> modified hinge region(U6-HC8)
<400> 103
   Glu Pro Arg Asp Cys Gly Cys Lys Pro Cys Pro Pro Cys Pro
   1 5 10
<210> 104
   <211> 13
   <212> PRT
   <213> Artificial Sequence
<220>
   <223> modified hinge region(U8-HC5)
<400> 104
   Glu Lys Cys Asp Lys Thr His Thr Cys Pro Pro Cys Pro
   1 5 10
<210> 105
   <211> 15
   <212> PRT
   <213> Artificial Sequence
<220>
   <223> human hinge region
<400> 105
   Glu Pro Lys Ser Cys Asp Lys Thr His Thr Cys Pro Pro Cys Pro
   1 5 10 15
<210> 106
   <211> 17
   <212> PRT
   <213> Artificial Sequence
<220>
   <223> CDR-L1 of antibody L3-11Y
<400> 106
   Lys Ser Ser Gln Ser Leu Leu Ala Trp Gly Asn Gln Asn Asn Tyr Leu
   1 5 10 15
   Ala
<210> 107
   <211> 114
   <212> PRT
   <213> Artificial Sequence
<220>
   <223> amino acid sequence of light chain variable region of antibody L3-11Y
<400> 107
   Asp Ile Gln Met Thr Gln Ser Pro Ser Ser Leu Ser Ala Ser Val Gly
   1 5 10 15
   Asp Arg Val Thr Ile Thr Cys Lys Ser Ser Gln Ser Leu Leu Ala Trp
   20 25 30
   Gly Asn Gln Asn Asn Tyr Leu Ala Trp Tyr Gln Gln Lys Pro Gly Lys
   35 40 45
   Ala Pro Lys Met Leu Ile Ile Trp Ala Ser Thr Arg Val Ser Gly Val
   50 55 60
   Pro Ser Arg Phe Ser Gly Ser Gly Ser Gly Thr Asp Phe Thr Leu Thr
   65 70 75 80
   Ile Ser Ser Leu Gln Pro Glu Asp Phe Ala Thr Tyr Tyr Cys Gln Gln
   85 90 95
   Ser Tyr Ser Arg Pro Tyr Thr Phe Gly Gln Gly Thr Lys Val Glu Ile
   100 105 110
   Lys Arg
<210> 108
   <211> 220
   <212> PRT
   <213> Artificial Sequence
<220>
   <223> amino acid sequence of light chain of antibody L3-11Y
<400> 108
   Asp Ile Gln Met Thr Gln Ser Pro Ser Ser Leu Ser Ala Ser Val Gly
   1 5 10 15
   Asp Arg Val Thr Ile Thr Cys Lys Ser Ser Gln Ser Leu Leu Ala Trp
   20 25 30
   Gly Asn Gln Asn Asn Tyr Leu Ala Trp Tyr Gln Gln Lys Pro Gly Lys
   35 40 45
   Ala Pro Lys Met Leu Ile Ile Trp Ala Ser Thr Arg Val Ser Gly Val
   50 55 60
   Pro Ser Arg Phe Ser Gly Ser Gly Ser Gly Thr Asp Phe Thr Leu Thr
   65 70 75 80
   Ile Ser Ser Leu Gln Pro Glu Asp Phe Ala Thr Tyr Tyr Cys Gln Gln
   85 90 95
   Ser Tyr Ser Arg Pro Tyr Thr Phe Gly Gln Gly Thr Lys Val Glu Ile
   100 105 110
   Lys Arg Thr Val Ala Ala Pro Ser Val Phe Ile Phe Pro Pro Ser Asp
           115                 120                 125
   Glu Gln Leu Lys Ser Gly Thr Ala Ser Val Val Cys Leu Leu Asn Asn
       130                 135                 140
   Phe Tyr Pro Arg Glu Ala Lys Val Gln Trp Lys Val Asp Asn Ala Leu
   145                 150                 155                 160
   Gln Ser Gly Asn Ser Gln Glu Ser Val Thr Glu Gln Asp Ser Lys Asp
                   165                 170                 175
   Ser Thr Tyr Ser Leu Ser Ser Thr Leu Thr Leu Ser Lys Ala Asp Tyr
               180                 185                 190
   Glu Lys His Lys Val Tyr Ala Cys Glu Val Thr His Gln Gly Leu Ser
           195                 200                 205
   Ser Pro Val Thr Lys Ser Phe Asn Arg Gly Glu Cys
       210                 215                 220
<210> 109
   <211> 1118
   <212> PRT
   <213> Artificial Sequence
<220>
   <223> human USP8 (NP_001122082)
<400> 109
   Met Pro Ala Val Ala Ser Val Pro Lys Glu Leu Tyr Leu Ser Ser Ser
    1               5                  10                  15
   Leu Lys Asp Leu Asn Lys Lys Thr Glu Val Lys Pro Glu Lys Ile Ser
               20                  25                  30
   Thr Lys Ser Tyr Val His Ser Ala Leu Lys Ile Phe Lys Thr Ala Glu
           35                  40                  45
   Glu Cys Arg Leu Asp Arg Asp Glu Glu Arg Ala Tyr Val Leu Tyr Met
       50                  55                  60
   Lys Tyr Val Thr Val Tyr Asn Leu Ile Lys Lys Arg Pro Asp Phe Lys
   65                  70                  75                  80
   Gln Gln Gln Asp Tyr Phe His Ser Ile Leu Gly Pro Gly Asn Ile Lys
                   85                  90                  95
   Lys Ala Val Glu Glu Ala Glu Arg Leu Ser Glu Ser Leu Lys Leu Arg
               100                 105                 110
   Tyr Glu Glu Ala Glu Val Arg Lys Lys Leu Glu Glu Lys Asp Arg Gln
           115                 120                 125
   Glu Glu Ala Gln Arg Leu Gln Gln Lys Arg Gln Glu Thr Gly Arg Glu
       130                 135                 140
   Asp Gly Gly Thr Leu Ala Lys Gly Ser Leu Glu Asn Val Leu Asp Ser
   145                 150                 155                 160
   Lys Asp Lys Thr Gln Lys Ser Asn Gly Glu Lys Asn Glu Lys Cys Glu
                   165                 170                 175
   Thr Lys Glu Lys Gly Ala Ile Thr Ala Lys Glu Leu Tyr Thr Met Met
   180 185 190
   Thr Asp Lys Asn Ile Ser Leu Ile Ile Met Asp Ala Arg Arg Met Gln
   195 200 205
   Asp Tyr Gln Asp Ser Cys Ile Leu His Ser Leu Ser Val Pro Glu Glu
   210 215 220
   Ala Ile Ser Pro Gly Val Thr Ala Ser Trp Ile Glu Ala His Leu Pro
   225 230 235 240
   Asp Asp Ser Lys Asp Thr Trp Lys Lys Arg Gly Asn Val Glu Tyr Val
   245 250 255
   Val Leu Leu Asp Trp Phe Ser Ser Ala Lys Asp Leu Gln Ile Gly Thr
   260 265 270
   Thr Leu Arg Ser Leu Lys Asp Ala Leu Phe Lys Trp Glu Ser Lys Thr
   275 280 285
   Val Leu Arg Asn Glu Pro Leu Val Leu Glu Gly Gly Tyr Glu Asn Trp
   290 295 300
   Leu Leu Cys Tyr Pro Gln Tyr Thr Thr Asn Ala Lys Val Thr Pro Pro
   305 310 315 320
   Pro Arg Arg Gln Asn Glu Glu Val Ser Ile Ser Leu Asp Phe Thr Tyr
   325 330 335
   Pro Ser Leu Glu Glu Ser Ile Pro Ser Lys Pro Ala Ala Gln Thr Pro
   340 345 350
   Pro Ala Ser Ile Glu Val Asp Glu Asn Ile Glu Leu Ile Ser Gly Gln
   355 360 365
   Asn Glu Arg Met Gly Pro Leu Asn Ile Ser Thr Pro Val Glu Pro Val
   370 375 380
   Ala Ala Ser Lys Ser Asp Val Ser Pro Ile Ile Gln Pro Val Pro Ser
   385 390 395 400
   Ile Lys Asn Val Pro Gln Ile Asp Arg Thr Lys Lys Pro Ala Val Lys
   405 410 415
   Leu Pro Glu Glu His Arg Ile Lys Ser Glu Ser Thr Asn His Glu Gln
   420 425 430
   Gln Ser Pro Gln Ser Gly Lys Val Ile Pro Asp Arg Ser Thr Lys Pro
   435 440 445
   Val Val Phe Ser Pro Thr Leu Met Leu Thr Asp Glu Glu Lys Ala Arg
   450 455 460
   Ile His Ala Glu Thr Ala Leu Leu Met Glu Lys Asn Lys Gln Glu Lys
   465 470 475 480
   Glu Leu Arg Glu Arg Gln Gln Glu Glu Gln Lys Glu Lys Leu Arg Lys
   485 490 495
   Glu Glu Gln Glu Gln Lys Ala Lys Lys Lys Gln Glu Ala Glu Glu Asn
   500 505 510
   Glu Ile Thr Glu Lys Gln Gln Lys Ala Lys Glu Glu Met Glu Lys Lys
           515                 520                 525
   Glu Ser Glu Gln Ala Lys Lys Glu Asp Lys Glu Thr Ser Ala Lys Arg
       530                 535                 540
   Gly Lys Glu Ile Thr Gly Val Lys Arg Gln Ser Lys Ser Glu His Glu
   545                 550                 555                 560
   Thr Ser Asp Ala Lys Lys Ser Val Glu Asp Arg Gly Lys Arg Cys Pro
                   565                 570                 575
   Thr Pro Glu Ile Gln Lys Lys Ser Thr Gly Asp Val Pro His Thr Ser
               580                 585                 590
   Val Thr Gly Asp Ser Gly Ser Gly Lys Pro Phe Lys Ile Lys Gly Gln
           595                 600                 605
   Pro Glu Ser Gly Ile Leu Arg Thr Gly Thr Phe Arg Glu Asp Thr Asp
       610                 615                 620
   Asp Thr Glu Arg Asn Lys Ala Gln Arg Glu Pro Leu Thr Arg Ala Arg
   625                 630                 635                 640
   Ser Glu Glu Met Gly Arg Ile Val Pro Gly Leu Pro Ser Gly Trp Ala
                   645                 650                 655
   Lys Phe Leu Asp Pro Ile Thr Gly Thr Phe Arg Tyr Tyr His Ser Pro
               660                 665                 670
   Thr Asn Thr Val His Met Tyr Pro Pro Glu Met Ala Pro Ser Ser Ala
           675                 680                 685
   Pro Pro Ser Thr Pro Pro Thr His Lys Ala Lys Pro Gln Ile Pro Ala
       690                 695                 700
   Glu Arg Asp Arg Glu Pro Ser Lys Leu Lys Arg Ser Tyr Ser Ser Pro
   705                 710                 715                 720
   Asp Ile Thr Gln Ala Ile Gln Glu Glu Glu Lys Arg Lys Pro Thr Val
                   725                 730                 735
   Thr Pro Thr Val Asn Arg Glu Asn Lys Pro Thr Cys Tyr Pro Lys Ala
               740                 745                 750
   Glu Ile Ser Arg Leu Ser Ala Ser Gln Ile Arg Asn Leu Asn Pro Val
           755                 760                 765
   Phe Gly Gly Ser Gly Pro Ala Leu Thr Gly Leu Arg Asn Leu Gly Asn
       770                 775                 780
   Thr Cys Tyr Met Asn Ser Ile Leu Gln Cys Leu Cys Asn Ala Pro His
   785                 790                 795                 800
   Leu Ala Asp Tyr Phe Asn Arg Asn Cys Tyr Gln Asp Asp Ile Asn Arg
                   805                 810                 815
   Ser Asn Leu Leu Gly His Lys Gly Glu Val Ala Glu Glu Phe Gly Ile
               820                 825                 830
   Ile Met Lys Ala Leu Trp Thr Gly Gln Tyr Arg Tyr Ile Ser Pro Lys
           835                 840                 845
   Asp Phe Lys Ile Thr Ile Gly Lys Ile Asn Asp Gln Phe Ala Gly Tyr
       850                 855                 860
   Ser Gln Gln Asp Ser Gln Glu Leu Leu Leu Phe Leu Met Asp Gly Leu
   865                 870                 875                 880
   His Glu Asp Leu Asn Lys Ala Asp Asn Arg Lys Arg Tyr Lys Glu Glu
                   885                 890                 895
   Asn Asn Asp His Leu Asp Asp Phe Lys Ala Ala Glu His Ala Trp Gln
               900                 905                 910
   Lys His Lys Gln Leu Asn Glu Ser Ile Ile Val Ala Leu Phe Gln Gly
           915                 920                 925
   Gln Phe Lys Ser Thr Val Gln Cys Leu Thr Cys His Lys Lys Ser Arg
       930                 935                 940
   Thr Phe Glu Ala Phe Met Tyr Leu Ser Leu Pro Leu Ala Ser Thr Ser
   945                 950                 955                 960
   Lys Cys Thr Leu Gln Asp Cys Leu Arg Leu Phe Ser Lys Glu Glu Lys
                   965                 970                 975
   Leu Thr Asp Asn Asn Arg Phe Tyr Cys Ser His Cys Arg Ala Arg Arg
               980                 985                 990
   Asp Ser Leu Lys Lys Ile Glu Ile Trp Lys Leu Pro Pro Val Leu Leu
           995                 1000                1005
   Val His Leu Lys Arg Phe Ser Tyr Asp Gly Arg Trp Lys Gln Lys Leu
      1010                1015                1020
   Gln Thr Ser Val Asp Phe Pro Leu Glu Asn Leu Asp Leu Ser Gln Tyr
   1025                1030                1035                1040
   Val Ile Gly Pro Lys Asn Asn Leu Lys Lys Tyr Asn Leu Phe Ser Val
                  1045                1050                1055
   Ser Asn His Tyr Gly Gly Leu Asp Gly Gly His Tyr Thr Ala Tyr Cys
              1060                1065                1070
   Lys Asn Ala Ala Arg Gln Arg Trp Phe Lys Phe Asp Asp His Glu Val
          1075                1080                1085
   Ser Asp Ile Ser Val Ser Ser Val Lys Ser Ser Ala Ala Tyr Ile Leu
      1090                1095                1100
   Phe Tyr Thr Ser Leu Gly Pro Arg Val Thr Asp Val Ala Thr
   1105                1110                1115
<210> 110
   <211> 5704
   <212> DNA
   <213> Artificial Sequence
<220>
   <223> human USP8 coding gene, wherein nucleptide sequence from 339th to 3695th positions is coding region (CDS)
<400> 110
   aatgcaaatc gggaaaaggg ggtgagctgg gctggcttcc gtcctggtag ccaaggctaa 60
   ttctccctcg agttcttggg agatgggcat ttggcgagaa ggctggcgtt agtgaagcgc 120
   gcccggcgtc acggtgagtg cgggtcttgg gccctagcac ctgttctctg ggaagtcgtc 180
   cgctgtgaac gatgaacgcc tttccttcca ccagctgctg gttaccccgg agacaagctc 240
   tgtccgcgga gaggagtggg acaactccta aaggaaagaa gcacttgtaa ggaaatatag 300
   catccattgt gaaagtggaa aagtaaagat aattcatcat gcctgctgtg gcttcagttc 360
   ctaaagaact ctacctcagt tcttcactaa aagaccttaa taagaagaca gaagttaaac 420
   cagagaaaat aagcactaag agttatgtgc acagtgccct gaagatcttt aagacagcag 480
   aagaatgcag attagatcgt gatgaggaaa gggcctatgt actatatatg aaatacgtga 540
   ctgtttataa tcttatcaaa aaaagacctg atttcaagca acagcaggat tatttccatt 600
   caatacttgg acctggaaac atcaaaaaag ctgtcgaaga agctgaaaga ctctctgaaa 660
   gccttaaatt aagatatgaa gaagctgaag tccggaaaaa acttgaggaa aaagacaggc 720
   aggaggaagc acagcggcta caacaaaaaa ggcaggaaac aggaagagag gatggtggca 780
   cattggctaa aggctctttg gagaatgttt tggattccaa agacaaaacc caaaagagca 840
   atggtgaaaa gaatgaaaaa tgtgagacca aagagaaagg agcaatcaca gcaaaggaac 900
   tatacacaat gatgacggat aaaaacatca gcttgattat aatggatgct cgaagaatgc 960
   aggattatca ggattcctgt attttacatt ctctcagtgt tcctgaagaa gccatcagtc 1020
   caggagtcac tgctagttgg attgaagcac acctgccaga tgattctaaa gacacatgga 1080
   agaagagggg gaatgtggag tatgtggtac ttcttgactg gtttagttct gccaaagatt 1140
   tacagattgg aacaactctc cggagtctga aagatgcact tttcaagtgg gaaagtaaaa 1200
   ctgtcctgcg caatgagcct ttggttttag agggaggcta tgaaaactgg ctcctttgtt 1260
   atccccagta tacaacaaat gctaaggtca ctccaccccc acgacgccag aatgaagagg 1320
   tgtctatctc attggatttt acttatccct cattggaaga atcaattcct tctaaacctg 1380
   ctgcccagac gccacctgca tctatagaag tagatgaaaa tatagaattg ataagtggtc 1440
   aaaatgagag aatgggacca ctgaatatat caactccagt tgaaccagtt gctgcttcta 1500
   aatctgatgt ttcacccata attcagccag tgcctagtat aaagaatgtt ccacagattg 1560
   atcgtactaa aaaaccagca gtcaaattgc ctgaagagca tagaataaaa tctgaaagta 1620
   caaaccatga gcaacaatct cctcagagtg gaaaagttat tcctgatcgt tccaccaagc 1680
   cagtagtttt ttctccaact ctcatgttaa cagatgaaga aaaggctcgt attcatgcag 1740
   aaactgctct tctaatggaa aaaaacaaac aagaaaaaga acttcgggaa aggcagcaag 1800
   aggaacagaa agagaaactg aggaaggaag aacaagaaca aaaagccaaa aagaaacaag 1860
   aagctgaaga aaatgaaatt acagagaagc aacaaaaagc aaaagaagaa atggagaaga 1920
   aagaaagtga acaggccaag aaagaagata aagaaacctc agcaaagagg ggcaaagaaa 1980
   taacaggagt aaaaagacaa agtaaaagtg aacatgaaac ttctgatgcc aagaaatctg 2040
   tagaagatag ggggaaaagg tgtccaaccc cagaaataca gaaaaagtca acaggagatg 2100
   tgccccatac atctgtgaca ggggattcag gttcaggcaa gccatttaag attaaaggac 2160
   aaccagaaag tggaattcta aggacaggaa cttttagaga ggatacagac gataccgaaa 2220
   gaaataaagc tcaacgagaa cctttgacaa gagcacgaag tgaagaaatg gggaggatcg 2280
   taccaggact gccttcaggc tgggccaagt ttcttgaccc aatcactgga acctttcgtt 2340
   attatcattc acccaccaac actgttcata tgtacccacc ggaaatggct ccttcatctg 2400
   cacctccttc cacccctcca actcataaag ccaagccaca gattcctgct gagcgggata 2460
   gggaaccttc caaactgaag cgctcctact cctccccaga tataacccag gctattcaag 2520
   aggaagagaa gaggaagcca acagtaactc caacagttaa tcgggaaaac aagccaacat 2580
   gttatcctaa agctgagatc tcaaggcttt ctgcttctca gattcggaac ctcaatcctg 2640
   tttttggagg ttctggacca gctcttactg gacttcgtaa cttaggaaat acttgttata 2700
   tgaactcaat attgcagtgc ctatgtaacg ctccacattt ggctgattat ttcaaccgaa 2760
   actgttatca ggatgatatt aacaggtcaa atttgttggg gcataaaggt gaagtggcag 2820
   aagaatttgg tataatcatg aaagccctgt ggacaggaca gtatagatat atcagtccaa 2880
   aggactttaa aatcaccatt gggaagatca atgaccagtt tgcaggatac agtcagcaag 2940
   attcacaaga attgcttctg ttcctaatgg atggtctcca tgaagatcta aataaagctg 3000
   ataatcggaa gagatataaa gaagaaaata atgatcatct cgatgacttt aaagctgcag 3060
   aacatgcctg gcagaaacac aagcagctca atgagtctat tattgttgca ctttttcagg 3120
   gtcaattcaa atctacagta cagtgcctca catgtcacaa aaagtctagg acatttgagg 3180
   ccttcatgta tttgtctcta ccactagcat ccacaagtaa atgtacatta caggattgcc 3240
   ttagattatt ttccaaagaa gaaaaactca cagataacaa cagattttac tgcagtcatt 3300
   gcagagctcg acgggattct ctaaaaaaga tagaaatctg gaagttacca cctgtgcttt 3360
   tagtgcatct gaaacgtttt tcctacgatg gcaggtggaa acaaaaatta cagacatctg 3420
   tggacttccc gttagaaaat cttgacttgt cacagtatgt tattggtcca aagaacaatt 3480
   tgaagaaata taatttgttt tctgtttcaa atcactacgg tgggctggat ggaggccact 3540
   acacagccta ttgtaaaaat gcagcaagac aacggtggtt taagtttgat gatcatgaag 3600
   tttctgatat ctccgtttct tctgtgaaat cttcagcagc ttatatcctc ttttatactt 3660
   cattgggacc acgagtaact gatgtagcca cataaggaga cataggttat aaactagtta 3720
   tcttttaaaa ggctcagcaa cacaactctt gaaatgctta tcaggataat ggtagctata 3780
   gctggccatt tagaggaatt ctaggacagt gggagctgtg ttactagcac tatataattc 3840
   cggtcagtgc tgacaaataa catttaacaa gtattgcagt aatcatcact tacaggtacc 3900
   atttatttca aaacaacttt tttagtctgc tccaaagtta aaataattaa ctagctaagc 3960
   attattattc gactggtcta aaaactattg ttatcttttt tttttccttt tcactgttat 4020
   ggccttttca catttctaaa tcccatcttg atatactatg aatactctag aatgatgtaa 4080
   agcagatagg aatgtatgtg tacatattta ttgcatactt gcacatcaaa tcgatgtaca 4140
   tagtttaaca cgtggtcctt ttgtgaaacc tagaactcag aggattgctt tttttctttc 4200
   agcctatttt gagttaactt cagtgctttc ttagggaaat gacagggcaa agcaattttt 4260
   ctgttggctt tgggctgtat ttgtgcacta aatctttatt ctaaaaaaaa aaatggaaac 4320
   tttaattttt ttaaaacgag aatttcattt acagctacat taaaatctta atgagaaaaa 4380
   taatttataa ccctgtgggt gttctgtctt taatattgta ttatcaaata taggacagta 4440
   aaaccataga ttttatatac acacgtgcta tataataaca cccagagtca ttctttcaag 4500
   actagtattc tcacatattg agaatattca ttctaaatat taaagtaaaa atgccgggag 4560
   tcaggcatga ttgcaaagtg aactgcatta taaactacat ctttacagag tgatgtatta 4620
   agagggttaa aggagcttat aatttattta accgagggac tcagttgcta tatatatagt 4680
   cagtaaaaca ctccatataa aaataagatt ctaaaagtgc ttcagaaaga gaccaccatt 4740
   agcaggctct cagggagaag atgaaaggat ggggttcaaa ttgtgaagct gacaactttt 4800
   catgttttac aattagtcta agagaccact tcttggctaa attattatat caaatatatt 4860
   caaatcatat tcttaaactc atcgagccat ttgaacaaaa attatttttg tttagcttca 4920
   tgagtatctt tggaaaataa tttgttgaat atatatgatt atgagatatt ttctgataaa 4980
   cactgaattt tgaaacctga actcactata taattgcagt gttttgaagg cctgcatcca 5040
   ttagcattgc attatattca cactgccttt tttagtgaac caagacccat cttctggacg 5100
   acagatttat cttaagatga aaggttgtat aacatgccca caaggcataa aaatgttaat 5160
   gatgcaagta agttctaaga gtttaatgac caagcaaaac tctaccacca gatgctgact 5220
   gcttgttttg cagtgttcag gaaacaccat tttcctggct cttaacgctt ttgtattggt 5280
   atggaaaagg gctggcagct atagaacagg agatccatag cattttgaac agaagtatct 5340
   ggaatctcac tgactcgtgt gttatcaaag ctatatcagg cctgggtgac tgaattcttg 5400
   cagaaagcag tgtagtggcc accatccaaa tcaccaaaat ggttctatgg gagaaaggaa 5460
   tgtcaaactt agtattcaca tatgaacact aactactgga acagaaatga tagggccaag 5520
   agatgctttt taaattgtcc cttattctaa attaaaagga agtgataatt ttgttgttaa 5580
   atcatgcata tagcctgact gctatattgc ttctcatttc attgtaacta cttatatgtt 5640
   gtgcccattg actatcatct gtgaataaag aaagacaata tttagcaaaa aaaaaaaaaa 5700
   aaaa 5704
<210> 111
   <211> 1118
   <212> PRT
   <213> Artificial Sequence
<220>
   <223> human USP8 active site mutant (C786S)
<400> 111
   Met Pro Ala Val Ala Ser Val Pro Lys Glu Leu Tyr Leu Ser Ser Ser
   1 5 10 15
   Leu Lys Asp Leu Asn Lys Lys Thr Glu Val Lys Pro Glu Lys Ile Ser
   20 25 30
   Thr Lys Ser Tyr Val His Ser Ala Leu Lys Ile Phe Lys Thr Ala Glu
   35 40 45
   Glu Cys Arg Leu Asp Arg Asp Glu Glu Arg Ala Tyr Val Leu Tyr Met
   50 55 60
   Lys Tyr Val Thr Val Tyr Asn Leu Ile Lys Lys Arg Pro Asp Phe Lys
   65 70 75 80
   Gln Gln Gln Asp Tyr Phe His Ser Ile Leu Gly Pro Gly Asn Ile Lys
   85 90 95
   Lys Ala Val Glu Glu Ala Glu Arg Leu Ser Glu Ser Leu Lys Leu Arg
   100 105 110
   Tyr Glu Glu Ala Glu Val Arg Lys Lys Leu Glu Glu Lys Asp Arg Gln
   115 120 125
   Glu Glu Ala Gln Arg Leu Gln Gln Lys Arg Gln Glu Thr Gly Arg Glu
   130 135 140
   Asp Gly Gly Thr Leu Ala Lys Gly Ser Leu Glu Asn Val Leu Asp Ser
   145 150 155 160
   Lys Asp Lys Thr Gln Lys Ser Asn Gly Glu Lys Asn Glu Lys Cys Glu
   165 170 175
   Thr Lys Glu Lys Gly Ala Ile Thr Ala Lys Glu Leu Tyr Thr Met Met
   180 185 190
   Thr Asp Lys Asn Ile Ser Leu Ile Ile Met Asp Ala Arg Arg Met Gln
   195 200 205
   Asp Tyr Gln Asp Ser Cys Ile Leu His Ser Leu Ser Val Pro Glu Glu
   210 215 220
   Ala Ile Ser Pro Gly Val Thr Ala Ser Trp Ile Glu Ala His Leu Pro
   225 230 235 240
   Asp Asp Ser Lys Asp Thr Trp Lys Lys Arg Gly Asn Val Glu Tyr Val
   245 250 255
   Val Leu Leu Asp Trp Phe Ser Ser Ala Lys Asp Leu Gln Ile Gly Thr
               260                 265                 270
   Thr Leu Arg Ser Leu Lys Asp Ala Leu Phe Lys Trp Glu Ser Lys Thr
           275                 280                 285
   Val Leu Arg Asn Glu Pro Leu Val Leu Glu Gly Gly Tyr Glu Asn Trp
       290                 295                 300
   Leu Leu Cys Tyr Pro Gln Tyr Thr Thr Asn Ala Lys Val Thr Pro Pro
   305                 310                 315                 320
   Pro Arg Arg Gln Asn Glu Glu Val Ser Ile Ser Leu Asp Phe Thr Tyr
                   325                 330                 335
   Pro Ser Leu Glu Glu Ser Ile Pro Ser Lys Pro Ala Ala Gln Thr Pro
               340                 345                 350
   Pro Ala Ser Ile Glu Val Asp Glu Asn Ile Glu Leu Ile Ser Gly Gln
           355                 360                 365
   Asn Glu Arg Met Gly Pro Leu Asn Ile Ser Thr Pro Val Glu Pro Val
       370                 375                 380
   Ala Ala Ser Lys Ser Asp Val Ser Pro Ile Ile Gln Pro Val Pro Ser
   385                 390                 395                 400
   Ile Lys Asn Val Pro Gln Ile Asp Arg Thr Lys Lys Pro Ala Val Lys
                   405                 410                 415
   Leu Pro Glu Glu His Arg Ile Lys Ser Glu Ser Thr Asn His Glu Gln
               420                 425                 430
   Gln Ser Pro Gln Ser Gly Lys Val Ile Pro Asp Arg Ser Thr Lys Pro
           435                 440                 445
   Val Val Phe Ser Pro Thr Leu Met Leu Thr Asp Glu Glu Lys Ala Arg
       450                 455                 460
   Ile His Ala Glu Thr Ala Leu Leu Met Glu Lys Asn Lys Gln Glu Lys
   465                 470                 475                 480
   Glu Leu Arg Glu Arg Gln Gln Glu Glu Gln Lys Glu Lys Leu Arg Lys
                   485                 490                 495
   Glu Glu Gln Glu Gln Lys Ala Lys Lys Lys Gln Glu Ala Glu Glu Asn
               500                 505                 510
   Glu Ile Thr Glu Lys Gln Gln Lys Ala Lys Glu Glu Met Glu Lys Lys
           515                 520                 525
   Glu Ser Glu Gln Ala Lys Lys Glu Asp Lys Glu Thr Ser Ala Lys Arg
       530                 535                 540
   Gly Lys Glu Ile Thr Gly Val Lys Arg Gln Ser Lys Ser Glu His Glu
   545                 550                 555                 560
   Thr Ser Asp Ala Lys Lys Ser Val Glu Asp Arg Gly Lys Arg Cys Pro
                   565                 570                 575
   Thr Pro Glu Ile Gln Lys Lys Ser Thr Gly Asp Val Pro His Thr Ser
               580                 585                 590
   Val Thr Gly Asp Ser Gly Ser Gly Lys Pro Phe Lys Ile Lys Gly Gln
           595                 600                 605
   Pro Glu Ser Gly Ile Leu Arg Thr Gly Thr Phe Arg Glu Asp Thr Asp
       610                 615                 620
   Asp Thr Glu Arg Asn Lys Ala Gln Arg Glu Pro Leu Thr Arg Ala Arg
   625                 630                 635                 640
   Ser Glu Glu Met Gly Arg Ile Val Pro Gly Leu Pro Ser Gly Trp Ala
                   645                 650                 655
   Lys Phe Leu Asp Pro Ile Thr Gly Thr Phe Arg Tyr Tyr His Ser Pro
               660                 665                 670
   Thr Asn Thr Val His Met Tyr Pro Pro Glu Met Ala Pro Ser Ser Ala
           675                 680                 685
   Pro Pro Ser Thr Pro Pro Thr His Lys Ala Lys Pro Gln Ile Pro Ala
       690                 695                 700
   Glu Arg Asp Arg Glu Pro Ser Lys Leu Lys Arg Ser Tyr Ser Ser Pro
   705                 710                 715                 720
   Asp Ile Thr Gln Ala Ile Gln Glu Glu Glu Lys Arg Lys Pro Thr Val
                   725                 730                 735
   Thr Pro Thr Val Asn Arg Glu Asn Lys Pro Thr Cys Tyr Pro Lys Ala
               740                 745                 750
   Glu Ile Ser Arg Leu Ser Ala Ser Gln Ile Arg Asn Leu Asn Pro Val
           755                 760                 765
   Phe Gly Gly Ser Gly Pro Ala Leu Thr Gly Leu Arg Asn Leu Gly Asn
       770                 775                 780
   Thr Ser Tyr Met Asn Ser Ile Leu Gln Cys Leu Cys Asn Ala Pro His
   785                 790                 795                 800
   Leu Ala Asp Tyr Phe Asn Arg Asn Cys Tyr Gln Asp Asp Ile Asn Arg
                   805                 810                 815
   Ser Asn Leu Leu Gly His Lys Gly Glu Val Ala Glu Glu Phe Gly Ile
               820                 825                 830
   Ile Met Lys Ala Leu Trp Thr Gly Gln Tyr Arg Tyr Ile Ser Pro Lys
           835                 840                 845
   Asp Phe Lys Ile Thr Ile Gly Lys Ile Asn Asp Gln Phe Ala Gly Tyr
       850                 855                 860
   Ser Gln Gln Asp Ser Gln Glu Leu Leu Leu Phe Leu Met Asp Gly Leu
   865                 870                 875                 880
   His Glu Asp Leu Asn Lys Ala Asp Asn Arg Lys Arg Tyr Lys Glu Glu
                   885                 890                 895
   Asn Asn Asp His Leu Asp Asp Phe Lys Ala Ala Glu His Ala Trp Gln
               900                 905                 910
   Lys His Lys Gln Leu Asn Glu Ser Ile Ile Val Ala Leu Phe Gln Gly
           915                 920                 925
   Gln Phe Lys Ser Thr Val Gln Cys Leu Thr Cys His Lys Lys Ser Arg
       930                 935                 940
   Thr Phe Glu Ala Phe Met Tyr Leu Ser Leu Pro Leu Ala Ser Thr Ser
   945                 950                 955                 960
   Lys Cys Thr Leu Gln Asp Cys Leu Arg Leu Phe Ser Lys Glu Glu Lys
                   965                 970                 975
   Leu Thr Asp Asn Asn Arg Phe Tyr Cys Ser His Cys Arg Ala Arg Arg
               980                 985                 990
   Asp Ser Leu Lys Lys Ile Glu Ile Trp Lys Leu Pro Pro Val Leu Leu
           995                 1000                1005
   Val His Leu Lys Arg Phe Ser Tyr Asp Gly Arg Trp Lys Gln Lys Leu
      1010                1015                1020
   Gln Thr Ser Val Asp Phe Pro Leu Glu Asn Leu Asp Leu Ser Gln Tyr
   1025               1030                1035                1040
   Val Ile Gly Pro Lys Asn Asn Leu Lys Lys Tyr Asn Leu Phe Ser Val
                  1045                1050                1055
   Ser Asn His Tyr Gly Gly Leu Asp Gly Gly His Tyr Thr Ala Tyr Cys
              1060                1065                1070
   Lys Asn Ala Ala Arg Gln Arg Trp Phe Lys Phe Asp Asp His Glu Val
          1075                1080                1085
   Ser Asp Ile Ser Val Ser Ser Val Lys Ser Ser Ala Ala Tyr Ile Leu
      1090                1095                1100
   Phe Tyr Thr Ser Leu Gly Pro Arg Val Thr Asp Val Ala Thr
   1105               1110                1115
<210> 112
   <211> 19
   <212> DNA
   <213> Artificial Sequence
<220>
   <223> shUSP8 mature antisense
<400> 112
   tatctcttcc gattatcag 19